(19) Europäisches Patentamt
European Patent Office
Office européen des brevets

(11) EP 1 527 101 B1

(12) **FASCICULE DE BREVET EUROPEEN**

(45) Date de publication et mention de la délivrance du brevet:
**27.10.2010 Bulletin 2010/43**

(21) Numéro de dépôt: **03756532.2**

(22) Date de dépôt: **07.08.2003**

(51) Int Cl.:
***C07K 16/26*** (2006.01) ***C12N 5/12*** (2006.01)
***G01N 33/53*** (2006.01) ***C07K 7/06*** (2006.01)
***C07K 7/08*** (2006.01) ***C07K 16/06*** (2006.01)
***G01N 33/74*** (2006.01) ***C12N 5/20*** (2006.01)

(86) Numéro de dépôt international:
**PCT/FR2003/002483**

(87) Numéro de publication internationale:
**WO 2004/014952 (19.02.2004 Gazette 2004/08)**

(54) **ANTICORPS SPECIFIQUES DU PROBNP(1-108) ET LEUR UTILISATION POUR LE DIAGNOSTIC DE L'INSUFFISANCE CARDIAQUE**

ANTIKÖRPER, DIE SPEZIFISCHE FÜR PROBNP(1-108) SIND, UND IHRE VERWENDUNG ZUR DIAGNOSE VON HERZINFARKT

PROBNP(1-108) SPECIFIC ANTIBODIES AND USES FOR DIAGNOSING HEART FAILURE

(84) Etats contractants désignés:
**AT BE BG CH CY CZ DE DK EE ES FI FR GB GR HU IE IT LI LU MC NL PT RO SE SI SK TR**

(30) Priorité: **07.08.2002 FR 0210063**

(43) Date de publication de la demande:
**04.05.2005 Bulletin 2005/18**

(83) **Déclaration conformément à la règle 32(1) CBE (solution de l'expert)**

(73) Titulaires:
- **Bio-Rad Pasteur**
  **92430 Marnes-la-Coquette (FR)**
- **CENTRE NATIONAL DE LA RECHERCHE SCIENTIFIQUE (CNRS)**
  **75016 Paris (FR)**
- **Université Montpellier I**
  **F-34060 Montpellier Cedex 1 (FR)**

(72) Inventeurs:
- **PAU, Bernard**
  **F-34080 MONTPELLIER (FR)**
- **GIULIANI, Isabelle**
  **F-34090 MONTPELLIER (FR)**
- **RIEUNIER, François**
  **F-78390 BOIS D'ARCY (FR)**

(74) Mandataire: **Colombet, Alain André et al**
**Cabinet Lavoix**
**2, Place d'Estienne d'Orves**
**75441 Paris Cedex 09 (FR)**

(56) Documents cités:
**WO-A-00/35951 WO-A-00/45176**
**US-A- 6 117 644 US-A- 6 162 902**

- **GOETZE JENS PETER ET AL: "Quantification of pro-B-type natriuretic peptide and its products in human plasma by use of an analysis independent of precursor processing." CLINICAL CHEMISTRY. UNITED STATES JUL 2002, vol. 48, no. 7, juillet 2002 (2002-07), pages 1035-1042, XP001149219 ISSN: 0009-9147**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne le domaine du diagnostic *in vitro* de l'insuffisance cardiaque ventriculaire.

L'insuffisance cardiaque (Congestive heart failure) est un syndrome clinique fréquent, en particulier chez les personnes âgées. Il se présente habituellement sous la forme d'un déclenchement insidieux de symptômes non-spécifiques tels que la toux à l'effort, la fatigue, et l'apparition d'oedèmes périphériques. Le diagnostic repose classiquement sur l'étude de divers paramètres tels que les signes cliniques (classés en 4 stades: stades I à IV de la NYHA (c'est-à-dire de l'Association de Cardiologie de New York), l'échocardiographie, la scintigraphie, le test d'effort, etc..

En raison de la gravité de la maladie cardiaque et, aussi, des coûts élevés de sa prise en charge, un diagnostic précoce de ce syndrome est, évidemment, très hautement souhaitable : il contribuerait à éviter la progression rapide du syndrome vers l'insuffisance cardiaque sévère. Identifier les personnes à risque d'insuffisance cardiaque est donc une nécessité. Cela permettrait aussi d'adapter un suivi thérapeutique plus rapide, plus facile et moins coûteux. Malheureusement, il n'existe pas de méthode diagnostique de l'insuffisance cardiaque totalement satisfaisante et totalement informative.

On a recherché depuis longtemps des marqueurs présymptomatiques prédictifs de l'insuffisance cardiaque. A cet égard, on a mis en évidence le fait que les cardiomyocytes fabriquent et sécrètent des peptides à activité natriurétique : un peptide d'origine auriculaire, l'ANP (Atrial Natriuretic Peptide) découvert chez le rat par de Bold et al. Life Science 1981, vol. 28(1) : 89-94, et un peptide natriurétique d'origine auriculo-ventriculaire dénommé BNP (Brain Natriuretic Peptide) découvert par Sudoh et al., Nature 1988, vol. 332 : 78-81 chez le porc, puis ensuite chez l'homme.

Le précurseur du BNP est le préproBNP(1-134), forme de stockage de la molécule à l'intérieur des cardiomyocytes. Celui-ci est clivé pour libérer un peptide signal et le proBNP(1-108). Le proBNP(1-108) consiste en un polypeptide de 108 acides aminés, de séquence:

$H_1$PLGSPGSASDLETSGLQEQRNHLQGKLSELQVEQTSLEPLQESPRPTGV WKSREVATEGIRGHRKMVLYTLRAPR$_{78}$S$_{77}$PKMVQGSGCFGRKMDRISSSSG LGCKVLRRH$_{108}$ (SEQ ID N° 1).

Il est clivé, avant et/ou pendant sa sécrétion, entre les acides aminés Arg$^{78}$ et Ser$^{77}$ en, d'une part, le BNP, encore désigné BNP(77-108) ou BNP-32, voire BNP(1-32), et la partie N-terminale de la prohormone, le BNP(1-76), encore désigné fragment N-terminal du proBNP ou NT-proBNP.

Le BNP ou BNP(77-108), forme vasoréactive de la molécule, consiste en un peptide de 32 acides aminés, de séquence :

S$_{77}$PKMVQGSGCFGRKMDRISSSSGLGCKVLRRH$_{108}$ (SEQ ID N° 2).

Le NT-proBNP ou BNP(1-76) consiste en les 76 acides aminés N-terminaux du proBNP(1-108) constituant la séquence suivante :

**$H_1$PLGSPGSASDLETSGLQEQRNHLQGKLSELQVEQTSLEPLQESPRPTGV WKSREVATEGIRGHRKMVLYTLRAPR$_{78}$ (SEQ ID N° 3).**

Le taux plasmatique de BNP hormonal, le BNP(77-108), est élevé chez les patients présentant une dystrophie ventriculaire. On a d'ailleurs décrit des dosages plasmatiques du BNP(77-108) l'utilisant comme marqueur prédictif de l'insuffisance cardiaque ventriculaire. Cependant, il est bien connu que l'hormone BNP(77-108) est peu stable. Il en résulte que son dosage nécessite des précautions particulières (Davidson, N. C. et al. Circulation 1995; 91:1276) (Gobinet-Georges et al. Clin. Chem. Lab. Med. 2000; 38:519-23). De plus, la demi-vie du BNP très courte et sa concentration plasmatique est peu élevée. Il en résulte qu'un certain nombre de réponses faussement négatives sont observées chez des sujets à risque d'insuffisance cardiaque. Ainsi, le dosage du BNP(77-108) ne permet pas de discriminer correctement les patients en stade I de la classification NYHA, des sujets sains (Clerico A. et al. J. Endocrinol. Invest. 1998; 21:170-9) (Del Ry S. et al. Scand. J. Clin. Lab. Invest. 2000; 60:81-90).

Pour contourner cette difficulté, la demande de brevet WO 93/24531 décrit une méthode de diagnostic *in vitro* de l'insuffisance cardiaque reposant sur la détection du BNP(1-76) (fragment N-terminal du proBNP), un composé abondant et qui a une longue demi-vie en comparaison de celle de l'hormone BNP(77-108). Toutefois, la méthode décrite dans la demande WO 93/24531 ne paraît pas simple à mettre en oeuvre sur le BNP(1-76) en échantillons sanguins. En effet, les seuls exemples montrés sont réalisés, non sur des sérums réels, mais sur des gammes étalons obtenues à l'aide d'un peptide synthétique, le peptide BNP(47-64), sous-séquence du BNP(1-76). Pour pallier cet inconvénient, un système automatisé hautement sophistiqué s'est avéré, depuis, nécessaire.

L'article Hunt et al. Biochemical and Biophysical Research Communications, vol. 214(3), 1995, pp. 1175-1183 décrit un dosage RIA de type compétitif du BNP(1-76) sur plasmas de patients souffrant d'insuffisance cardiaque, faisant intervenir un antisérum dirigé contre le fragment N-terminal du proBNP(1-13). L'article montre précisément que, chez des insuffisants cardiaques, le taux de BNP(1-76), très bien corrélé à celui du BNP(77-108), est considérablement élevé par rapport au taux observé chez des sujets témoins. Cependant, le protocole décrit pour extraire spécifiquement le seul BNP(1-76) plasmatique est complexe, puisqu'il nécessite une extraction du plasma sur cartouche Sep-pak C18™ (Millipore-Waters), suivie d'une chromatographie HPLC. Par ailleurs, cet article souligne que, dans le dosage RIA ainsi mis en oeuvre, le proBNP(1-108) ne paraît pas reconnu. Il suggère plutôt que le proBNP(1-108) pourrait être sécrété dans la circulation à partir du tissu cardiaque mais serait ensuite rapidement dégradé en un peptide plus petit, par clivage des acides N-terminaux. Ou encore, selon l'article, le proBNP(1-108) serait présenté d'une façon telle que l'antisérum anti proBNP(1-108) serait incapable de se lier à lui. Enfin, les auteurs suggèrent que le BNP(1-76) (fragment N-terminal du proBNP) pourrait même être un marqueur plus spécifique du dysfonctionnement cardiaque que le BNP(77-108) ou que le fragment N-terminal du proANP.

L'article Karl et al. (Scand. J. Clin. Lab. Invest. 1999 ; 59(suppl 230) : 177-181) décrit une méthode de détection du BNP(1-76) similaire à celle de la demande de brevet WO93/24531, mais il ne fournit aucun résultat obtenu sur échantillons de patients.

L'article Schulz et al. Scand. J. Clin. Lab. Invest., 2001, vol. 61, pp. 33-42*,* décrit également un dosage radioimmunologique spécifique du BNP(1-76) (fragment N-terminal du proBNP), sans extraction, à l'aide d'un antisérum dirigé contre les acides aminés 1-21 de ce fragment. Les auteurs confirment l'intérêt du dosage du BNP (1-76) en diagnostic de l'insuffisance cardiaque ventriculaire ainsi que sa bonne corrélation avec le dosage du BNP(77-108). Lors d'une étude des différentes formes circulantes du proBNP(1-108), ils avancent l'hypothèse que le proBNP(1-108) circulerait dans le sang, à la fois sous forme de prohormone intacte et de produits de clivage, BNP(1-76) (fragment N-terminal du proBNP) et BNP(77-108). Cependant rien n'est dit ou suggéré dans l'article concernant une éventuelle activité physiologique du proBNP(1-108) ou un intérêt diagnostique quelconque du proBNP(1-108) en tant que marqueur prédictif ou diagnostique de l'insuffisance cardiaque ventriculaire.

L'article Shimizu et al. Clinica Chimica Acta, 2002, vol. 316, pp. 129-135, présente une étude sur la dégradation du BNP humain dans le sang et les formes moléculaires circulantes de BNP immunoréactif dans le plasma d'insuffisants cardiaques. Il observe dans le plasma de ces derniers la présence de deux formes de BNP immunoréactif : un BNP de haut poids moléculaire (de 36 KD, qui pourrait correspondre à un trimère du proBNP(1-108)) et un BNP de bas poids moléculaire. Ce dernier correspond à la présence simultanée d'une forme de produit de dégradation du BNP-32 ayant perdu la sérine et la proline N-terminales (c'est la des-SerPro-BNP (BNP3-32)) de la forme hormonale du BNP-32 (ici appelée BNP(1-32)). Le proBNP(1-108) et le BNP hormonal (BNP-32, BNP(1-32) ou encore BNP(77-108)) sont donc sécrétés par le coeur dans le sang. Néanmoins, les auteurs semblent suggérer que le proBNP(1-108) sous sa forme oligomérisée (trimère) est présent en concentration proche de celle du BNP(77-108) circulant, mais ils ne la mesurent pas. Par voie de conséquence, la corrélation de la concentration du proBNP(1-108) avec l'état clinique des patients n'est pas étudiée. Il s'ensuit que l'intérêt diagnostique ou pronostique du proBNP(1-108) sérique n'y est pas démontré. Il n'est pas non plus suggéré qu'il soit possible de doser ce dernier en routine.

Par ailleurs, un certain nombre d'épitopes présents sur le proBNP(1-108) sont connus. C'est ainsi que, dans le cadre de la détection du BNP(77-108), l'épitope de séquence $S_{77}$PKMVQGSGC$_{88}$ (SEQ ID N° 105) correspondant aux 10 acides aminés N-terminaux (AA 1-10) du BNP(77-108) est décrit dans la demande WO 97/32900. Similairement, dans le cadre de la détection du BNP(1-76) (fragment N-terminal du proBNP), l'épitope de séquence $R_{65}$KMVLYTLRAPR$_{76}$ (SEQ ID N° 106) correspondant aux 12 acides aminés C-terminaux du BNP(1-76) (fragment N-terminal du proBNP) est décrit dans la demande WO 00/35951, et la séquence voisine $H_{64}$RKMVLYTLRAPR$_{78}$ (SEQ ID N° 107) est décrite dans la demande WO 00/45176 Toutefois, aucune de ces demandes de brevet ne décrit ou ne suggère l'existence d'un épitope intermédiaire ou hybride entre ces séquences.

Il existe donc toujours un besoin, dans le cadre du diagnostic précoce de l'insuffisance cardiaque, de disposer d'une méthode qui évite les inconvénients de l'art antérieur. En particulier, il existe un besoin de disposer d'une méthode simple, utilisable en routine et fiable, qui évite les inconvénients de la détection du BNP(77-108), molécule peu abondante et peu stable, tout en évitant les extractions complexes, occasionnées par le dosage d'autres formes moléculaires de BNP, et qui peuvent aller éventuellement jusqu'à requérir une automatisation sophistiquée.

Les auteurs de la présente invention se sont donc attachés à mettre au point une méthode alternative pour résoudre le problème posé. Au coeur de la présente invention se trouve la découverte inattendue, faite par les inventeurs, d'un épitope aux propriétés uniques situé dans le domaine de la séquence charnière $Y_{70}$TLRAPR$_{76}$S$_{77}$PKMVQGSG$_{85}$ (SEQ ID N° 4) ou de la séquence $Y_{70}$TLRAPR$_{76}$S77PKMVQGS$_{84}$ (SEQ ID N° 108) du proBNP(1-108) et comprenant au minimum la séquence RAPR$_{76}$S$_{77}$P (SEQ ID N° 5).

En effet, lors d'une immunisation pratiquée chez le lapin avec un peptide de la région charnière du proBNP (1-108), de séquence CY$_{70}$TLRAPR$_{76}$S$_{77}$PKMVQGSG$_{85}$ (SEQ ID N°16) ou encore avec le peptide de séquence CY$_{70}$TLRAPR$_{78}$S$_{77}$PKMVQGS$_{84}$ (SEQ ID N° 109), ils ont découvert de façon surprenante que l'antisérum obtenu non seulement contenait des anticorps qui reconnaissaient de façon spécifique ledit peptide de la région charnière sans substantiellement reconnaître les formes du BNP(1-76) et du BNP(77-108), mais en plus avait la capacité de reconnaître le proBNP(1-108) circulant.

Les auteurs de la présente invention ont, en outre, montré pour la première fois que le proBNP(1-108) circulant était effectivement un marqueur prédictif de l'insuffisance cardiaque et qu'il était présent en concentration significativement plus élevée chez les insuffisants cardiaques que chez des sujets témoins sains.

Les auteurs ont également découvert qu'une autre façon d'obtenir ce type d'anticorps est d'immuniser des animaux à l'aide de la molécule complète de proBNP(1-108). En effet, les auteurs ont trouvé que l'immunisation avec la molécule complète de proBNP(1-108) permettait d'induire l'apparition d'anticorps reconnaissant de façon spécifique une séquence de la région charnière.

De plus, les auteurs de la présente invention ont démontré que l'épitope minimum reconnu par les anticorps selon l'invention avait la séquence suivante : $RAPR_{76}S_{77}P$. Ils ont montré, en outre, qu'une bonne façon d'obtenir les anticorps objets de la présente invention est d'immuniser des animaux avec un peptide de formule générale:

$$a_1 - X_1 - RAPRSP - X_2 - a_2 \qquad (I)$$

où

- $a_1$ peut être H ou représenter une fonction ou un groupement chimique choisi parmi une fonction thiol, alcool, aminoxy, amine primaire ou secondaire, un groupement amino-carboxyle, un groupement biotinyle et un groupement acétyle,
- $X_1$ représente une séquence peptidique de 0 à 3 acides aminés, issue de la séquence naturelle du proBNP(1-108) ou non,
- $X_2$ représente une séquence peptidique de 0 à 8 acides aminés, de préférence 7 acides aminés, issue de la séquence naturelle du proBNP(1-108) ou non,
- $a_2$ peut représenter une fonction OH, $NH_2$ ou un groupement alcoxyle.

De même, les auteurs de la présente invention ont montré qu'il était possible d'obtenir les mêmes anticorps spécifiques par immunisation d'un animal avec un peptide comprenant la séquence $RAPR_{76}S_{77}P$ ou avec un peptide de formule :

$$X\text{-}Y_{70}TLRAPR_{76}S_{77}PKMVQGSG_{85}\text{-} Z \qquad (II)$$

où X peut être H ou représenter soit un groupement acétyle, soit 1 à 3 acides aminés n'appartenant pas à la séquence du proBNP(1-108), et où Z peut représenter une fonction OH, ou 1 à 3 acides aminés n'appartenant pas à la séquence du proBNP(1-108).

De plus, les auteurs de la présente invention ont montré qu'il était possible d'obtenir les mêmes anticorps spécifiques par immunisation d'un animal avec un peptide de formule :

$$X\text{-}Y_{70}TLRAPR_{76}S_{77}PKMVQGS_{84}\text{-} Z \qquad (III)$$

où X peut être H ou représenter soit un groupement acétyle, soit 1 à 3 acides aminés n'appartenant pas à la séquence du proBNP(1-108), et où Z peut représenter une fonction OH, ou 1 à 3 acides aminés n'appartenant pas à la séquence du proBNP(1-108).

Les auteurs de la présente invention ont de plus mis au point une méthode simple et fiable de diagnostic précoce de l'insuffisance cardiaque reposant sur la détection du proBNP(1-108) circulant dans le sang et une trousse permettant la mise en oeuvre de cette détection du proBNP(1-108) circulant.

La présente invention est définie par les revendications 1 à 24.

La présente invention divulgue un anticorps anti proBNP(1-108) caractérisé en ce que, d'une part, il reconnaît de façon spécifique la séquence $Y_{70}TLRAPR_{76}S_{77}PKMVQGSG_{88}$ du proBNP(1-108) et ne reconnaît substantiellement pas le BNP(1-76) ni le BNP(77-108), et, d'autre part, a la capacité de reconnaître spécifiquement le proBNP(1-108) circulant dans des échantillons sériques ou plasmatiques humains.

La présente invention divulgue de plus un anticorps anti proBNP(1-108) caractérisé en ce que, d'une part, il reconnaît de façon spécifique la séquence $Y_{70}TLRAPR_{76}S_{77}PKMVQGS_{84}$ du proBNP(1-108) et ne reconnaît substantiellement pas le BNP(1-76) ni le BNP(77-108), et d'autre part, a la capacité de reconnaître spécifiquement le proBNP (1-108) circulant dans des échantillons sériques ou plasmatiques humains.

La présente invention divulgue plus particulièrement un anticorps anti proBNP(1-108) caractérisé en ce que, d'une part, il reconnaît de façon spécifique la séquence $RAPR_{76}S_{77}P$ du proBNP(1-108) et ne reconnaît substantiellement pas le BNP(1-76) ni le BNP(77-108), et, d'autre part, a la capacité de reconnaître spécifiquement le proBNP(1-108) circulant dans des échantillons sériques ou plasmatiques humains.

La présente invention divulgue en outre un procédé d'obtention d'anticorps anti proBNP(1-108) reconnaissant de façon spécifique la séquence $Y_{70}TLRAPR_{76}S_{77}PKMVQGSG_{85}$, la séquence $Y_{70}TLRAPR_{76}S_{77}PKMVQGS_{84}$ et/ou la séquence $RAPR_{76}S_{77}P$ du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains caractérisé en ce que l'on immunise un animal avec la molécule entière de proBNP(1-108), puis en ce que l'on épuise, à l'aide du peptide BNP(77-108) et/ou du peptide BNP(1-76), l'antisérum obtenu.

Par « épuisement d'un antisérum » obtenu contre un antigène spécifique donné (l'antigène immunisant), on entend l'élimination d'anticorps non spécifiques, potentiellement présents dans cet antisérum, par la mise en contact et l'incubation dudit antisérum avec des « antigènes non spécifiques », c'est à dire des antigènes différents de l'antigène immunisant, puis séparation et élimination immunologiques des anticorps ayant réagi avec lesdits « antigènes non spécifiques » et récupération de l'antisérum ainsi épuisé (c'est-à-dire appauvri en anticorps non spécifiques). L'épuisement sert classiquement à rendre spécifique un antiserum dirigé contre un antigène donné.

Dans le présent cas, un antisérum reconnaissant la séquence $Y_{70}TLRAPR_{76}S_{77}PKMVQGSG_{85}$, et/ou la séquence $Y_{70}TLPR_{76}S_{77}PKMVQGS_{84}$ ou la séquence $RAPR_{76}S_{77}P$ du proBNP(1-108) peut être épuisé, c'est à dire rendu spécifique, par mise en contact avec la BNP(77-108) et/ou le BNP(1-76) susnommés, ces derniers pouvant, par exemple, être immobilisés en phase solide et servir de support à une chromatographie par immunoadsorption selon des techniques conventionnelles, connues de l'homme du métier. L'anticorps présent finalement dans l'antisérum épuisé est ici un anticorps polyclonal monospécifique,

La présente invention divulgue encore un peptide de formule :

$$a_1\text{-}X_1\text{-RAPRSP-}X_2\text{-}a_2 \qquad (I)$$

où

$a_1$ peut être H ou représenter une fonction ou un groupement chimique choisi parmi une fonction thiol, alcool, aminoxy, amine primaire ou secondaire, un groupement amino-carboxyle, un groupement biotinyle et un groupement acétyle,

$X_1$ représente une séquence peptidique de 0 à 3 acides aminés, issue de la séquence naturelle du proBNP(1-108) ou non,

$X_2$ représente une séquence peptidique de 0 à 8 acides aminés, de préférence 7 acides aminés, issue de la séquence naturelle du proBNP(1-108) ou non,

$a_2$ peut représenter une fonction OH, $NH_2$ ou un groupement alcoxyle.

La présente invention divulgue encore un peptide de formule :

$$X\text{-}Y_{70}TLRAPR_{76}S_{77}PKMVQGSG_{85}\text{-}Z \qquad (II)$$

où X peut être H, ou représenter soit un groupement acétyle, soit 1 à 3 acides aminés n'appartenant pas à la séquence du proBNP(1-108), et où Z peut représenter une fonction OH, ou 1 à 3 acides aminés n'appartenant pas à la séquence du proBNP(1-108).

La présente invention divulgue de plus un peptide de formule:

$$X\text{-}Y_{70}\text{-}TLRAPR_{76}S_{77}PKMVQGS_{84}\text{-}Z \qquad (III)$$

où X peut être H, ou représenter soit un groupement acétyle, soit 1 à 3 acides aminés n'appartenant pas à la séquence du proBNP(1-108), et où Z peut représenter une fonction OH, ou 1 à 3 acides aminés n'appartenant pas à la séquence du proBNP(1-108).

A noter que dans les formules (II) et (III) ci-dessus, le numéro des acides aminés ($Y_{70}$, $R_{76}$, $S_{77}$, $S_{84}$ ou $G_{85}$) a été maintenu simplement pour aider à la compréhension de l'invention et au repérage de cette séquence vis-à-vis de la séquence proBNP(1-108). Il en est de même des autres séquences présentées avec des numéros dans cette demande.

L'invention concerne aussi tout peptide contenant la séquence $X\text{-}Y_{70}TLRAPR_{76}S_{77}PKMVQGSG_{85}\text{-}Z$ (II) ou la séquence $X\text{-}Y_{70}TLRAPR_{76}S_{77}PKMVQGS_{84}\text{-}Z$ (III), ou l'une des séquences (II) ou (III) susdite sous forme substituée, de façon conservatrice ou non, en l'un quelconque des acides aminés de la position 70 à la position 85 ou 84, respectivement, à condition qu'elle garde intacte (en particulier non substituée) la portion $RAPR_{76}S_{77}P$.

Il s'agit donc d'un peptide comprenant une séquence dérivée de la séquence $X\text{-}Y_{70}TLRAPR_{76}S_{77}PKMVQCSG_{85}\text{-}Z$ (II) ou de la séquence $X\text{-}Y_{70}TLRAPR_{76}SnPKMVQGS_{84}\text{-}Z$ (III) par substitution d'un ou plusieurs parmi les acides aminés $Y_{70}$, $T_{71}$, $L_{72}$, $K_{79}$, $M_{80}$, $V_{81}$, $Q_{82}$, $G_{83}$, $S_{84}$ et $G_{85}$, avec X pouvant être absent ou représenter soit une fonction $NH_2$, soit 1 à 3 acides aminés n'appartenant pas à la séquence du proBNP(1-108), et où Z pouvant être absent ou représenter soit une fonction OH, soit 1 à 3 acides aminés n'appartenant pas à la séquence du proBNP(1-108).

L'invention concerne enfin le peptide de séquence $Y_{70}TLRAPR_{76}S_{77}PKMVqGSG_{85}$, et le peptide de séquence $Y_{70}TLRAPR_{76}S_{77}PKMVQGS_{84}$.

L'invention divulgue encore un procédé d'obtention d'anticorps anti proBNP(1-108) reconnaissant de façon spécifique la séquence $Y_{70}TLRAPR_{76}S_{77}PKMVQGSG_{85}$, $Y_{70}TLRAPR_{76}S_{77}PKMVQGS_{84}$ et/ou la séquence $RAPR_{76}S_{77}P$ du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains caractérisé en ce que l'on immunise un animal avec un peptide de formule :

$$a_1\text{-}X_1\text{-RAPRSP-}X_2\text{-}a_2 \qquad \text{(I)}$$

où $a_1$, $X_1$, $X_2$, et $a_2$ ont la même signification que ci-dessus,
et, éventuellement, en ce que l'on épuise, à l'aide du peptide BNP(77-108) et/ou du peptide BNP(1-76), l'antisérum obtenu. L'anticorps ainsi obtenu est un anticorps monospécifique.

L'invention divulgue encore un procédé d'obtention d'anticorps anti proBNP(1-108) reconnaissant de façon spécifique la séquence $Y_{70}TLRAPR_{76}S_{77}PKMVQGSG_{85}$, $Y_{70}TLRAPR_{76}S77PKMVQGS_{84}$ et/ou la séquence $RAPR_{76}S_{77}P$ du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains caractérisé en ce que l'on immunise un animal avec un peptide de formule :

$$X\text{-}Y_{70}TLRAPR_{76}S_{77}PKMVQGSGB_{85}\text{-}Z \qquad \text{(II)}$$

ou avec un peptide de formule $X\text{-}Y_{70}TLRAPR_{76}S_{77}PKMVQGS_{84}\text{-}Z$ (III),
où X et Z sont tels que définis ci-dessus et, éventuellement, en ce que l'on épuise, à l'aide du peptides BNP(77-108) et/ou du peptide BNP(1-76), l'antisérum obtenu.

L'invention divulgue encore un procédé d'obtention d'hybridome sécréteur d'anticorps anti proBNP(1-108) reconnaissant de façon spécifique la séquence $Y_{70}TLRAPR_{76}S_{77}PKMVQGSG_{85}$ $Y_{70}TLRAPR_{76}S_{77}PKMVQGS_{84}$ et/ou la séquence $RAPR_{76}S_{77}P$ du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains caractérisé en ce que l'on immunise un animal avec un peptide de formule :

$$X\text{-}Y_{70}TLRAPR_{76}S_{77}PKMVQGSG_{85}\text{- }Z \qquad \text{(II)}$$

ou avec un peptide de formule $X\text{-}Y_{70}TLRAPR_{76}S_{77}PKMVQGS_{84}\text{-}Z$ (III),
sous forme substituée, de façon conservatrice ou non, à condition qu'elle garde intacte (en particulier non substituée) la portion $RAPR_{76}S_{77}P$, où X et Z sont tels que définis ci-dessus et, éventuellement, en ce que l'on épuise, à l'aide du peptide BNP(77-108) et/ou du peptide BNP(1-76), l'antisérum obtenu.

L'invention divulgue encore un procédé d'obtention d'anticorps anti proBNP(1-108) reconnaissant de façon spécifique la séquence $Y_{70}TLRAPR_{76}S_{77}PKMVQGSG_{85}$, $Y_{70}TLRAPR_{76}S_{77}PKMVQGS_{84}$ et/ou la séquence $RAPR_{76}S_{77}P$ du proBNP(1-108) à l'exclusion substantielle des peptides BNP(1-76) et BNP(77-108), et ayant la capacité de reconnaître spécifiquement le proBNP(1-108) circulant dans des échantillons sériques ou plasmatiques humains caractérisé en ce que l'on immunise un animal avec le peptide de séquence $Y_{70}TLRAPR_{76}S_{77}PKMVQGSG_{85}$, ou le peptide de séquence $Y_{70}TLRAPR_{76}S_{77}PKMVQGS_{84}$ et, éventuellement, en ce que l'on épuise, à l'aide du peptide BNP (77-108) et/ou du peptide BNP(1-76), l'antisérum obtenu.

L'invention divulgue encore un procédé d'obtention d'hybridome sécréteur d'anticorps anti proBNP(1-108) reconnaissant de façon spécifique la séquence $Y_{70}TLRAPR_{76}S_{77}PKMVQGSG_{85}$, la séquence $Y_{70}TLRAPR_{76}S_{77}PKMVQGS_{84}$ et/ou la séquence $RAPR_{76}S_{77}P$ du proBNP(1-108) à l'exclusion substantielle du BNP (1-76) et du BNP(77-108), et ayant la capacité de reconnaître spécifiquement le proBNP(1-108) circulant dans des échantillons sériques ou plasmatiques humains caractérisé en ce que :

- on immunise un animal avec un peptide choisi parmi les peptides de formules suivantes :

$$a_1 \text{ - } X_1 \text{ - RAPRSP - } X_2\text{- } a_2 \qquad \text{(I)}$$

où $a_1$, $X_1$, $X_2$, et $a_2$ ont la même signification que ci-dessus,

X-$Y_{70}$TLRAP$R_{76}S_{77}$PKMVQGS$G_{85}$- Z (II)

où X et Z ont la même signification que ci-dessus,

X-$Y_{70}$TLRAP$R_{76}$SnPKMVQG$S_{84}$-Z (III)

où X et Z ont la même signification que ci-dessus,

X-$Y_{70}$TLRAP$R_{76}S_{77}$PKMVQGS$G_{85}$- Z (II)

sous forme substituée, de façon conservatrice ou non, à condition qu'elle garde intacte (en particulier non substituée) la portion RAP$R_{76}S_{77}$P, où X et Z ont la même signification que ci-dessus,

X-$Y_{70}$TLRAP$R_{76}S_{77}$PKMVQG$S_{84}$-Z (III)

sous forme substituée, de façon conservatrice ou non, à condition qu'elle garde intacte (en particulier non substituée) la portion RAP$R_{76}S_{77}$P,
où X et Z ont la même signification que ci-dessus,

$Y_{70}$TLRAP$R_{76}S_{77}$PKMVQGS$G_{85}$, et

$Y_{70}$TLRAP$R_{76}S_{77}$PKMVQG$S_{84}$

- on prélève des lymphocytes sécréteurs d'immunoglobulines de cet animal, et en ce que
- on procède à une fusion des lymphocytes avec des cellules de myélome pour obtenir au moins un hybridome sécréteur d'immunoglobulines.

Ceci correspond à la technique classique d'obtention des hybridomes dont le principe est décrit dans Köhler et Milsteln, (1976) Nature (London), 256:495-497.
L'invention divulgue un tel hybridome et l'anticorps anti proBNP(1-108) monoclonal sécrété par ledit hybridome. Plus particulièrement, l'invention a pour objet l'hybridome 3D4 déposé à la CNCM sous le n° CNCM I-3073 et l'anticorps secrété par cet hybridome.
La présente invention divulgue en outre une méthode de diagnostic *in vitro* de l'insuffisance cardiaque chez un humain comprenant la mise en contact d'un échantillon biologique, de préférence de sang, plasma ou sérum, avec un anticorps anti proBNP(1-108) reconnaissant de façon spécifique la séquence $Y_{70}$TLRAP$R_{76}S_{77}$PKMVQGS$G_{85}$, la séquence $Y_{70}$TLRAP$R_{76}S_{77}$PKMVQG$S_{84}$ et/ou la séquence RAP$R_{76}S_{77}$P du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains, et la détection du proBNP(1-108) dans l'échantillon.
L'invention fournit de manière générale une méthode de diagnostic *in vitro* de l'insuffisance cardiaque chez un humain comprenant :

a) la mise en contact d'un échantillon biologique, de préférence de sang, plasma ou sérum, avec un anticorps anti proBNP(1-108) reconnaissant de façon spécifique la séquence $Y_{70}$TLRAP$R_{76}S_{77}$PKMVQGS$G_{85}$, la séquence $Y_{70}$TLRAP$R_{76}S_{77}$PKMVQG$S_{84}$ et/ou la séquence RAP$R_{76}S_{77}$P du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains,
b) l'incubation du mélange dans des conditions permettant la formation de complexes antigènes-anticorps ; et
c) la révélation des complexes antigènes-anticorps formés, éventuellement à l'aide d'un anticorps de détection, marqué, capable de se lier spécifiquement au proBNP(1-108) présent dans le premier complexe, ou à l'aide d'un antigène de détection, marqué, capable de se lier à l'anticorps dirigé contre ledit proBNP(1-108) présent dans le premier complexe.

En particulier, l'invention fournit une méthode de diagnostic de l'insuffisance cardiaque qui comprend, en plus des étapes a, b et c susdites, une étape d) de corrélation de la quantité des complexes antigènes-anticorps révélés à l'état clinique du sujet.
La présente invention divulgue de plus une trousse de détection du proBNP(1-108) dans un échantillon biologique, notamment dans un échantillon de sang, plasma ou sérum, contenant au moins un anticorps anti proBNP(1-108) reconnaissant de façon spécifique la séquence $Y_{70}TLRAPR_{76}S_{77}PKMVQGSG_{85}$, la séquence $Y_{70}TLRAPR_{76}S_{77}PKMVQGS_{84}$ et/ou la séquence $RAPR_{76}S_{71}P$ du proBNP(1-108) à l'exclusion substantielle du BNP (1-76) et du BNP(77-108), et ayant la capacité de reconnaître spécifiquement le proBNP(1-108) circulant dans des échantillons sériques ou plasmatiques humains.

L'invention vise enfin une trousse de détection du proBNP(1-108) dans un échantillon biologique, notamment dans un échantillon de sang, plasma ou sérum, contenant, comme étalon et/ou témoin, un composé contenant au moins un peptide choisi dans le groupe de peptides de formules suivantes :

$a_1$-$X_1$-RAPRSP-$X_2$-$a_2$ (I)

où $a_1$, $X_1$, $X_2$, et $a_2$ ont la même signification que ci-dessus,

X-$Y_{10}TLRAPR_{76}S_{77}PKMVQGSG_{85}$- Z (II)

où X et Z ont la même signification que ci-dessus,

X-$Y_{70}TLRAPR_{76}S_{77}PKMVQGSG_{85}$- Z (II)

sous forme substituée, de façon conservatrice ou non, à condition qu'elle garde intacte (en particulier non substituée) la portion $RAPR_{76}S_{77}P$,
où X et Z ont la même signification que ci-dessus,

X-$Y_{70}TLRAPR_{76}S_{77}PKMVQGS_{84}$-Z (III),

où X et Z ont la même signification que ci-dessus,

X- $Y_{70}TLRAPR_{76}S_{77}PKMVQGS_{84}$-Z (III)

sous forme substituée, de façon conservatrice ou non, à condition qu'elle garde intacte (en particulier non substituée) la portion $RAPR_{76}S_{77}P$,
où X et Z ont la même signification que ci-dessus,

$Y_{70}TLRAPR_{76}S_{77}PKMVQGSG_{85}$,

$Y_{70}TLRAPR_{76}S_{77}PKMVQGS_{84}$.

*Définitions*

Dans le contexte de l'invention, un *« échantillon biologique »* ou encore *un « échantillon de fluide biologique »* est de préférence constitué par un liquide biologique, tel que du sang, plasma, sérum, de l'urine, du liquide céphalorachidien, de la salive, etc...

Par « insuffisance cardiaque », on entend l'état pathologique dans lequel une anomalie de la fonction cardiaque est responsable de l'incapacité du coeur à pomper le sang de façon suffisante pour répondre aux besoins métaboliques de l'organisme et/ou dans lequel le coeur pourvoit aux besoins mais avec des pressions de remplissage anormalement élevées. Il peut notamment s'agir d'une insuffisance ventriculaire droite et/ou gauche.

Le terme « *anticorps* » se réfère à tout anticorps entier ou fragment fonctionnel d'un anticorps (obtenu par génie génétique ou non), comprenant, ou consistant en, au moins un site de combinaison antigénique, permettant audit anticorps de se lier à au moins un déterminant antigénique d'un composé antigénique. A titre d'exemple de fragments d'anticorps on peut citer les fragments Fab, Fab', $F(ab')_2$ ainsi que les fragments variables à simple chaîne (chaînes scFv).

Les anticorps anti proBNP(1-108) selon l'invention peuvent être de type polyclonal ou monoclonal. Un anticorps polyclonal anti proBNP(1-108) selon l'invention peut, entre autres, être obtenu par immunisation d'un animal tel qu'un lapin, une souris, etc.. à l'aide du proBNP(1-108) entier, prélèvement puis épuisement de l'antisérum obtenu sur, par exemple, un immunoadsorbant contenant du BNP(77-108) et/ou du BNP(1-76) selon des méthodes en soi connues de l'homme du métier. Un anticorps monoclonal anti proBNP(1-108) selon l'invention peut, entre autres, être obtenu par la méthode classique de Köhler et Milstein (Nature (London), 256 : 495- 497 (1975)).

La production d'anticorps monoclonaux ou de sérums polyclonaux monospécifiques, ou d'anticorps obtenus par criblage de banques génomiques, utiles dans le cadre de l'invention relève de techniques conventionnelles, qui sont détaillées plus loin.

Par « anticorps de capture », on entend un anticorps ou une partie d'anticorps, de préférence fixé sur une phase solide, qui est capable de retenir l'antigène proBNP(1-108) présent dans un échantillon biologique, par liaison affine.

La présence de l'antigène dans l'échantillon biologique est révélée par des « moyens de détection ». S'agissant de la détection de l'antigène, l'invention prévoit notamment une détection à l'aide d'au moins un « anticorps de détection ». Un tel anticorps de détection, marqué, est capable de se lier à l'antigène capturé, par liaison affine, en reconnaissant un site épitopique, différent de celui reconnu par l'anticorps de capture.

Le terme « marqué » se réfère aussi bien à un marquage direct (par le biais d'enzymes, radioisotopes, fluorochromes, composés luminescents, etc) qu'à un marquage indirect (par exemple, par le biais d'anticorps eux-mêmes marqués de manière directe ou à l'aide de réactifs d'une « paire d'affinité » marquée, telle que, mais non exclusivement, la paire avidine marquée-biotine, etc..).

Par « fragment antigénique », on entend toute partie du proBNP(1-108) capable d'induire la synthèse d'anticorps substantiellement spécifique du seul proBNP(1-108) chez un animal immunisé.

Conformément à la présente invention, un « fragment antigénique » contient au moins le « site épitopique » ou épitope $RAPR_{76}S_{77}P$. Un « site épitopique » ou « épitope » est une séquence d'acides aminés qui est reconnue par au moins un anticorps et permet la liaison spécifique de celui-ci.

Le terme «anticorps polyclonal monospécifique », s'applique à tout anticorps polyclonal présentant une spécificité pour un seul épitope. Ceci signifie que l'anticorps est capable de lier une séquence d'acides aminés de la séquence du proBNP(1-108) contenant les acides aminés comprenant l'épitope, mais est incapable de lier une séquence d'acides aminés de la séquence du proBNP(1-108) qui ne contient pas les acides aminés comprenant l'épitope.

Le terme « spécifiquement », quand il se réfère à une reconnaissance ou une liaison spécifique d'un anticorps pour un antigène, signifie que l'anticorps interagit avec l'antigène sans interaction substantielle avec d'autres antigènes, ou si l'on parle de reconnaissance « spécifique » avec un épitope, par reconnaissance quasi-exclusive de cet épitope. Des constantes d'association supérieures à $10^8$ $L.mol^{-1}$ sont préférables.

Par « substitution conservatrice », on entend notamment la substitution d'un acide aminé d'une classe par un acide aminé de la même classe, substitution qui ne modifie pas significativement l'immunoréactivité du peptide obtenu par rapport à celle du peptide d'origine. Parmi les différentes classe d'acides aminés on distingue généralement les acides aminés à chaîne latérale polaire (tel que l'asparagine, la glutamine, la sérine, la thréonine et la tyrosine), les acides aminés à chaîne latérale non polaire (tel que la glycine, l'alanine, la valine, la leucine, l'isoleucine, la phénylalanine, la méthionine, le tryptophane et la cystéine), les acides aminés à chaîne latérale basique (tel que la lysine, l'arginine et l'histidine); et les acides aminés à chaîne latérale acide (tel que l'acide aspartique et l'acide glutamique).

Par « substitution non conservatrice », on entend tout autre type de substitution, qui ne modifie pas significativement l'immunoréactivité du peptide obtenu par rapport à celle du peptide d'origine.

Par l'expression « ne reconnaît substantiellement pas le BNP(1-76) ni le BNP(77-108) », on entend que l'anticorps visé par l'invention présente une réaction croisée avec le BNP(1-76) ou le BNP(77-108) inférieure à 20%, particulièrement inférieure à 10%, de façon préférée inférieure à 5%. La détermination du pourcentage de la réaction croisée se fait selon des méthodes en soi connues de l'homme du métier, par exemple, celle illustrée dans l'exemple 5.

La non-reconnaissance « *substantielle* » des peptides BNP(1-76) et BNP(77-108) correspond de préférence à une absence ou quasi-absence de réaction des anticorps de l'invention avec ces peptides.

Par l'expression « à l'exclusion substantielle du BNP(1-76) et du BNP(77-108) », on entend que l'anticorps « ne reconnaît substantiellement pas le BNP(1-76) ni le BNP(77-108) » au sens vu plus haut.

*Production d'anticorps spécifiques*

Les anticorps utilisés dans la présente invention sont des anticorps spécifiques de l'antigène, et, pour cette raison, sont des anticorps monoclonaux ou des anticorps polyclonaux monospécifiques, c'est à dire qu'ils ne reconnaissent spécifiquement qu'un épitope.

Les anticorps monoclonaux peuvent être obtenus selon la méthode classique de fusion lymphocytaire et culture d'hybridomes décrite par Köhler et Milstein, Nature, 1975, 256: 495-497. D'autres méthodes de préparation d'anticorps monoclonaux sont également connues (Harlow et al, ed., 1988 « Antibodies : a laboratory manual »). Les anticorps monoclonaux peuvent être préparés en immunisant un mammifère (par exemple une souris, un rat, un lapin, voire un être humain, etc..) et en utilisant la technique de fusion lymphocytaire conduisant à des hybridomes (Köhler et Milstein, 1975).

Des techniques alternatives à cette technique usuelle existent. On peut, par exemple, produire des anticorps monoclonaux par expression d'un acide nucléique cloné à partir d'un hybridome. On peut également produire des anticorps par la technique d'expression sur phage (« phage display »), en introduisant des ADNc d'anticorps dans des vecteurs, qui sont typiquement des phages filamenteux qui présentent des banques de gènes V à la surface du phage (par exemple fUSE5 pour E. coli, Scott, J.K. et Smith, G.P., Science, 1990, 249 :386-390). Des protocoles de construction de ces banques d'anticorps sont décrits dans Marks et al, 1991, J. Mol. Biol, 222 :581-597).

Les anticorps polyclonaux peuvent être obtenus à partir du sérum d'un animal immunisé contre un antigène de nature peptidique selon les modes opératoires usuels. Les anticorps polyclonaux ainsi obtenus pourront, si besoin est, et par épuisement avec du BNP(77-108) et du BNP1-76, selon des techniques en soit connues de l'homme du métier comme, par exemple, l'immunoadsorption sur colonne, être rendus monospécifiques de la séquence $Y_{70}TLRAPR_{76}S_{77}PKMVQGSG_{85}$, de la séquence $Y_{70}TLRAPR_{76}S_{77}PKMVQG_{84}$ et/ou d'un peptide comprenant la séquence $RAPR_{76}S_{77}P$, assurant ainsi la spécificité vis à vis du proBNP(1-108), à l'exclusion substantielle du BNP(1-76) et du BNP(77-108).

*Anticorps de capture et/ou de détection*

En technique sandwich, l'anticorps de capture est choisi de manière à ce qu'il reconnaisse spécifiquement un épitope, sur l'antigène naturel du patient, tandis que l'anticorps de détection est choisi, de préférence, mais non obligatoirement de manière à ce qu'il reconnaisse spécifiquement un autre épitope, sur l'antigène naturel du patient.

L'échantillon biologique peut être éventuellement traité dans une étape préalable, ou mis directement en présence d'au moins un anticorps de capture dans des conditions favorisant l'exposition de l'épitope à détecter.

Le procédé de diagnostic selon l'invention peut être réalisé selon divers formats bien connus de l'homme du métier: en phase solide ou en phase homogène ; en un temps ou en deux temps ; en méthode sandwich ou en méthode compétitive, à titre d'exemples non limitatifs.

Selon un mode de réalisation préféré, l'anticorps de capture est immobilisé sur une phase solide. On peut-utiliser, à-titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque Estapor™), ou encore des tubes à essai en polystyrène ou polypropylène, etc...

Un format d'immunoessai de type sandwich entre deux anticorps (de capture et de détection) est particulièrement avantageux pour la détection des antigènes présents dans l'échantillon biologique.

Un format d'immunoessai de détection des antigènes par compétition est également possible. D'autres modalités d'immunoessais sont encore envisageables et bien connues de l'homme du métier.

Dosages ELISA, radioimmunoessais, ou toute autre technique de détection peuvent être mis en oeuvre pour révéler la présence des complexes antigènes-anticorps formés.

*Trousses*

Les trousses et réactifs utiles pour la détection du proBNP(1-108) dans un échantillon de fluide biologique, conformément au procédé de l'invention, peuvent être fournis pour une mise en pratique de l'invention facile et applicable à de nombreux échantillons biologiques.

Des trousses de détection du proBNP(1-108) dans un échantillon biologique peuvent contenir au moins un anticorps tel que défini précédemment. D'autres trousses, contenant comme étalon et/ou témoin, au moins un peptide de formule (I), (II) ou un peptide substitué tel que définis précédemment, peuvent également être utiles à la mise en oeuvre de l'invention.

L'invention concerne donc une trousse de détection du proBNP(1-108) dans un échantillon de fluide biologique, comprenant :

- au moins un anticorps anti proBNP(1-108) reconnaissant de façon spécifique la séquence $Y_{70}TLRAPR_{76}S_{77}PKMVQGSG_{85}$, la séquence $Y_{70}TLRAPR_{76}S_{77}PKMVQGS_{84}$, et/ou la séquence $RAPR_{76}S_{77}P$ du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains, et qui est de préférence un anticorps de capture dudit proBNP(1-108) présent dans l'échantillon biologique, et
- un conjugué marqué capable de se lier spécifiquement aux complexes antigènes anticorps formés.

Comme cela est décrit ci-dessus, l'anticorps de capture peut être présenté de manière avantageuse sous une forme immobilisée sur une phase solide, telle qu'une microplaque, par exemple, mais non exclusivement.

Une trousse préférée comprend au moins :

- un anticorps de capture anti proBNP(1-108) reconnaissant de façon spécifique la séquence $Y_{70}TLRAPR_{76}S_{77}PKMVQGSG_{85,}$ la séquence $Y_{70}TLRAPR_{76}S_{77}PKMVQGS_{84}$, et/ou la séquence $RAPR_{76}S_{77}P$ du proBNP(1-108) à l'exclusion substantielle du BNP(1-76) et du BNP(77-108), et ayant la capacité de reconnaître spécifiquement le proBNP(1-108) circulant dans des échantillons sériques ou plasmatiques humains, ledit anticorps de capture étant immobilisé sur une phase solide ; et

- un anticorps de détection, marqué, dirigé contre un autre épitope, intact, du proBNP(1-108), ou éventuellement,
- un antigène de détection, marqué, qui est un peptide du proBNP(1-108) ou le proBNP(1-108) lui-même.

Selon un mode de réalisation particulier, une trousse de détection du proBNP(1-108) dans un échantillon biologique, peut contenir :

- dans un récipient, au moins un anticorps tel que défini précédemment ;
- dans un autre récipient au moins un peptide tel que défini précédemment, utile notamment comme étalon et/ou témoin.

Les figures et exemples suivants illustrent l'invention sans en limiter la portée.

## LEGENDE DES FIGURES:

La figure 1 représente la réactivité des anticorps polyclonaux du lapin # 046 805 purifiés sur protéine A-sépharose avec le proBNP (1-108), le fragment BNP(1-76), le BNP(77-108), ou la GST (Glutathion-S-transférase, utilisée comme protéine témoin) adsorbés sur microplaque à 0,25 μg/ml.

La figure 2 représente la réactivité des anticorps polyclonaux du filtrat du lapin # 046 805 obtenus après épuisement sur résine BNP-$K_3$-NHS-sépharose. Les anticorps polyclonaux sont préparés sous forme de gammes de dilutions de 2 à 20μg/ml, puis testés sur des cupules revêtues de polypeptide proBNP(1-108), BNP(1-76) ou BNP(77-108) adsorbé à 0,25 μg/ml.

La figure 3 représente la réactivité des anticorps polyclonaux du filtrat du lapin # 046 832 obtenus après épuisement sur résine BNP-$K_3$-NHS-sépharose. Les anticorps polyclonaux sont préparés sous forme de gammes de dilutions de 5 à 20 μg/ml, puis testés sur des cupules revêtues de polypeptide proBNP(1-108), BNP(1-76) ou BNP(77-108) adsorbé à 0,25 μg/ml.

La figure 4 représente la réactivité de l'éluat du sérum polyclonal du lapin # 046 805 obtenu après épuisement sur résine BNP-$K_3$-NHS-sépharose. Les anticorps polyclonaux sont préparés sous forme de gammes de dilutions de 2 à 20 μg/ml, puis testés sur des cupules revêtues de polypeptide proBNP(1-108), BNP(1-76) ou BNP(77-108) adsorbé à 0,25 μg/ml.

La figure 5 représente la réactivité de l'éluat du sérum polyclonal du lapin # 046 832 obtenu après épuisement sur résine BNP-$K_3$-NHS-sépharose. Les anticorps polyclonaux sont préparés sous forme de gammes de dilutions de 5 à 20 μg/ml, puis testés sur des cupules revêtues de polypeptide proBNP(1-108), BNP(1-76) ou BNP(77-108) adsorbé à 0,25 μg/ml.

La figure 6 représente une analyse épitopique à l'aide de la technique spot du sérum polyclonal du lapin # 046 805 avant épuisement. (Pour cet exemple, le bruit de fond est de 30,4 ± 5,93 unités d'intensité relatives.)

La figure 7 représente une analyse épitopique à l'aide de la technique spot du sérum polyclonal du lapin # 046 805 après épuisement sur résine BNP-$K_3$-NHS-sépharose. (Pour cet exemple, le bruit de fond est de 31,3 ± 6,31 unités d'intensité relatives.)

La figure 8 représente la réactivité du surnageant de culture de l'hybridome 3D4 dilué au 1/2, testé sur des cupules revêtues soit de proBNP(1-108), soit de BNP(1-76), soit de -BNP(77-108), soit de peptide YTLRAPRSPKMVQGSG (C13P30), adsorbé à 1 μg/ml.

La figure 9 montre une photographie du gel d'acrylamide à 16% dans lequel on a fait migrer chaque protéine (1 μg de protéine par piste). Piste 1 : marqueur de poids moléculaire ; piste 2 : proBNP(1-108)-GST ; piste 3 : GST (protéine témoin négatif); piste 4 : BNP(1-76)-GST ; piste 5 : BNP(77-108).

La figure 10 montre les résultats du test en western-blot de l'anticorps produit par l'hybridome 3D4 sur le proBNP (1-108)-GST (piste 2), la GST (protéine témoin négatif) (piste 3), le BNP(1-76)-GST (piste 4), et le BNP(77-108) (piste 5), déposés sur gel (1μg de protéine par gel), puis transférés sur membrane de nitrocellulose.

La figure 11 représente une courbe d'étalonnage du dosage IRMA du proBNP(1-108).

La figure 12 montre les résultats en cpm (coups par minute de radioactivité) du dosage IRMA du proBNP(1-108) des prélèvements de 14 sujets sains et de 15 patients souffrant d'insuffisance cardiaque.

La figure 13 présente la corrélation entre les concentrations de proBNP(1-108) (en pg/ml) déterminées à l'aide du dosage radioimmunométrique selon l'invention et les concentrations de BNP(1-76) (pg/ml) déterminées, à l'aide du dosage sur l'automate Elecsys® (marque de la société Hoffmann La Roche), des prélèvements de 14 patients souffrant d'insuffisance cardiaque.

La figure 14 représente une courbe d'étalonnage du dosage ELISA du proBNP(1-108) selon l'invention.

La figure 15 représente une évaluation de la réaction croisée, vis à vis du BNP(1-76) et du BNP(77-108), de l'anticorps polyclonal du lapin # 046 805 non épuisé couplé à la biotine, utilisé en sandwich dans le dosage ELISA proBNP(1-108) conjointement à l'anticorps polyclonal anti-BNP(77-108).

La figure 16 représente l'évaluation de la réaction croisée, vis à vis du BNP(1-76) et du BNP(77-108), de l'anticorps polyclonal du lapin # 046 805 non épuisé couplé à la biotine, utilisé en sandwich dans le dosage ELISA proBNP (1-108) conjointement à l'anticorps polyclonal anti-NT-proBNP(1-29).

## EXEMPLES

### Exemple 1 : synthèse de peptides pour immunisation

Les peptides synthétiques sont produits par les techniques standards bien connues de l'homme du métier. On peut citer, comme exemple, la synthèse de type Merrifield qui est avantageuse vu sa facilité de mise en oeuvre (Merrifield, (1963); R.C.Sheppard (1971) ; Atherton et al. (1989)). Comme synthétiseur automatique, on peut utiliser le synthétiseur "9050 Plus Pep Synthesizer" de Millipore, le "Pioneer" de Perspective, ou le synthétiseur "433A" de ABI. Les peptides peuvent également être obtenus par synthèse en phase homogène.

Les synthèses ci-après ont été réalisées sur un synthétiseur Pioneer, en utilisant la chimie « Fmoc » (9-fluorénylméthyloxy carbonyl) : à chaque étape les réactifs (c'est à dire l'acide aminé protégé et les activateurs de couplage (TBTU(2-(1H-benzotriazol-1-yl)-1,1,3,3-tétraméthyluronium tétrafluoroborate)/ HOBt (N-hydroxybenzotriazol)) sont ajoutés en excès (dans un rapport « moles de réactifs /moles de groupes substituables sur la résine » = 5). A la fin de la synthèse, le peptide est séparé de la résine par une solution à base d'acide trifluoroacétique (réactif K). Le peptide est ensuite précipité dans une solution d'éther refroidie, puis purifié par HPLC.

Les inventeurs ont procédé à la synthèse des peptides contenant la séquence d'acides aminés de la région charnière $R_{76}S_{77}$ suivants :

| | |
|---|---|
| SEQ ID N° 6 : | C-G-R-A-P-R-S-P |
| SEQ ID N° 7 : | Acetyl -C-G-R-A-P-R-S-P |
| SEQ ID N° 8 : | C-G-R-A-P-R-S-P-K |
| SEQ ID N° 9 : | Acetyl -C-G-R-A-P-R-S-P-K |
| SEQ ID N° 10: | C-G-R-A-P-R-S-P-K-M-V |
| SEQ ID N° 11 : | C-G-R-A-P-R-S-P-K-M-V-Q-G-S-G |
| SEQ ID N° 12 : | R-A-P-R-S-P-G-C |
| SEQ ID N° 13 : | Acetyl -R-A-P-R-S-P-G-C |
| SEQ ID N° 14 : | Acetyl -C-Y-T-L-R-A-P-R-S-P-K |
| SEQ ID N° 15 : | C-H-R-K-M-V-L-Y-T-L-R-A-P-R-S-P-K |
| SEQ ID N° 16 : | C-Y-T-L-R-A-P-R-S-P-K-M-V-Q-G-S-G (peptide C13P30) |
| SEQ ID N° 17 : | C-F-T-L-R-A-P-R-S-P-K-M-V-Q-G-S-G |
| SEQ ID N° 18 : | C-F-S-I-R-A-P-R-S-P-K-M-V-Q-G-S-G |
| SEQ ID N° 19 : | C-Y-T-L-R-A-P-R-S-P-K-M-V-Q-G-S-βA |
| S EQ ID N° 20 : | C-Y-T-L-R-A-P-R-S-P-K-M-V-Q-A-T-βA |
| SEQ ID N° 21 : | C-F-S-I-R-A-P-R-S-P-K-M-V-Q-A-T-βA |
| SEQ ID N° 22 : | GF-S-I-R-A-P-R-S-P-A-L-A-S-G-T-A |
| ainsi que | |
| SEQ ID N° 109 : | C-Y-T-L-R-A-P-R-S-P-K-M-V-Q-G-S (peptide CN32) |
| SEQ ID N° 110 : | C-Y-T-L-R-A-P-R-S-P-K |
| SEQ ID N° 111 : | C-Y-T-L-R-A-P-R-S-P-K-M-V |
| SEQ ID N° 112 : | C-Y-T-L-R-A-P-R-S-P-K-M-V-Q |
| SEQ ID N° 113 : | C-Y-T-L-R-A-P-R-S-P-K-M-V-Q-G |
| SEQ ID N° 114 : | Acétyl-C-T-L-R-A-P-R-S-P-K-M-V-Q |
| SEQ ID N° 115 : | C-T-L-R-A-P-R-S-P-K-M-V-Q-G |
| SEQ ID N° 116 : | C-T-L-R-A-P-R-S-P-K-M-V-Q-G-S |
| SEQ ID N° 117 : | C-T-L-R-A-P-R-S-P-K-M-V-Q-G-S-G |
| SEQ ID N° 118 : | C-L-R-A-P-R-S-P-K-M-V |
| SEQ ID N° 119 : | C-L-R-A-P-R-S-P-K-M-V-Q |

(suite)

| | |
|---|---|
| SEQ ID N° 120 : | L-R-A-P-R-S-P-K-M-V-Q-C |
| SEQ ID N° 121 : | C-L-R-A-P-R-S-P-K-M-V-Q-G-S |
| SEQ ID N ° 122 : | C-L-R-A-P-R-S-P-K-M-V-Q-G-S-G |
| NB : pA signifie béta-alanine. | |

L'invention a donc aussi pour objet un peptide quelconque choisi dans le groupe constitué par les séquences ci-dessus.

**Exemple 2: Couplage d'un peptide à une protéine porteuse pour immunisation**

On couple le peptide à une protéine porteuse la KLH (Keyhole Lympet Hemocyanin), La thyroglobuline; la BSA (Bovine Serum Albumin, via différentes fonctions (thiol, amine, aldéhyde...) de façon à rendre le peptide plus immunogène. L'agent de couplage utilisé pour lié le peptide à la protéine peut être hétéro- ou homo-bifonctionel. Les réactifs les plus couramment utilisés sont le BS 3, le sSMCC, le SPDP, le glutaraldéhyde... L'une des techniques de couplage utilisées est celle qui utilise comme agent chimique de couplage le glutaraldéhyde et comme protéine porteuse la KLH. Le couplage de la KLH sur le peptide est réalisé à partir des fonctions amines du peptide (groupement N-terminal et groupement amine porté par la lysine principalement).

Une solution du peptide C13P30 de séquence YTLRAPRSPKMVQGSG-$NH_2$ (SEQ ID N° 16) ou de peptide CN32 de séquence YTLRAPRSPKMVQGS-$NH_2$ (SEQ ID N° 109) à 5mg/ml est préparée en tampon PBS Dulbecco NaCl 0,15M à pH 7,4. Un flacon de 20mg de KLH lyophilisée en tampon PBS (Pierce # 77600) est repris par 2ml d'eau PPI (eau pour préparation injectable), de façon à obtenir une solution de KLH à 10mg/ml en tampon PBS.

1 ml de la solution de peptide (soit 5mg) est mélangée avec 1,25ml de la solution de KLH (soit 12,5mg). Sous hotte aspirante, 2,25ml d'une solution de glutaraldéhyde à 2% préparée extamporanément (à partir de glutaladéhyde à 25%, Sigma # G-5882) sont ajoutés au mélange. Afin d'éviter la formation de complexes de KLH, la solution de glutaraldéhyde à 2% est ajoutée goutte à goutte et sous agitation du mélange. La réaction de conjugaison se fait pendant 2 heures 30 d'incubation à température ambiante. La réaction de couplage est stoppée par ajout d'une solution de borohydrure de sodium à 100 mg/ml de façon à atteindre une concentration finale de 10mg/ml. Le mélange est mis à incuber une nuit à 4°C. Enfin la solution est dialysée sur une nuit à 4°C contre du tampon PBS Dulbecco à pH 7,4. La solution est enfin aliquotée et conservée à -80°C.

**Exemple 3 : Immunisations avec des peptides**

Pour la production d'anticorps polyclonaux, des lapins (femelles de souche New Zealand) ont été immunisés à l'aide du peptide Cys-YTLRAPRSPKMVQGSG-$NH_2$ couplé à la KLH selon l'exemple 2. A la première injection, une émulsion de 1,5 ml de peptide couplé à la KLH avec 1,5 ml d'adjuvant de Freund complet (Sigma # F-5881) est réalisée, et 1 ml de cette dernière émulsion (soit 200 $\mu$g de peptide) est injecté par voie intradermique à chacun des lapins. Deux rappels sont réalisés à 20 jours d'intervalle en injectant par voie intradermique 1ml d'une émulsion de peptide couplé à la KLH (soit 200$\mu$g de peptide) avec de l'adjuvant de Freund incomplet (Sigma # F-5506). Vingt jours après le second rappel, un troisième rappel est effectué de la même manière que les précédents rappels mais en injection sous-cutanée. Vingt jours après ce dernier rappel, et après évaluation du titre en anticorps obtenu, les lapins sont saignés. Plus particulièrement les anticorps polyclonaux des lapins identifiés par les numéros # 046 805 et 046 832 obtenus par immunisation avec le peptide C-YTLRAPRSPKMVQGS-NH2 (SEQ ID N° 16) couplé à la KLH, et du lapin identifié # L01235, obtenus par immunisation avec le peptide C-YTLRAPRSPKMVQGS-NH2 (SEQ ID N°109) couplé à la KLH, ont été utilisés pour la suite des travaux.

**Exemple 4 : obtention et purification des anticorps**

Avant purification, les sérums de lapins sont centrifugés 30 minutes à 4 500 tours/minute à 4°C. Après décantation, le sérum est dilué de moitié dans du tampon glycine 1,5M, à pH 8,0 contenant du NaCl 1M.

**Exemple 4.a : purification des IgG sur protéine A-sépharose**

La purification des anticorps polyclonaux est réalisée par chromatographie d'affinité sur un gel de protéine A-sépharose (Amersham Biosciences #17.1279.02). La protéine A extraite de *Staphylococcus aureus* se combine spécifiquement au fragment Fc des molécules d'IgG. Ensuite les IgG, toutes sous classes confondues, sont éluées à pH 3,0.

Tous les tampons utilisés sont dégazés pendant 15 minutes dans un bain à ultrasons avant passage dans la colonne, afin de prévenir la formation de bulles.

Une colonne de chromatographie est préparée à l'aide de 12 ml de protéine A-sépharose. La colonne de gel est équilibrée pendant 40 minutes avec de l'eau distillée et dégazée, puis pendant 40 minutes avec du tampon PBS Dulbecco à pH 7,4 contenant du NaCl 0,5 M, et enfin avec du tampon glycine 1,5 M à pH 8,0 contenant du NaCl 1M, ceci jusqu'à obtention d'une ligne de base correcte.

Ensuite, 10ml de sérum de lapin dilué de moitié en tampon glycine 1,5 M à pH 8,0 contenant du NaCl 1 M, sont passés dans la colonne à un débit de 0,5ml/mn. Après apparition du pic d'albumine, les IgG du sérum sont éluées à l'aide d'une solution d'acide citrique 0,1M amenée à pH 3,0 avec du tampon tris sodium de citrate 0,1M. Le pic d'élution des IgG est récupéré et rapidement mis en dialyse en tampon PBS Dulbecco à pH 7,4 une nuit à 4°C. La concentration des IgG est déterminée après dialyse par lecture de la densité optique à 280 nm contre du tampon PBS.

Après purification sur protéine A, la réactivité des anticorps polyclonaux est testée en ELISA sur des cupules revêtues soit de proBNP(1-108), soit de BNP(1-76), soit de BNP(77-108) adsorbé à 0,25 $\mu$g/ml. Les résultats obtenus pour les anticorps polyclonaux du lapin # 046 805 sont présentés dans la figure 1. Les anticorps polyclonaux du lapin # 046 832 ont donné des résultats identiques. Les anticorps polyclonaux des lapins # 046 805 et # 046 832 sont relativement spécifiques du proBNP(1-108). Nous avons cependant pu noter la présence d'une faible réactivité anti-BNP(77-108) à des concentrations élevées, réactivité que nous avons pu supprimer en rendant les anticorps polyclonaux de ces lapins monospécifiques par épuisement sur résine BNP-$K_3$-NHS-sépharose, selon le procédé décrit dans l'exemple 4.b.

**Exemple 4.b : Epuisement des IgG sur résine BNP-$K_3$-NHS-sépharose**

Le but de cette opération est d'éliminer la réactivité croisée observée vis à vis du BNP(77-108).

La réactivité croisée de l'anticorps polyclonal obtenue étant localisée en position N-terminale de la molécule de BNP(77-108), il était important de bien présenter cette région du BNP(77-108) aux immunoglobulines à éliminer. A ces fins, le BNP(77-108) a été synthétisé avec une extension de 3 résidus de lysine en position C-terminale (BNP-$K_3$) afin de favoriser son couplage à la résine NHS-sépharose par son extrémité C-terminale.

Le BNP-$K_3$: S-P-K-M-V-Q-G-S-G-C-F-G-R-K-M-D-R-I-S-S-S-S-G-L-G-C-K-V-L-R-R-H-K-K-K (SEQ ID n° 104) a été synthétisé sur un synthétiseur Pioneer, en utilisant la chimie « Fmoc » (9-fluorénylméthyloxy carbonyl) citée ci-dessus sous l'exemple 1.

5 mg de BNP-$K_3$ sont dissous à l'aide du tampon NaHCO3 100mM, à pH 8,3 aditionné de NaCl 0,5M à une concentration de 10mg/ml. 2ml de résine NHS-sépharose (NHS-activated Sepharose 4 Fast Flow, Amerscham Biosciences # 17.0906.01) sont centrifugés 30 secondes à 1000 tours/mn à 4°C. La résine est lavée avec 15ml d'une solution d'HCl 1 mM froid. Après centrifugation et élimination de la solution d'HCl, la résine est mélangée au ligand BNP-$K_3$ à 10mg/ml. Le mélange est mis à incuber 1 heure à température ambiante sous agitation lente. Après centrifugation et élimination de la solution de ligand, les groupements non réactifs de la résine sont bloqués à l'aide de 5ml de tampon glycine 0,1M, à pH 8,0. Le mélange est mis à incuber 1 heure à température ambiante sous agitation lente. Après centrifugation et élimination du tampon de blocage, la résine est reprise par 5 ml de tampon NaHCO3 100mM, à pH 8,3 additionné de NaCl 0,5M. Quatre lavages à l'aide de ce tampon sont réalisés. Au dernier lavage, le mélange est déposé sur une colonne de chromatographie.

Après préparation de la colonne de chromatographie, 10mg d'IgG du lapin # 046 805 ou du lapin # 046 832 sont déposés sur la colonne. Une pompe péristaltique reliée à la colonne permet de faire circuler les IgG du sérum de lapin toute une nuit à 4°C. Le lendemain, la solution d'IgG est récupérée (= filtrat). La fraction d'IgG liée sur le BNP-$K_3$ est éluée (= éluat) à l'aide du tampon Tris 20mM, à pH 8,0 contenant de l'urée 5M. On teste ensuite l'éluat et le filtrat afin de s'assurer de l'efficacité de l'épuisement. Les figures 2 et 3 montrent les résultats des tests réalisés en ELISA avec le filtrat des sérums polyclonaux des lapins # 046 805 et # 046 832, respectivement. Les figures 4 et 5 montrent les résultats des tests réalisés en ELISA avec l'éluat des sérums polyclonaux des lapins # 046 805 et # 046 832, respectivement.

Comme c'était attendu, le filtrat des sérums polyclonaux des lapins # 046 805 et # 046 832 est spécifique du proBNP(1-108) : aucune réactivité n'est observée sur le BNP(1-76) ni sur le BNP(77-108). L'éluat conserve une réactivité vis à vis du proBNP(1-108) importante, mais également une réactivité vis à vis du BNP(77-108). Ces résultats confirment l'efficacité de l'épuisement du sérum polyclonal du lapin sur la résine BNP-$K_3$-NHS-sépharose.

Le BNP(77-108) provient de Sigma (# B-5900), tandis que le proBNP(1-108) et le BNP(1-76) ont été produits sous forme de protéines recombinées exprimées après clonage dans le vecteur pGEX-2T (Amersham Pharmacie Biotech) et transfection chez E coli, par les techniques classiques bien connues de l'homme du métier. Le vecteur d'origine a été fourni par la société Berlex Biosciences (Richmond, CA, USA) et sa préparation est décrite par Yan et al. (PNAS, 2000, vol. 97, pp. 8525-8529). La concentration du proBNP(1-108) et celle du BNP(1-76) ont été déterminées par la méthode Bradford de dosage colorimétrique des protéines (Bradford M. Anal. Biochem. 1976; 72:248-54).

**Exemple 5: Détermination du pourcentage de réaction croisée des anticorps anti proBNP(1-108) des lapins # 046 805 et # L01235 vis à vis du BNP(1-76) et du BNP(77-108)**

*Matériels :*

1) Phase solide : microplaque Maxisorp à fond plat, Nunc (Danemark).
2) Le BNP(77-108) provient de Sigma (# B-5900), tandis que le proBNP(1-108) et le BNP(1-76) ont été produits sous forme de protéines recombinées. La concentration de ces solutions de protéines a été déterminée par la méthode Bradford de dosage colorimétrique des protéines (Bradford M. Anal. Biochem. 1976;72:248-54).
3) Le conjugué utilisé est un anticorps polyclonal anti-IgG de lapin couplé à la peroxidase (Sigma # A-9169)
4) Tampon de saturation : tampon PBS Dulbecco à pH 7,4 contenant 1% d'albumine bovine sérique (BSA, Sigma #A-7888)
5) Tampon de dilution : tampon PBS Dulbecco à pH 7,4 contenant 0,1% de BSA et 0,1% de Tween 20.
6) Solution de lavage : tampon PBS Dulbecco à pH 7,4 contenant 0,1% de Tween 20.
7) Solution de révélation: la solution de révélation est composée :

    7a) d'un tampon substrat : Solution d'acide citrique 0,01 M, et de citrate trisodique 0,04M contenant de l'$H_2O_2$ à 0,33%, de pH final 5,6, et
    7b) d'un chromogène : comprimés d'OPD (orthophénylènediamine). 1 comprimé d'OPD à dissoudre dans 10ml de tampon substrat.

8) Solution d'arrêt : H2SO4 4N.

*Protocole :*

Le test consiste à évaluer l'immunoréactivité des anticorps polyclonaux directement sur les différentes protéines immobilisées dans les cupules d'une plaque de microtitrage.

✓ Plusieurs solutions de sensibilisation en tampon PBS Dulbecco pH 7,4 sont tout d'abord préparées : une première contenant du BNP(77-108) à 0,25μg/ml, une deuxième contenant du proBNP(1-108) à 0,25μg/ml, une troisième contenant du BNP(1-76) à 0,25μglml, et une quatrième contenant de la GST (protéine témoin bruit de fond) à 0,25μg/ml.
✓ 100μl de chacune de ces solutions sont déposés séparément dans les puits d'une microplaque.
✓ La microplaque est mise à incuber pendant une nuit à 4°C.
✓ Après élimination de la solution de sensibilisation, la microplaque est lavée à l'aide de 300 μl d'un tampon PBS Dulbecco à pH 7,4 contenant 0,1% de Tween 20, puis saturée par addition de 250μl du tampon PBS Dulbecco à pH 7,4 contenant 1% de BSA.
✓ La microplaque est alors mise à incuber pendant 1 heure à 37°C.
✓ La microplaque est ensuite lavée (3 fois) à l'aide de la solution de lavage.
✓ Dans chaque cupule, 100μl de solution d'anticorps polyclonal, préalablement dilué à 2 et 1 μg/ml pour le sérum polyclonal du lapin # 046 805 et au 1/2500 et 1/5000 pour le sérum polyclonal du lapin # L01235, sont déposés.
✓ Le milieu réactionnel est mis à incuber 2 heures à température ambiante
✓ La microplaque est ensuite lavée 3 fois avec 300μl de la solution de lavage.
✓ 100μl du conjugué anticorps polyclonal anti-IgG de lapin couplé à la peroxydase, dilué au 1/8000 en tampon de dilution, sont ajoutés dans chaque puits de la microplaque.
✓ Le milieu réactionnel est mis à incuber pendant 1 heure à température ambiante.
✓ Les plaques sont ensuite lavées (5 lavages) avec 300μl de la solution de lavage. Dans chaque cupule, 100 μl de la solution de révélation sont distribués. On laisse la réaction se développer à l'obscurité pendant 20 minutes à température ambiante (18-24°C).
✓ Ensuite 50 μl de la solution d'arrêt sont distribués dans chaque cupule.
✓ Après arrêt de la réaction, la lecture de la densité optique est effectuée au spectrophotomètre à 490/620 nm.

**Tableau I: Résultats de la détermination du pourcentage de réaction croisée des anticorps polyclonaux des lapins #046 805, et #L01235 vis à vis du BNP(1-76) et du BNP(77-108)**

Pour un anticorps anti proBNP(1-108) donné, testé sur le BNP(77-108) adsorbé à 0,25μg/ml, sur le proBNP(1-108) adsorbé à 0,25μg/ml et sur la GST adsorbée à 0,25μg/ml, on calcule le pourcentage de la réaction croisée de l'anticorps avec le BNP(77-108) à l'aide de la formule suivante:

$$\% = \frac{DO_{(BNP77\text{-}108)} - DO_{(GST)}}{DO_{(proBNP1\text{-}108)} - DO_{(GST)}} \times 100$$

Pour un anticorps anti proBNP(1-108) donné, testé sur le BNP(1-76) adsorbé à 0,26μg/ml, sur le proBNP(1-108) adsorbé à 0,25μg/ml et sur la GST adsorbée à 0,25μg/ml, on calcule le pourcentage de la réaction croisée de l'anticorps avec le BNP(1-76) à l'aide de la formule suivante :

$$\% = \frac{DO_{(BNP1\text{-}76)} - DO_{(GST)}}{DO_{(proBNP1\text{-}108)} - DO_{(GST)}} \times 100$$

| | % réaction croisée | | | |
|---|---|---|---|---|
| | sérum polyclonal #046 805 à 1 μg/ml | sérum polyclonal #046 805 à 2 μg/ml | sérum polyclonal #L01235 1/5000 | sérum polyclonal #L01235 1/2500 |
| BNP(77-108) à 0,25μg/ml | 1,44% | 2,13% | 3,55% | 4,3% |
| BNP(1-76) à 0,25μg/ml | 1,00% | 1,29% | 0,21% | 0,00% |

Conclusion : La réaction croisée des anticorps polyclonaux de ces sérums est inférieure à 2% sur le BNP(1-76) et inférieure à 5% sur le BNP(77-108). La réactivité croisée vis à vis du BNP(77-108) peut être éliminée par le procédé d'épuisement décrit dans l'exemple 4.b. Cependant, comme c'est décrit dans les exemples 19 et 20, dans les conditions d'un dosage immunoenzymométrique du proBNP(1-108), et aux concentrations de BNP(77-108) et de BNP(1-76) habituellement retrouvées chez les patients, ces anticorps polyclonaux peuvent être utilisés dans leur version non épuisée sans entraîner l'apparition de réaction croisée.

**Exemple 6 : identification de l'épitope reconnu par les sérums polyclonaux des lapins #046 805 et #046 832 avant et après épuisement sur résine BNP-$K_3$-NHS-sépharose**

Les sérums polyclonaux des lapins # 046 805 et # 046 832 avant et après épuisement sur résine BNP-$K_3$-NHS-sépharose ont été testés par la méthode spot afin d'identifier l'épitope reconnu par ces sérums polyclonaux. Cette méthode, décrite par Frank (Tetrahedron, 1992; 48:9217-32), permet la synthèse rapide sur membrane de nitrocellulose d'un grand nombre de peptides de séquences prédéfinies. Les protocoles utilisés sont ceux décrits par Molina et al. (Pept. Res., 1996; 9:151-5). Sur une feuille de papier sont créées des zones circulaires (spots) d'environ 5 mm de diamètre, comportant une fonction aminée, qui servent de point d'ancrage à l'acide aminé C-terminal du peptide synthétique. L'allongement de la chaîne peptidique s'effectue par additions successives de Fmoc-acides aminés activés. Les chaînes latérales des acides aminés sont bloquées par des groupements chimiques adéquats. Tous les peptides sont synthétisés avec leur résidu N-terminal N-acétylé. A l'issue de la synthèse, les chaînes latérales sont déprotégées par action de l'acide trifluoroacétique. Ce traitement n'affecte pas la liaison entre le peptide et le support cellulosique, et la réactivité des peptides peut être évaluée par un essai colorimétrique.

La série de peptides synthétisés sur membrane est constituée de 32 pentadécapeptides décalés de 3 résidus d'acides aminés représentant la totalité de la séquence du proBNP(1-108).

**Tableau II : Membrane spot constituée de 32 pentadécapeptides décalés de 3 en 3 résidus d'acides aminés représentant la totalité de la séquence du proBNP(1-108).**

| Séquence | N° spot | Séquence | N° spot |
|---|---|---|---|
| HPLGSPGSASDLETS (SEQ ID n°23) | 1 | GVWKSREVATEGIRG (SEQ ID n° 39) | 17 |
| GSPGSASDLETSGLQ (SEQ ID n° 24) | 2 | KSREVATEGIRGHRK (SEQ ID n° 40) | 18 |
| GSASDLETSGLQEQR (SEQ ID n° 25) | 3 | EVATEGIRGHRKMVL (SEQ ID n° 41) | 19 |
| SDLETSGLQEQRNHL (SEQ ID n° 26) | 4 | TEGIRGHRKMVLYTL (SEQ ID n° 42) | 20 |
| ETSGLQEQRNHLQGK (SEQ ID n° 27) | 5 | IRGHRKMVLYTLRAP (SEQ ID n° 43) | 21 |
| GLQEQRNHLQGKLSE (SEQ ID n° 28) | 6 | HRKMVLYTLRAPRSP (SEQ ID n° 44) | 22 |
| EQRNHLQGKLSELQV (SEQ ID n° 29) | 7 | MVLYTLRAPRSPKMV (SEQ ID n° 45) | 23 |
| NHLQGKLSELQVEQT (SEQ ID n° 30) | 8 | YTLRAPRSPKMVQGS (SEQ ID n° 46) | 24 |
| QGKLSELQVEQTSLE (SEQ ID n° 31) | 9 | RAPRSPKMVQGSGCF (SEQ ID n° 47) | 25 |
| LSELQVEQTSLEPLQ (SEQ ID n° 32) | 10 | RSPKMVQGSGCFGRK (SEQ ID n° 48) | 26 |
| LQVEQTSLEPLQESP (SEQ ID n° 33) | 11 | KMVQGSGCFGRKMDR (SEQ ID n° 49) | 27 |
| EQTSLEPLQESPRPT (SEQ ID n° 34) | 12 | QGSGCFGRKMDRISS (SEQ ID n° 50) | 28 |

(suite)

| Séquence | N° spot | Séquence | N° spot |
|---|---|---|---|
| SLEPLQESPRPTGVW (SEQ ID n° 35) | 13 | GCFGRKMDRISSSSG (SEQ ID n° 51) | 29 |
| PLQESPRPTGVWKSR (SEQ ID n° 36) | 14 | GRKMDRISSSSGLGC (SEQ ID n° 52) | 30 |
| ESPRPTGVWKSREVA (SEQ ID n° 37) | 15 | MDRISSSSGLGCKVL (SEQ ID n° 53) | 31 |
| RPTGVWKSREVATEG (SEQ ID n° 38) | 16 | ISSSSGLGCKVLRRH (SEQ ID n° 54) | 32 |

Après saturation de la membrane à l'aide de 30 ml du tampon TBS (Tris-Buffer Saline) à pH 7,0 additionné de Tween 20 à 0,1%, de Blocking buffer à 5% (Euromedex # SU-07-250), et de saccharose à 5%, la réactivité de 20 ml du sérum polyclonal de lapin dilué à 10µg/ml est testée (sur une incubation de 1 heure 30 minutes à 37°C sous agitation). Après lavage avec le tampon TBS à pH 7,0 additionné de Tween 20 à 0,1%, 20 ml de conjugué anti-IgG de lapin couplé à la phosphatase alcaline (Sigma # A-8025) sont mis à incuber 1 heure à température ambiante sous agitation. Enfin, après une dernière série de lavages avec 30 ml de solution de lavage, la révélation des spots est réalisée par addition de 30 ml de solution de substrat (solution de 30ml de tampon CBS (Citrate Buffer Saline) à pH 7,0 contenant 120µl de BCIP (5-bromo-4-chloro-3-Indolyl phosphate) 0,15M, 150µl de $MgCl_2$ 1M et 180µl de MTT (Thiazolyl blue tetrazolium bromide) 0,1M. Après scanérisation de la membrane, l'intensité des spots de la membrane est évaluée (en unités - d'intensité relatives) à l'aide d'un logiciel de traitement d'image. Le bruit de fond est calculé à partir des spots non détectés par l'antisérum. Les résultats de l'analyse épitopique du sérum polyclonal du lapin # 046 805 avant épuisement sont présentés dans la figure 6. Cinq peptides (spots 22 à 26) sont détectés par le sérum polyclonal, et la séquence commune à ces cinq peptides est $R_{76}S_{77}P$. Cependant, la réactivité est nettement augmentée lorsque le motif RAP se rajoute en position N-terminale du motif $R_{76}S_{77}P$. Les résultats de l'analyse épitopique du sérum polyclonal du lapin # 046 805 après épuisement sur résine BNP-$K_3$-NHS-sépharose sont présentés dans la figure 7. Quatre peptides (spots 22 à 25) sont détectés par le sérum polyclonal, et la séquence commune à ces 4 peptides est $RAPR_{76}S_{77}P$. Contrairement à ce qui est observé pour le sérum polyclonal avant épuisement, aucune réactivité sur le motif $R_{76}S_{71}P$ seul n'est observé (spot 26). Ceci explique qu'après épuisement sur résine BNP-$K_3$-NHS-sépharose, le sérum polyclonal ne détecte plus le BNP(77-108) [pour rappel, le motif $S_{77}P$ correspond aux deux premiers acides aminés de la séquence du BNP(77-108)]. En conclusion l'épitope reconnu par le sérum polyclonal du lapin # 046 805 rendu monospécifique est $RAPR_{76}S_{77}P$.

Des résultats tout à fait identiques ont été obtenus à l'aide du sérum polyconal du lapin # 046 832 : l'épitope reconnu par le sérum polyclonal de ce lapin rendu monospécifique est également $RAPR_{76}S_{77}P$.

**Exemple 7 : identification de l'épitope minimum des sérums polyclonaux des lapins # 046 805 et # 046 832 avant et après épuisement, par la méthode de l'Ala-scan**

Les sérums polyclonaux des lapins # 046 805 et # 046 832, avant et après épuisement sur résine BNP-$K_3$-NHS-sépharose, ont été testés par la méthode spot (décrite dans l'exemple 6) afin d'identifier l'épitope minimum, dans le peptide charnière, permettant la reconnaissance spécifique du seul proBNP(1-108) par les sérums polyclonaux. On a synthétisé une membrane constituée de 16 pentadécapeptides reprenant la séquence du peptide charnière YTLRAPRSPKMVQGS et porteurs d'une substitution de proche en proche d'un acide aminé par un résidu alanine (Alanine-scanning ou « Ala-scan ») ou glycine, substitution à chaque fois décalée vers la droite d'un résidu d'acide aminé (tableau III). Les résultats de l'analyse Ala-scan des sérums polyclonaux des lapins # 046 805 et # 046 832 avant et après épuisement sur résine BNP-$K_3$-NHS-sépharose sont présentés dans le tableau III.

**Tableau III : membrane spot à 16 pentadécapeptides YTLRAPRSPKMVQGS porteurs d'une substitution de proche en proche de chaque acide aminé par un résidu alanine ou glycine. Réactivité des sérums polyclonaux des lapins # 046 805 et # 046 832 avant et après épuisement sur résine BNP-$K_3$-NHS-sépharose.**

| SEQ ID | Séquence | N° spot | Réactivité (en unités d'intensité relatives) sérum polyclonal lapin # 046 805 non épuisé | Réactivité (en unités d'intensité relatives) sérum polyclonal lapin # 046 805 épuisé | Réactivité (en unités d'intensité relatives) sérum polyclonal lapin# 046 832 non épuisé | Réactivité (en unités d'intensité relatives) sérum polyclonal lapin # 046 832 épuisé |
|---|---|---|---|---|---|---|
| SEQ ID n° 46 | YTLRAPRSPKMV QGS | 716 | 84 | 69 | 151 | 132 |
| SEQ ID n° 55 | ATLRAPRSPKMV QGS | 717 | 90 | 68 | 148 | 130 |
| SEQ ID n° 56 | YALRAPRSPKMV QGS | 718 | 101 | 71 | 154 | 130 |
| SEQ ID n° 57 | YTARAPRSPKMV QGS | 719 | 108 | 77 | 152 | 133 |
| SEQ ID n° 58 | YTLAAPRSPKMV QGS | 720 | 101 | **39** | 148 | **106** |
| SEQ ID n° 59 | YTLRGPRSPKMV QGS | 721 | 93 | **39** | 156 | 132 |
| SEQ ID n° 60 | YTLRAARSPKMV QGS | 722 | 103 | 96 | 137 | **48** |
| SEQ ID n° 61 | YTLRAPASPKMV QGS | 723 | 50 | **23** | 150 | **116** |
| SEQ ID n° 62 | YTLRAPRAPKMV QGS | 724 | 58 | **44** | 148 | **113** |
| SEQ ID n° 63 | YTLRAPRSAKMV QGS | 725 | **74** | **41** | **85** | **67** |
| SEQ ID n° 64 | YTLRAPRSPAMV QGS | 726 | 138 | 118 | 157 | 148 |
| SEQ ID n° 65 | YTLRAPRSPKAV QGS | 727 | 98 | 77 | 150 | 130 |
| SEQ ID n° 66 | YTLRAPRSPKMA QGS | 728 | 100 | 68 | 138 | 130 |

(suite)

| SEQ ID | Séquence | N° spot | Réactivité (en unités d'intensité relatives) sérum polyclonal lapin # 046 805 non épuisé | Réactivité (en unités d'intensité relatives) sérum polyclonal lapin # 046 805 épuisé | Réactivité (en unités d'intensité relatives) sérum polyclonal lapin# 046 832 non épuisé | Réactivité (en unités d'intensité relatives) sérum polyclonal lapin # 046 832 épuisé |
|---|---|---|---|---|---|---|
| SEQ ID n° 67 | YTLRAPRSPKMV AGS | 729 | 95 | 71 | 142 | 133 |
| SEQ ID n° 68 | YTLRAPRSPKMV QAS | 730 | 107 | 78 | 143 | 134 |
| SEQ ID n° 69 | YTLRAPRSPKMV QGA | 731 | 112 | 68 | 153 | 132 |
| NB : les résidus alanine ou glycine substitués aux acides aminés initiaux sont soulignés (A et G). | | | | | | |

Les acides aminés critiques dans la reconnaissance de l'épitope reconnu par le sérum polyclonal du lapin # 046 805 avant épuisement sont $R_{76}S_{77}P$, tandis qu'après épuisement du sérum polyclonal du lapin # 046 805 sur résine BNP-$K_3$-NHS-sépharose, outre le motif $R_{76}S_{77}P$, le motif RA devient contributeur. Pour le sérum polyclonal du lapin # 046 832 avant épuisement, seule la proline $P_{78}$ est contributrice, tandis qu'après épuisement du sérum polyclonal du lapin # 046 832 sur résine BNP-$K_3$-NHS-sépharose, le motif contributeur est R-$PR_{76}S_{77}P$. Ces résultats montrent donc que l'épitope minimum obligatoire dans la reconnaissance spécifique du proBNP(1-108) par les anticorps polyclonaux des lapins # 046 805 et #046 832 est $RAPR_{76}S_{77}P$,

**Exemple 8 : identification de l'épitope minimum du sérum polyclonal du lapin #L01235, par la méthode de l'Ala-scan**

Le sérum polyclonal du lapin # L01235, spécifique d'emblée du proBNP(1-108) (épuisement inutile), a été testé par la méthode spot (décrite dans l'exemple 6) afin d'identifier l'épitope minimum, dans le peptide charnière, permettant la reconnaissance spécifique du seul proBNP(1-108) par le sérum polyclonal. La membrane utilisée est celle décrite dans l'exemple 7. Les résultats de l'analyse Ala-scan du sérum polyclonal du lapin # L01235 sont présentés dans le tableau IV.

**Tableau IV : membrane spot à 16 pentadécapeptides YTLRAPRSPKMVQGS porteurs d'une substitution de proche en proche de chaque acide aminé par un résidu alanine ou glycine. Réactivité du sérum polyclonal du lapin # L01235.**

| SEQ ID | Séquence | Réactivité (en unités d'intensité relatives) sérum polyclonal du lapin # L01235 |
|---|---|---|
| SEQ ID n° 46 | YTLRAPRSPKMVQGS | 94 |
| SEQ ID n° 55 | ATLRAPRSPKMVQGS | 91 |
| SEQ ID n° 56 | YALRAPRSPKMVQGS | 84 |
| SEQ ID n° 57 | YTARAPRSPKMVQGS | 92 |
| SEQ ID n° 58 | YTLAAPRSPKMVQGS | **66** |
| SEQ ID n° 59 | YTLRGPRSPKMVQGS | 71 |
| SEQ ID n° 60 | YTLRAARSPKMVQGS | **67** |

(suite)

| SEQ ID | Séquence | Réactivité (en unités d'intensité relatives) sérum polyclonal du lapin # L01235 |
|---|---|---|
| SEQ ID n° 61 | YTLRAPASPKMVQGS | 71 |
| SEQ ID n° 62 | YTLRAPRAPKMVQGS | **47** |
| SEQ ID n° 63 | YTLRAPRSAKMVQGS | **53** |
| SEQ ID n° 64 | YTLRAPRSPAMVQGS | 102 |
| SEQ ID n° 65 | YTLRAPRSPKAVQGS | 73 |
| SEQ ID n° 66 | YTLRAPRSPKMAQGS | 70 |
| SEQ ID n° 67 | YTLRAPRSPKMVAGS | 70 |
| SEQ ID n° 68 | YTLRAPRSPKMVQAS | 83 |
| SEQ ID n° 69 | YTLRAPRSPKMVQGA | 80 |
| NB : les résidus alanine ou glycine substitués aux acides aminés initiaux sont soulignés (A et G). | | |

Comme pour les sérums polyclonaux des lapins # 046 805 et # 046 832 (exemple 7), ces résultats montrent que l'épitope minimum obligatoire dans la reconnaissance spécifique du proBNP(1-108) par les anticorps polyclonaux du lapin # L01235 est $RAPR_{76}S_{77}P$.

**Exemple 9 : Immunisation de souris avec la protéines recombinée proBNP(1-108)**

On a immunisé des souris de souche BALB/c (femelles âgées de 6 semaines) à l'aide du proBNP(1-108) recombiné décrit dans l'exemple 4.b, par les techniques classiques bien connues de l'homme du métier. A la première injection, une émulsion de 1 ml de proBNP(1-108) avec 1ml d'adjuvant de Freund complet (Sigma # F-5881) est réalisée, et 300μl de cette émulsion (soit 100μg de protéine) sont injectés par voie sous-cutanée à chacune des souris. Deux rappels sont réalisés à 15 jours d'intervalle par injection par voie intrapéritonéale de 300μl d'une émulsion de proBNP (1-108) (soit 100μg de protéine) avec de l'adjuvant de Freund incomplet (Sigma # F-5506). Quinze jours après le second rappel, un troisième rappel est effectué de la même manière que les précédents rappels mais en injection sous-cutanée. Enfin, 15 jours après ce dernier rappel, on réalise des prélèvements sanguins sur les souris pour analyser la réponse immunitaire par la technique spot. La réponse immunitaire de la souris 12 s'est avérée particulièrement intéressante pour la suite des travaux.

**Exemple 10 : Identification des épitopes reconnus par les anticorps polyclonaux de la souris 12 sur la séquence du proBNP(1-108)**

La méthode spot utilisée pour identifier les épitopes reconnus par les anticorps polyclonaux de la souris 12 sur la séquence du proBNP(1-108) est décrite dans l'exemple 6.

La série de peptides synthétisés sur membrane est constituée de 94 pentadécapeptides, décalés chacun d'un résidu d'acide aminé, représentant la totalité de la séquence du proBNP(1-108). La réactivité du sérum polyclonal de la souris 12 dilué au 1/500ème est testée sur cette membrane comme c'est décrit dans l'exemple 6. Après scanérisation de la membrane, l'intensité des spots de la membrane est évaluée (en unités d'intensité relatives) à l'aide d'un logiciel de traitement d'image. Le bruit de fond, calculé à partir des spots non détectés par antisérum, était de 30 unités d'intensité relatives.

Trois régions situées en position N-terminale de la séquence du proBNP(1-108) sont ainsi particulièrement bien détectées par les anticorps de l'antisérum de la souris 12 : séquence $H_1PLGSPGSASDLETS_{15}$ (SEQ ID N° 23) (spots 1 à 12), séquence $L_{17}QEQRNHLQGK_{27}$ (SEQ ID N° 123) (spots 17 à 27) et séquence $L_{38}EPLQESPRPTG_{49}$ (SEQ ID N° 124) (spots 38 à 49).

De façon surprenante, antisérum de la souris 12 contient également des anticorps capables de reconnaître des spots dont la séquence peptidique comprend le motif $RAPR_{76}S_{77}P$ : spots 64 à 68. Les séquences peptidiques des spots sont décrites dans le tableau V.

**Tableau V : Epitopes de la région charnière reconnus, à l'aide de la technique spot, par l'antisérum de la souris 12.**

| N° spot | séquence peptidique du spot détecté | réactivité en unités relatives d'intensité | N° spot | séquence peptidique du spot détecté | réactivité en unités relatives d'intensité |
|---|---|---|---|---|---|
| 1 | HPLGSPGSASDLETS (SEQ ID n° 23) | 177,0 | 38 | LEPLQESPRPTGVWK (SEQ ID n° 88) | 177,0 |
| 2 | PLGSPGSASDLETSG (SEQ ID n° 70) | 170,0 | 39 | EPLQESPRPTGVWKS (SEQ ID n° 89) | 188,0 |
| 3 | LGSPGSASDLETSGL (SEQ ID n° 71) | 145,7 | 40 | PLQESPRPTGVWKSR (SEQ ID n° 90) | 109,0 |
| 4 | GSPGSASDLETSGLQ (SEQ ID n° 24) | 166,3 | 41 | LQESPRPTGVWKSRE (SEQ ID n° 91) | 166,0 |
| 5 | SPGSASDLETSGLQE (SEQ ID n° 72) | 185,0 | 42 | QESPRPTGVWKSREV (SEQ ID n° 92) | 164,0 |
| 6 | PGSASDLETSGLQEQ (SEQ ID n° 73) | 169,3 | 43 | ESPRPTGVWKSREVA (SEQ ID n° 93) | 147,0 |
| 7 | GSASDLETSGLQEQR (SEQ ID n° 25) | 155,2 | 44 | ESPRPTGVWKSREVA (SEQ ID n° 93) | 149,0 |
| 8 | SASDLETSGLQEQRN (SEQ ID n° 74) | 176,5 | 45 | SPRPTGVWKSREVAT (SEQ ID n° 94) | 175.0 |
| 9 | ASDLETSGLQEQRNH (SEQ ID n° 75) | 108,0 | 46 | PRPTGVWKSREVATE (SEQ ID n° 95) | 186,0 |
| 10 | SDLETSGLQEQRNHL (SEQ ID n° 76) | 118,3 | 47 | RPTGVWKSREVATEG (SEQ ID n° 16) | 171,0 |
| 11 | DLETSGLQEQRNHLQ (SEQ ID n° 77) | 121,0 | 48 | PTGVWKSREVATEGI (SEQ ID n° 96) | 148,0 |
| 12 | LETSGLQEQRNHLQG (SEQ ID n° 78) | 101,0 | 49 | TGVWKSREVATEGIR (SEQ ID n° 97) | 72,0 |
| 17 | LQEQRNHLQGKLSEL (SEQ ID n° 79) | 99,0 | 61 | IRGHRKMVLYTLRAP (SEQ ID n° 98) | 105,8 |
| 18 | QEQRNHLQGKLSELQ (SEQ ID n° 80) | 179,8 | 82 | RGHRKMVLYTLRAPR (SEQ ID n° 99) | 97,7 |
| 19 | EQRNHLQGKLSELQV (SEQ ID n° 29) | 189,6 | 63 | GHRKMVLYTLRAPRS (SEQ ID n° 100) | 95,9 |
| 20 | QRNHLQGKLSELQVE (SEQ ID n° 81) | 211,8 | 64 | HRKMVLYTLRAPRSP (SEQ ID n° 53) | 92,8 |
| 21 | RNHLQGKLSELQVEQ (SEQ ID n° 82) | 219,0 | 65 | RKMVLYTLRAPRSPK (SEQ ID n° 101) | 48,0 |
| 22 | NHLQGKLSELQQVEQT (SEQ ID n° 30) | 213,3 | 66 | KMVLYTLRAPRSPKM (SEQ ID n° 102) | 48,0 |
| 23 | HLQGKLSELQVEQTS (SEQ ID n° 83) | 202,6 | 67 | MVLYTLRAPRSPKMV (SEQ ID n° 45) | 42,1 |
| 24 | LQGKLSELQVEQTSL (SEQ ID n° 84) | 189,5 | 68 | VLYTLRAPRSPKMVQ (SEQ ID n° 103) | 38,4 |
| 25 | QGKLSELQVEQTSLE (SEQ ID n° 85) | 187,3 | | | |

(suite)

| N° spot | séquence peptidique du spot détecté | réactivité en unités relatives d'intensité | N° spot | séquence peptidique du spot détecté | réactivité en unités relatives d'intensité |
|---|---|---|---|---|---|
| 26 | GKLSELQVEQTSLEP (SEQ ID n° 86) | 36,6 | | | |
| 27 | KLSELQVEQTSLEPL (SEQ ID n° 87) | 69,9 | | | |

**Exemple 11 : Obtention d'anticorps monoclonaux reconnaissant de façon spécifique le proBNP(1-108), à l'exclusion substantielle du BNP(1-76) et du BNP(77-108)**

La souris (femelle BALB/c de 5 semaines) sélectionnée pour la production d'anticorps monoclonaux a été immunisée avec le peptide SEQ ID N° 16 C-YTLRAPRSPKMVQGSG-NH2 (C13P30) couplé à la KLH (Keyhole LimpetHemocyanin) selon le protocole suivant : 100μg du peptide couplé à la KLH dilué volume à volume avec de l'adjuvant complet de Freund ont été injectés par voie sous-cutanée. Quatre rappels ont été effectués à un intervalle de 3 semaines avec 100μg du peptide couplé à la KLH dilué volume à volume avec de l'adjuvant incomplet de Freund, injectés par voie sous-cutanée.

Trois jours avant la réalisation de la fusion lymphocytaire, la souris a subi une hyper-immunisation selon le protocole suivant : la dose totale d'immunogène, ici 100μg de peptide C-YTLRAPRSPKMVQGSG-NH2 couplé à la KLH en tampon PBS stérile, est fractionnée en 4 injections. La première et la seconde injections correspondent chacune à 1/10ème de la dose totale. Ces injections sont réalisées par voie sous-cutanée en différents endroits et à 45 minutes d'intervalle. La troisième injection, qui correspond aux 2/10èmes de la dose totale, est réalisée 45 minutes après la seconde injection, par voie sous-cutanée. Trente minutes après la troisième injection, une injection par voie intrapéritonéale de 100μl d'une solution de prométhazine à 1mg/ml en PBS stérile (Phénergan à 2,5%, Laboratoires Medeva Parma) est réalisée afin de prévenir tout choc anaphylactique. Enfin 15 minutes après, la dernière injection correspondant aux 6/10èmes de la dose totale est effectuée par voie intrapéritonéale.

L'hybridation lymphocytaire est réalisée selon la méthode décrite par Koëhler et Milstein (Nature, 1975; 256: 495-97). Elle est réalisée à partir des cellules lymphocytaires extraites de la rate de la souris et des cellules myélomateuses (P3-X63-Ag8.653) préalablement mises en culture en milieu RPMI 1640 (Bio-Whittaker # BE12-167F), complémenté avec un mélange de L-glutamine, pénicilline et streptomycine (Sigma # G-6784), additionné de 10% de sérum de veau foetal préalablement décomplémenté (Bio-Whittaker #BE02701E) et de 8-azaguanine (Sigma # A-8526). Les cellules lymphocytaires et les cellules myélomateuses, préalablement placées en milieu RPMI 1640 (Bio-Whittaker # BE12-167F) complémenté avec un mélange de L-glutamine, pénicilline et streptomycine (Sigma # G-6784) sans addition de sérum de veau foetal, sont mélangées à raison de 5 cellules lymphocytaires pour 1 cellule myélomateuse. Après centrifugation du mélange 7 minutes à 900 tr/mn à température ambiante, et remise en suspension du culot cellulaire, 1ml de polyéthylène glycol Hybri-Max® (Sigma # P-7777) est ajouté. Après 1 minute d'incubation au bain marie à 37°C, les cellules sont centrifugées pendant 1 minute et trente secondes à 1000 tr/mn à température ambiante. Enfin après incubation de 2 minutes au bain marie à 37°C, le culot est remis en suspension et 6 ml de milieu RPMI 1640 (Bio-Whittaker # BE12-167F) complémenté avec un mélange de L-glutamine, pénicilline et streptomycine (Sigma # G-6784) préalablement placé à 37°C, sont ajoutés à raison de 100μl toutes les 5 secondes, et 9ml de ce même milieu sont ajoutés en une fois. Après centrifugation pendant 10 minutes à 900 tr/mn à TA, et élimination du surnageant, le culot est repris par du milieu RPMI 1640 (Bio-Whittaker # BE12-167F), complémenté avec un mélange de L-glutamine, pénicilline et streptomycine (Sigma # G-6784), additionné de 15% de sérum de veau foetal préalablement décomplémenté (Bio-Whittaker # BE02701 E) et de HAT (Hypoxanthine, aminoptérine, thymidine Sigma # H-0262), de façon à distribuer, sous 100μl, 120 000 cellules par puits. La solution est déposée sous 100μl dans les puits des plaques de culture à 96 puits préalablement ensemencés de macrophages murins. Les plaques sont ensuite placées dans un incubateur à $CO_2$. Quinze jours après l'hybridation lymphocytaire, le nombre de clones présents dans les plaques de fusion est estimé et exprimé en pourcentage de développement des hybridomes.

La sélection des hybridomes est effectuée en ELISA sur proBNP(1-108), BNP(1-76), BNP(77-108) et un peptide porteur de la séquence $RAPR_{76}S_{77}P$ (C13P30). Seuls les hybridomes sécrétant des anticorps capables de détecter le proBNP(1-108) et le peptide C13P30 porteur de la séquence $RAPR_{76}S_{77}P$ et ne reconnaissant substantiellement pas le BNP(1-76) ni le BNP(77-108), sont retenus. Les hybridomes sélectionnés sont maintenus en culture et clonés en dilution limite. Les hybridomes ainsi clonés peuvent ensuite être utilisés pour la production de l'anticorps monoclonal en liquide d'ascite.

C'est ainsi que les inventeurs ont produit un hybridome murin, le clone 3D4, sécrétant une immunoglobuline

d'isotype $IgG_1\kappa$ présentant les caractéristiques des anticorps selon l'invention. Cet hybridome a été déposé le 31 juillet 2003 à la CNCM (Collection Nationale de Cultures de Microorganismes, Institut Pasteur, 25, rue du Docteur Roux, 75 724 Paris, Cedex 15, France) sous le numéro d'enregistrement CNCM I-3073.

L'invention a donc encore pour objets l'hybridome 3D4 déposé à la CNCM sous le numéro d'enregistrement CNCM I-3073 et l'anticorps monoclonal qu'il sécrète.

**Exemple 12 : Validation en ELISA de la spécificité de l'anticorps produit par l'hybridome 3D4**

*Matériels :*

1) Phase solide : microplaque Maxisorp à fond plat, Nunc (Danemark).
2) Le BNP(77-108) provient de Sigma (# B-5900), tandis que le proBNP(1-108) et le BNP(1-76) ont été produits sous forme de protéines recombinées. La concentration de ces solutions de protéines a été déterminée par la méthode Bradford de dosage colorimétrique des protéines (Bradford M. Anal. Biochem. 1976;72:248-54-).
3) Le conjugué utilisé est un anticorps polyclonal d'âne anti-IgG de souris couplé à la peroxydase (Jackson Immunoresearch # 715-035-150)
4) Tampon de saturation : tampon PBS Dulbecco à pH 7,4 contenant 1% d'albumine bovine sérique (BSA, Sigma # A-7888)
5) Tampon de dilution : tampon PBS Dulbecco à pH 7,4 contenant 0,1% de BSA et 0,1% de Tween 20.
6) Solution de lavage : tampon PBS Dulbecco à pH 7,4 contenant 0,1% de Tween 20.
7) Solution de révélation: la solution de révélation est composée :

   7a) d'un tampon substrat : Solution d'acide citrique 0,01M, et de citrate trisodique 0,04M contenant de l'$H_2O_2$ à 0,33%, de pH final 5,6, et
   7b) d'un chromogène : comprimés d'OPD (orthophénylènediamine). 1 comprimé d'OPD à dissoudre dans 10ml de tampon substrat.

8) Solution d'arrêt : H2SO4 4N.

*Protocole :*

Le test consiste à évaluer l'immunoréactivité du surnageant de culture de l'hybridome 3D4 directement sur les différentes protéines immobilisées dans les cupules d'une plaque de microtitration.

✓ Plusieurs solutions de sensibilisation en tampon PBS Dulbecco pH 7,4 sont tout d'abord préparées : une première contenant du BNP(77-108) à 1µg/ml, une deuxième contenant du proBNP(1-108) à 1µg/ml, et une troisième contenant du BNP(1-76) à 1µg/ml.
✓ 100µl de chacune de ces solutions sont déposés séparément dans les puits d'une microplaque.
✓ La microplaque est mise à incuber pendant une nuit à 4°C.
✓ Après élimination de la solution de sensibilisation, la microplaque est lavée à l'aide d'un tampon PBS Dulbecco à pH 7,4 contenant 0,1% de Tween 20, puis saturée par addition de 250µl du tampon PBS Dulbecco à pH 7,4 contenant 1 % de BSA.
✓ La microplaque est alors mise à incuber pendant 1 heure à 37°C.
✓ La microplaque est ensuite lavée (3 fois) avec 300 µl de la solution de lavage.
✓ Dans chaque cupule, 100µl de surnageant de l'hybridome 3D4, préalablement dilué au 1/2 dans le tampon de dilution, sont déposés.
✓ Le milieu réactionnel est mis à incuber 2 heures à température ambiante
✓ La microplaque est ensuite lavée 3 fois avec 300µl de la solution de lavage.
✓ 100 µl de conjugué, anticorps polyclonal anti-IgG de souris couplé à la peroxydase dilué au 1/2000 en tampon de dilution, sont ajoutés à chaque puits de la microplaque.
✓ Le milieu réactionnel mis à incuber pendant 1 heure à température ambiante.
✓ Les plaques sont ensuite lavées (5 lavages) avec 300µl de la solution de lavage. Dans chaque cupule, 100 µl de la solution de révélation sont distribués. On laisse la réaction se développer à l'obscurité pendant 20 minutes à température ambiante (18-24°C).
✓ Ensuite 50 µl de la solution d'arrêt sont distribués dans chaque cupule.
✓ Après arrêt de la réaction, la lecture de la densité optique est effectuée au spectrophotomètre à 490/620 nm.

La figure 8 illustre le résultat de ce test : l'anticorps produit dans le surnageant de l'hybridome 3D4 est capable de détecter uniquement le proBNP(1-108) et le peptide d'immunisation C13P30, aucune réactivité n'est obtenue sur le BNP(1-76) et le BNP(77-108). Ces résultats valident la spécificité de l'anticorps produit par l'hybridome 3D4 pour le proBNP(1-108) à l'exclusion du BNP(1-76) et du BNP(77-108).

L'anticorps selon l'invention issu de l'hybridome 3D4 est donc bien un anticorps reconnaissant de façon spécifique le proBNP(1-108), à l'exclusion substantielle du BNP(1-76) et du BNP(77-108).

**Exemple 13 : Validation en western-blot de la spécificité de l'anticorps produit par l'hybridome 3D4**

*Matériels :*

1) Appareillage d'électrophorèse SDS-PAGE classique
2) Gel de concentration : acrylamide à 5%
3) Gel de séparation : acrylamide à 16%
4) Tampon anode : Tris 0,2M, pH 8,9
5) Tampon cathode : Tris-tricine 0,1M ; SDS à 0,1%
6) Tampon de l'échantillon : Tris 0,5M, pH 6,8 ; glycérol à 25% ; SDS à 2% ; béta-mercaptoéthanol 14,4mM ; bleu de bromophénol à 0,1%
7) Appareil de transfert électrophorétique
8) Tampon de transfert : Tris base 25 mM ; glycine 190 mM ; méthanol à 20% ; SDS à 0,05%
9) Le BNP(77-108) provient de Sigma (# B-5900), tandis que le proBNP(1-108)-GST, le BNP(1-76)-GST et la GST (utilisée comme témoin négatif) ont été produits sous forme de protéines recombinées. La concentration de ces solutions de protéines a été déterminée par la méthode Bradford de dosage colorimétrique des protéines (Bradford M. Anal. Biochem. 1976;72:248-54).
10) Le conjugué utilisé est un anticorps polyclonal d'âne anti-IgG de souris couplé à la peroxydase (Jackson Immunoresearch # 715-035-150)
11) Trousse ECL de detection en western-blot (Amersham Biosciences # RPN2106)

*Protocole :*

La mise oeuvre de ce test comprend trois grandes étapes : la migration électrophorétique des différentes protéines dans un gel d'acrylamide à 16%, ensuite le transfert de ces protéines sur une membrane de nitrocellulose, et enfin le westem-blot.

✓ Diverses solutions sont préparées en tampon d'échantillon sous un volume final de 35 μl : une première comprenant 1μg de proBNP(1-108)-GST, une deuxième comprenant 1μg de GST, une troisième comprenant 1μg de BNP(1-76)-GST, et une quatrième comprenant 1μg de BNP(77-108).
✓ Chaque solution est mise a incubée au bain marie pendant 5 minutes à 100°C, puis déposée dans un puits du gel. La migration au travers le gel d'acrylamide est réalisée sous voltage constant à 120 V pendant 1 heure 30. La figure 9 correspond à une photographie du gel d'acrylamide obtenu. Les protéines déposées sont colorées par le bleu de coomassie.
✓ Après migration, les protéines du gel sont mises à transférer sur une membrane de nitrocellulose durant 1 heure sous 120 mA.
✓ La membrane de nitrocellulose est ensuite lavée avec du tampon PBS + Tween à 0,1%, et mise à saturer pendant 30 minutes à température ambiante en tampon PBS + Tween à 0,1% + lait écrémé à 2%
✓ Après 3 lavages avec du tampon PBS + Tween 0,1%, la membrane est mise à incuber pendant 1 heure à +37°C, sous agitation, avec 5 ml de surnageant de l'hybridome 3D4.
✓ Après 3 lavages avec le tampon PBS + Tween à 0,1%, la membrane est mise à incuber pendant 1 heure à température ambiante sous agitation avec 10 ml de conjugué anti-IgG de souris couplé à la peroxydase dilué au 1/2000 en tampon PBS + Tween 0,1% + lait écrémé à 2%
✓ Après 3 lavages avec le tampon PBS + Tween 0,1%, la membrane est trempée dans le réactif de révélation ECL, avant d'être exposée à un film photographique pendant 4 minutes.

Comme le montre la figure 10 qui correspond à l'image scannée de la photographie obtenue, l'anticorps produit dans le surnageant de l'hybridome 3D4 est capable de détecter uniquement la bande correspondant au proBNP(1-108). Le BNP(1-76), le BNP(77-108), ainsi que la GST ne sont pas détectées par l'anticorps de l'hybridome 3D4. Ces résultats valident également la spécificité l'anticorps produit par l'hybridome 3D4 pour le proBNP(1-108).

L'anticorps selon l'invention issu de l'hybridome 3D4 est donc bien un anticorps reconnaissant de façon spécifique le proBNP(1-108), à l'exclusion substantielle du BNP(1-76) et du BNP(77-108).

**Exemple 14 : Identification de l'épitope reconnu par l'anticorps produit par l'hybridome 3D4 par la méthode spot**

Le surnageant de l'hybridome 3D4 a été testé par la méthode spot (décrite dans l'exemple 6) afin d'identifier l'épitope reconnu par l'anticorps de l'hybridome 3D4. Les résultats obtenus indiquent que l'anticorps 3D4 reconnait effectivement des séquences peptidiques comprenant le motif $RAPR_{76}S_{77}P$.

Ces résultats sont en accord avec ceux obtenus en ELISA (exemple 12) et western-blot (exemple 13) validant la spécificité de l'anticorps 3D4 pour le proBNP(1-108).

**Exemple 15 : dosage radioimmunométrique du proBNP(1-108)**

*Matériels :*

1) Phase solide : microplaque Maxisorp à fond plat et puits sécables, Nunc (Danemark).
2) L'anticorps de capture utilisé est l'anticorps polyclonal obtenu à partir du sérum du lapin # 046 805 non épuisé.
3) Le conjugué utilisé est un anticorps anti-BNP(77-108) marqué à l'$I^{125}$ (anti-BNP-$I^{125}$). Il s'agit de l'anticorps traceur du kit BNP Shionoria commercialisé par la société Shionogi.
4) Tampon de saturation : tampon PBS Dulbecco à pH 7,4 contenant 1% d'albumine bovine sérique (BSA, Sigma # A-7888)
5) Tampon de dilution : tampon PBS Dulbecco à pH 7,4 contenant 0,1% de BSA et 0,1% de tween 20 (Sigma, # P-1379).
4) Tampon de lavage : tampon PBS Dulbecco à pH 7,4 contenant 0,1% de Tween 20.
5) Etalon proBNP(1-108): produit sous forme de protéine recombinée.

*Protocole :*

Le principe du test utilisé est basé sur la méthode radioimmunologique de type sandwich , réalisé en microplaque à fond plat et à cupules sécables. Il s'agit d'un test en un temps où échantillon (ou solution étalon de proBNP(1-108)) et traceur sont ajoutés l'un après l'autre sans lavage intermédiaire.

- ✓ Une solution de sensibilisation est tout d'abord préparée avec l'anticorps polyclonal du lapin # 046 805 dilué en tampon PBS Dulbecco à pH 7,4 à 40µg/ml. 300µl de cette solution sont déposés dans chacun des puits de la microplaque.
- ✓ La microplaque est mise à incuber pendant une nuit à 4°C.
- ✓ Après élimination de la solution de sensibilisation, la microplaque est lavée à l'aide de 300 µl d'un tampon PBS Dulbecco à pH 7,4 contenant 0,1% de Tween 20, puis saturée par addition de 300µl du tampon PBS Dulbecco à pH 7,4 contenant 1% de BSA.
- ✓ La microplaque est alors mise à incuber pendant 1 heure à 37°C.
- ✓ La microplaque est ensuite lavée (3 fois) à l'aide de 300 µl de la solution de lavage (tampon PBS Dulbecco à pH 7,4 additionné de Tween 20 à 0.1%).
- ✓ Dans chaque cupule, 100µl de solution étalon de proBNP(1-108), de sérum ou de plasma sont déposés. Le proBNP(1-108), s'il est présent, se lie à l'anticorps de capture retenu sur la phase solide.
- ✓ 200µl de la solution de traceur anti-BNP-$I^{125}$ prête à l'emploi (extraite du kit BNP Shionoria de Shionogi) sont ajoutés dans chacun des puits.
- ✓ Le milieu réactionnel est mis à incuber pendant une nuit (18-22h) à 4°C.
- ✓ Le lendemain, la microplaque est lavée (3 fois) à l'aide de 300µl de la solution de lavage. Au dernier lavage et après aspiration de la solution de lavage, chaque cupule est transférée dans un tube préalablement identifié.
- ✓ La radioactivité présente dans chaque cupule et proportionnelle à la quantité de traceur fixée, donc à la quantité de proBNP(1-108) présente dans l'échantillon, est mesurée à l'aide d'un compteur gamma.

La figure 11 présente les résultats obtenus à l'aide d'une gamme étalon de proBNP(1-108).

**Exemple 16 : résultats du dosage d'échantillons humains**

A l'aide du dosage IRMA proBNP(1-108) selon l'invention décrit dans l'exemple 15 et du dosage BNP(1-76) commercialisé par la société Roche et réalisé sur l'automate Elecsys®, 14 prélèvements de sujets sains et 15 prélèvements de patients souffrant d'insuffisance cardiaque ont été testés. Les échantillons sanguins ont tous été prélevés sur tube contenant de l'EDTA. La figure 12 présente les résultats en cpm (coups par minute) des tests de ces échantillons avec le dosage IRMA proBNP(1-108) selon l'invention. Les résultats obtenus sur les prélèvements de patients souffrant d'insuffisance cardiaque sont significativement plus élevés que ceux obtenus sur les prélèvements de sujets sains. Ces résultats mettent en évidence la présence de proBNP(1-108) circulant dans les prélèvements de patients souffrant d'insuffisance cardiaque et constituent la première démonstration que le proBNP(1-108) sérique est un marqueur prédictif de l'insuffisance cardiaque. La figue 13 présente la corrélation entre les concentrations de proBNP(1-108) (en pg/ml) déterminées à l'aide du dosage IRMA proBNP selon l'invention et les concentrations de BNP(1-76) (pg/ml) déterminées à l'aide du dosage Roche sur l'automate Elecsys®, des prélèvements de 14 patients souffrant d'insuffisance cardiaque. La corrélation observée est significative avec un coefficient $R^2$ = 0,85.

**Exemple 17 : couplage de l'anticorps polyclonal du lapin #046 805 à la biotine**

La méthode de couplage utilise un dérivé N-Hydroxy Succinimide (NHS) de la biotine qui réagit avec les amines primaires des IgG pour former une liaison amide.

Une solution de (+)-Biotine N-succinimidyl ester (Fluka # 14405) à 100mM est préparée par dissolution de la Biotine dans de la diméthylfomamide. La biotinylation est réalisée dans un flacon en verre. 500 $\mu$g de l'anticorps polyclonal du lapin # 046 805 préalablement purifié mais non épuisé sont déposés dans le flacon avec 17$\mu$l de la solution de biotine 100mM (le rapport molaire biotine/anticorps est de 500). La réaction se fait en tampon PBS Dulbecco à pH 7,4. Le mélange réactionnel est mis à incuber pendant 1 heure 30 à température ambiante sous agitation lente. Après couplage, la biotine est inactivée par ajout d'un volume de tampon glycine 2M. Le mélange est mis incuber pendant 10 minutes à température ambiante sous agitation lente. Enfin le mélange est mis à dialyser une nuit à 4˚C contre du tampon PBS Dulbecco à pH 7,4. Le lendemain, une solution d'azoture de sodium est ajoutée à 0,02% en concentration finale. Le conjugué est conservé à 4˚C.

**Exemple 18 : Dosage immunoenzymométrique du proBNP(1-108)**

Un dosage immunoenzymométrique selon l'invention a également été mis au point.

*Matériels :*

1) Phase solide : microplaque Maxisorp à fond plat, Nunc (Danemark).
2) L'anticorps de capture utilisé est un anticorps polyclonal anti-BNP(77-108) commercialisé par la société Strategic Biosolution (# B9105RA00-A0).
3) Le conjugué utilisé est l'anticorps polyclonal du lapin # 046 805 non épuisé couplé à la biotine selon le procédé décrit dans l'exemple 17.
4) Conjugué streptavidine-peroxydase (Amersham Pharmacia Blotech # RPN1231V)
5) Tampon de saturation : tampon PBS Dulbecco à pH 7,4 contenant 1% d'albumine bovine sérique (BSA, Sigma # A-7888)
6) Tampon de dilution : tampon PBS Dulbecco à pH 7,4 contenant 0,1 % de BSA et 0,1% de Tween 20.
7) Solution de lavage : tampon PBS Dulbecco à pH 7,4 contenant 0,1% de Tween 20.
8) Etalon proBNP(1-108) : protéine recombinée.
9) Solution de révélation: la solution de révélation est composée :

   9a) d'un tampon substrat : Solution d'acide citrique 0,01 M, et de citrate trisodique 0,04M contenant de l'$H_2O_2$ à 0,33%, de pH final 5,6, et
   9b) d'un chromogène : comprimés d'OPD (orthophénylènediamine). 1 comprimé d'OPD à dissoudre dans 10ml de tampon substrat.

10) Solution d'arrêt : H2SO4 4N.

*Protocole :*

Le principe du test utilisé est basé sur la méthode enzymoimmunologique de type sandwich, réalisé en microplaque à fond plat. Il s'agit d'un test en deux temps où l'échantillon (ou la solution étalon) est tout d'abord mis à incuber avec l'anticorps de capture, puis après incubation et lavages, l'anticorps de détection est ajouté.

- ✔ Une solution de sensibilisation est tout d'abord préparée avec l'anticorps polyclonal anti-BNP(77-108) dilué en tampon PBS Dulbecco à pH 7,4 à 10μg/ml. 100μl de cette solution sont déposés dans chacun des puits de la microplaque.
- ✔ La microplaque est mise à incuber pendant une nuit à 4°C.
- ✔ Après élimination de la solution de sensibilisation, la microplaque est lavée à l'aide de 300 μl d'un tampon PBS Dulbecco à pH 7,4 contenant 0,1% de Tween 20, puis saturée par addition de 250μl du tampon PBS Dulbecco à pH 7,4 contenant 1% de BSA.
- ✔ La microplaque est alors mise à incuber pendant 1 heure à 37°C.
- ✔ La microplaque est ensuite lavée (3 fois) à l'aide de la solution de lavage.
- ✔ Dans chaque cupule, 100μl de solution étalon de proBNP(1-108), de sérum ou de plasma sont déposés. Le proBNP(1-108), s'il est présent, se lie à l'anticorps de capture retenu sur la phase solide.
- ✔ Le milieu réactionnel est mis à incuber 2 heures à température ambiante
- ✔ La microplaque est ensuite lavée (5 lavages) avec 300μl de la solution de lavage.
- ✔ 100 μl d'anticorps polyclonal du lapin # 046 805 biotinylé (concentré à 6μg/ml), sont ajoutés dans chaque puits de la microplaque.
- ✔ Le milieu réactionnel mis à incuber pendant 2 heures à température ambiante.
- ✔ La microplaque est ensuite lavée (5 lavages) avec 300μl de la solution de lavage.
- ✔ Enfin 100μl de conjugué streptavidine-POD dilué au 1/1000ème sont ajoutés dans chacun des puits de la microplaque.
- ✔ Le milieu réactionnel est mis à incuber pendant 1 heure 30 à température ambiante.
- ✔ Les plaques sont ensuite lavées (5 lavages) avec 300μl de la solution de lavage Dans chaque cupule, 100 μl de la solution de révélation sont distribués. On laisse la réaction se développer à l'obscurité pendant 20 minutes à température ambiante (18-24°C).
- ✔ Ensuite 50 μl de la solution d'arrêt sont distribués dans chaque cupule.
- ✔ Après arrêt de la réaction, la lecture de la densité optique est effectuée au spectrophotomètre à 490/620 nm.

La figure 14 présente les résultats obtenus à l'aide d'une gamme étalon de proBNP(1-108).

**Exemple 19: Evaluation de la réaction croisée, vis à vis du BNP(1-76) et du BNP(77-108), de l'anticorps polyclonal du lapin # 046 805 non épuisé couplé à la biotine, utilisé en sandwich dans le dosage ELISA proBNP(1-108) conjointement à l'anticorps polyclonal anti-BNP(77-108).**

A l'aide du dosage ELISA du proBNP(1-108) décrit dans l'exemple 18, on a évalué la réaction croisée de l'anticorps anti proBNP(1-108) selon l'invention (lapin # 046 805), non épuisé, biotinylé, vis à vis du BNP(1-76) et du BNP(77-108). Des gammes de concentrations de 5ng/ml à 100ng/ml ont été réalisées avec du proBNP(1-108), du BNP (1-76) et du BNP(77-108) et testées à l'aide du dosage ELISA ProBNP(1-108) décrit dans l'exemple 18. Les résultats de la variation de la densité optique à 490nm en fonction de la concentration sont représentés dans la figure 15 pour chacune des protéines. Aucune réaction croisée n'est observée vis à vis du BNP(1-76) ou du BNP(77-108) : le signal obtenu est équivalent au bruit de fond, quelle que soit la concentration testée. Aux concentrations de BNP(1-76) et de BNP(77-108) habituellement retrouvées chez les patients (de l'ordre du ng/ml pour les concentrations les plus élevées), l'anticorps polyclonal du lapin # 046 805 peut être utilisé dans sa version non épuisée sans entraîner l'apparition de réaction croisée.

**Exemple 20 : Evaluation de la réaction croisée, vis à vis du BNP(1-76) et du BNP(77-108), de l'anticorps polyclonal du lapin # 046 805 non épuisé couplé à la biotine, utilisé en sandwich dans le dosage ELISA proBNP (1-108) conjointement à l'anticorps polyclonal anti-NT-proBNP(1-29).**

A l'aide d'un dosage ELISA du proBNP(1-108) utilisant le protocole et les réactifs décrits dans l'exemple 18, mis à part que l'anticorps polyclonal anti-BNP(77-108) est ici remplacé par un anticorps polyclonal anti-NT-proBNP (1-29), on a évalué la réaction croisée de l'anticorps polyclonal du lapin # 046 805 non épuisé, biotinylé vis à vis du BNP(1-76) et du BNP(77-108).

L'anticorps polyclonal anti-NT-proBNP(1-29) a été produit selon le protocole décrit dans l'exemple 3, mis à part que le peptide utilisé comme immunogène était le peptide NT-proBNP(1-29) couplé à la KLH. Des gammes de concentrations de 5ng/ml à 100ng/ml ont été réalisées avec du proBNP(1-108), du BNP(1-76) et du BNP(77-108) et testées à l'aide du dosage ELISA du proBNP(1-108). Les résultats de la variation de la densité optique à 490nm en fonction de la concentration sont représentés dans la figure 16 pour chacune des protéines. Aucune réaction croisée de l'anticorps polyclonal du lapin #046 805 selon l'invention n'est observée vis à vis du BNP(1-76) ou du BNP(77-108), le signal obtenu est équivalent au bruit de fond, quelle que soit la concentration testée. Aux concentrations de BNP (1-76) et de BNP(77-108) habituellement retrouvées chez les patients (de l'ordre du ng/ml), l'anticorps polyclonal du lapin # 046 805 peut être utilisé dans sa version non épuisé sans entraîner l'apparition d'une réaction croisée.

En résumé, il ressort clairement de tout l'exposé qui précède que la présente invention a permis de découvrir un nouvel épitope, $RAPR_{76}S_{77}P$, situé sur la région charnière du proBNP(108) humain, d'en dériver des peptides immunogéniques le contenant, et d'obtenir des anticorps spécifiques du proBNP(108), qui ne reconnaissent substantiellement pas le BNP(1-76) et le BNP(77-108), et dont certains d'entre eux ont la capacité de reconnaître spécifiquement le proBNP (1-108) circulant dans des échantillons sériques ou plasmatiques humains.

Il apparaît aussi clairement que la présente invention a permis la mise au point d'un dosage du proBNP(1-108) circulant permettant ainsi un diagnostic de l'insuffisance cardiaque de façon simple, utilisable en routine et fiable.

## Revendications

1. Anticorps anti proBNP(1-108) **caractérisé en ce que**, d'une part, il reconnaît un épitope comprenant la séquence $RAPR_{76}S_{77}P$ (SEQ ID N° 5) du proBNP(1-108) et ne reconnaît substantiellement pas les peptides BNP(1-76) ni le BNP(77-108), et, d'autre part, a la capacité de reconnaître spécifiquement le proBNP(1-108) circulant dans des échantillons sériques ou plasmatiques humains.

2. Anticorps anti proBNP(1-108) selon la revendication 1 qui reconnaît de façon spécifique la séquence $V_{70}TLRAPR_{76}S_{77}PKMVQGSG_{85}$ (SEQ ID N° 4) du proBNP(1-108).

3. Anticorps anti proBNP(1-108) selon la revendication 1 qui reconnaît de façon spécifique la séquence $Y_{70}TLRAPR_{78}S_{77}PKMVQGS_{84}$ (SEQ ID N° 108) du proBNP(1-108).

4. Anticorps anti proBNP(1-108) selon l'une des revendications 1 à 3, lequel anticorps est monoclonal.

5. Anticorps selon la revendication 4, secrété par l'hybridome 3D4 déposé à la CNCM sous le N°CNCM I-3073

6. hybridome produisant un anticorps tel que défini dans la revendication 5, nommé 3D4 et déposé à la CNCM sous le N°CNCM I-3073.

7. Peptide de formule:

$$a_1 - X_1 - RAPRSP-X_2- a_2 \qquad (I)$$

où

$a_1$ peut être H ou représenter une fonction ou un groupement chimique choisi parmi une fonction thiol, alcool, aminoxy, amine primaire ou secondaire, un groupement amino-carboxyle, un groupement biotinyle et un groupement acétyle,
$X_1$ représente une séquence peptidique de 0 à 3 acides aminés, issue de la séquence naturelle du proBNP (1-108) ou non,
$X_2$ représente une séquence peptidique de 0 à 7 acides aminés, issue de la séquence naturelle du proBNP (1-108) ou non,
$a_2$ peut représenter une fonction OH, $NH_2$ ou un groupement alcoxyle.

8. Peptide de formule :

$$X-Y_{70}TLRAPR_{76}S_{77}PKMVQGSG_{85}-Z \qquad (II)$$

où X peut être H, ou représenter soit un groupement acétyle, soit 1 à 3 acides aminés n'appartenant pas à la séquence du proBNP(1-108), et où Z peut représenter une fonction OH, ou 1 à 3 acides aminés n'appartenant pas à la séquence du proBNP(1-108).

9. Peptide selon la revendication 8, ayant la séquence $Y_{70}TLRAPR_{76}S_{77}PKMVQGSG_{85}$ (SEQ ID N° 4).

**10.** Peptide de formule :

$$X\text{-}Y_{70}TLRAPR_{76}S_{77}PKMVQGS_{84}\text{-}Z \qquad (III)$$

où X peut être H, ou représenter soit un groupement acétyle, soit 1 à 3 acides aminés n'appartenant pas à la séquence du proBNP(1-108), et où Z peut représenter une fonction OH, ou 1 à 3 acides aminés n'appartenant pas à la séquence du proBNP(1-108).

**11.** Peptide selon la revendication 10, ayant la séquence $Y_{70}TLRAPR_{76}S_{77}PKMVQGS_{84}$ (SEQ ID N° 108).

**12.** Peptide comprenant une séquence dérivée de la séquence du peptide de formule (II) tel que défini dans la revendication 8 ou du peptide de formule (III) tel que défini dans la revendication 10 par substitution d'un ou plusieurs parmi les acides aminés $Y_{70}$, $T_{71}$, $L_{72}$, $K_{79}$, $M_{80}$, $V_{81}$, $Q_{82}$, $G_{83}$, $S_{84}$ et $G_{85}$.

**13.** Peptide selon la revendication 7, ayant une séquence choisie dans le groupe constitué par les séquences suivantes :

SEQ ID N° 16 : C-Y-T-L-R-A-P-R-S-P-K-M-V-Q-G-S-G (peptide C13P30)
SEQ ID N° 109 : C-Y-T-L-R-A-P-R-S-P-K-M-V-Q-G-S (peptide CN32)
SEQ ID N° 6 : C-G-R-A-P-R-S-P
SEQ ID N°7 : Acétyl -C-G-R-A-P-R-S-P
SEQ ID N° 8 : C-G-R-A-P-R-S-P-K
SEQ ID N° 9 : Acétyl -C-G-R-A-P-R-S-P-K
SEQ ID N° 10 : C-G-R-A-P-R-S-P-K-M-V
SEQ ID N° 11 : C-G-R-A-P-R-S-P-K-M-V-Q-G-S-G
SEQ ID N° 12 : R-A-P-R-S-P-G-C
SEQ ID N° 13 : Acétyl -R-A-P-R-S-P-G-C
SEQ ID N° 110 : C-Y-T-L-R-A-P-R-S-P-K
SEQ ID N° 111 : C-Y-T-L-R-A-P-R-S-P-K-M-V
SEQ ID N° 112 : C-Y-T-L-R-A-P-R-S-P-K-M-V-Q
SEQ ID N° 113 : C-Y-T-L-R-A-P-R-S-P-K-M-V-Q-G
SEQ ID N° 19 : C-Y-T-L-R-A-P-R-S-P-K-M-V-Q-G-S-βA
SEQ ID N° 20 : C-Y-T-L-R-A-P-R-S-P-K-M-V-O-A-T-βA
SEQ ID N° 114 : Acétyl-C-T-L-R-A-P-R-S-P-K-M-V-Q
SEQ ID N° 115 : C-T-L-R-A-P-R-S-P-K-M-V-Q-G
SEQ ID N° 116 : C-T-L-R-A-P-R-S-P-K-M-V-Q-G-S
SEQ ID N° 117 : C-T-L-R-A-P-R-S-P-K-M-V-Q-G-S-G
SEQ ID N° 118 : C-L-R-A-P-R-S-P-K-M-V
SEQ ID N° 119 : C-L-R-A-P-R-S-P-K-M-V-Q
SEQ ID N° 120 : L-R-A-P-R-S-P-K-M-V-Q-C
SEQ ID N° 121 : C-L-R-A-P-R-S-P-K-M-V-Q-G-S
SEQ ID N° 122 : C-L-R-A-P-R-S-P-K-M-V-Q-G-S-G

**14.** Trousse de détection du proBNP(1-108) dans un échantillon biologique, contenant au moins un anticorps tel que défini dans l'une des revendications 1 à 5.

**15.** Trousse de détection du proBNP(1-108) dans un échantillon biologique, contenant, comme étalon et/ou témoin, au moins un peptide tel que défini dans l'une des revendications 7 à 13.

**16.** Trousse de détection du proBNP(1-108) dans un échantillon biologique selon la revendication 14 ou 15, contenant :

(i) dans un récipient, au moins un anticorps tel que défini dans l'une des revendications 1 à 5;
(ii) dans un autre récipient au moins un peptide tel que défini dans l'une des revendications 7 à 13.

**17.** Procédé d'obtention d'un anticorps anti proBNP(1-108) tel que défini à l'une des revendications 1, 2 et 3 dans lequel on immunise un animal avec la molécule entière de proBNP(1-108),
puis on épuise, à l'aide du peptide BNP(77-108) et/ou du peptide BNP(1-76), l'antisérum obtenu.

**18.** Procédé d'obtention d'un anticorps anti proBNP(1-108), tel que défini à l'une des revendications 1, 2 et 3, dans lequel on immunise un animal avec un peptide tel que défini dans l'une des revendications 7 à 13 et, éventuellement, on épuise, à l'aide du peptide BNP(77-108) et/ou du peptide BNP(1-76), l'antisérum obtenu.

**19.** Procédé d'obtention d'hybridome sécréteur d'un anticorps anti proBNP(1-108), tel que défini dans la revendication 4 ou 5, dans lequel
on immunise un animal avec un peptide tel que défini dans l'une des revendications 7 à 13,
on prélève des lymphocytes sécréteurs d'immunoglobulines de cet animal,
on procède à une fusion des lymphocytes avec des cellules de myélome pour obtenir au moins un hybridome sécréteur d'immunoglobuline,
et on sélectionne un hybridome sécréteur d'anticorps monoclonaux spécifiques.

**20.** Procédé selon l'une des revendications 18 et 19, dans lequel le peptide est un peptide de formule (II) ayant la séquence $Y_{70}TLRAPR_{76}S_{77}PKMVQGSG_{85}$ (SEQ ID N° 4).

**21.** Procédé selon l'une des revendications 18 et 19, dans lequel le peptide est un peptide de formule (III) ayant la séquence $Y_{70}TLRAPR_{76}S_{77}PKMVQGS_{84}$ (SEQ ID N° 108).

**22.** Méthode de diagnostic *in vitro* de l'insuffisance cardiaque chez un humain comprenant la mise en contact d'un échantillon biologique avec un anticorps anti proBNP(1-108) tel que défini dans l'une des revendications 1 à 5, et la détection du proBNP(1-108) dans l'échantillon.

**23.** Méthode de diagnostic *in vitro* de l'insuffisance cardiaque chez un humain comprenant :

a) la mise en contact d'un échantillon biologique avec un anticorps anti proBNP(1-108) tel que défini à l'une des revendications 1 à 5,
b) l'incubation du mélange dans des conditions permettant la formation de complexes antigènes-anticorps ; et
c) la révélation des complexes antigènes-anticorps formés,

éventuellement à l'aide d'un anticorps de détection, marqué, capable de se lier spécifiquement au proBNP(1-108) présent dans le premier complexe, ou à l'aide d'un antigène de détection, marqué, capable de se lier à l'anticorps dirigé contre ledit proBNP(1-108) présent dans le premier complexe.

**24.** Méthode de diagnostic selon la revendication 23 qui comprend en plus une étape d) de corrélation de la quantité des complexes antigènes-anticorps révélés à l'état clinique du sujet.

## Claims

**1.** An anti proBNP(1-108) antibody, **characterised in that**, on the one hand, it recognises an epitope comprising the sequence $RAPR_{76}S_{77}P$ (SEQ ID No. 5) of proBNP(1-108) and substantially does not recognise the peptides BNP (1-76) nor BNP(77-108), and, on the other hand, has the ability specifically to recognise proBNP(1-108) circulating in human serum or plasma samples.

**2.** An anti proBNP(1-108) antibody according to claim 1 which recognises in a specific manner the sequence $Y_{70}TLRAPR_{76}S_{77}PKMVQGSG_{85}$ (SEQ ID No. 4) of proBNP(1-108) .

**3.** An anti proBNP(1-108) antibody according to claim 1 which recognises in a specific manner the sequence $Y_{70}TLRAPR_{76}S_{77}PKMVQGS_{84}$ (SEQ ID No. 108) of proBNP(1-108).

**4.** An anti proBNP(1-108) antibody according to any one of claims 1 to 3, which antibody is monoclonal.

**5.** An antibody according to claim 4, secreted by the hybridoma 3D4 deposited at the CNCM under CNCM No. I-3073.

**6.** A hybridoma producing an antibody as defined in claim 5, named 3D4 and deposited at the CNCM under CNCM No. I-3073.

**7.** A peptide of the formula:

$$a_1\text{-}X_1\text{-RAPRSP-}X_2\text{-}a_2 \qquad (I)$$

in which

- $a_1$ may be H or represent a chemical function or group selected from among a thiol, alcohol, aminoxy, or primary or secondary amine function or an aminocarboxyl group, a biotinyl group and an acetyl group,
- $X_1$ represents a peptide sequence of 0 to 3 amino acids, which may or may not originate from the natural sequence of proBNP(1-108),
- $X_2$ represents a peptide sequence of 0 to 7 amino acids, which may or may not originate from the natural sequence of proBNP(1-108),
- $a_2$ may represent an OH or $NH_2$ function or an alkoxyl group.

**8.** A peptide of the formula:

$$X\text{-}Y_{70}TLRAPR_{76}S_{77}PKMVQGSG_{85}\text{-}Z \qquad (II)$$

in which X may be H, or represent either an acetyl group, or 1 to 3 amino acids which do not belong to the proBNP (1-108) sequence, and in which Z may represent an OH function, or 1 to 3 amino acids which do not belong to the proBNP(1-108) sequence.

**9.** A peptide according to claim 8, having the sequence $Y_{70}TLRAPR_{76}S_{77}PKMVQGSG_{85}$ (SEQ ID No. 4).

**10.** A peptide of the formula:

$$X\text{-}Y_{70}TLRAPR_{76}S_{77}PKMVQGS_{84}\text{-}Z \qquad (III)$$

in which X may be H, or represent either an acetyl group, or 1 to 3 amino acids which do not belong to the proBNP (1-108) sequence, and in which Z may represent an OH function, or 1 to 3 amino acids which do not belong to the proBNP(1-108) sequence.

**11.** A peptide according to claim 10, having the sequence $Y_{70}TLRAPR_{76}S_{77}PKMVQGSG_{84}$ (SEQ ID No. 108).

**12.** A peptide comprising a sequence derived from the peptide sequence of the formula (II) as defined in claim 8 or from the peptide of the formula (III) as defined in claim 10 by substitution of one or more from among the amino acids $Y_{70}$, $T_{71}$, $L_{72}$, $K_{79}$, $M_{80}$, $V_{81}$, $Q_{82}$ $G_{83}$, $S_{84}$ and $G_{85}$.

**13.** A peptide according to claim 7, having a sequence selected from among the group made up of the following sequences:

SEQ ID No. 16: C-Y-T-L-R-A-P-R-S-P-K-M-V-Q-G-S-G (peptide C13P30)
SEQ ID No. 109: C-Y-T-L-R-A-P-R-S-P-K-M-V-Q-G-S (peptide CN32)
SEQ ID No. 6: C-G-R-A-P-R-S-P
SEQ ID No. 7: Acetyl-C-G-R-A-P-R-S-P
SEQ ID No. 8: C-G-R-A-P-R-S-P-K
SEQ ID No. 9: Acetyl-C-G-R-A-P-R-S-P-K
SEQ ID No. 10: C-G-R-A-P-R-S-P-K-M-V
SEQ ID No. 11: C-G-R-A-P-R-S-P-K-M-V-Q-G-S-G
SEQ ID No. 12: R-A-P-R-S-P-G-C
SEQ ID No. 13: Acetyl-R-A-P-R-S-P-G-C
SEQ ID No. 110: C-Y-T-L-R-A-P-R-S-P-K
SEQ ID No. 111: C-Y-T-L-R-A-P-R-S-P-K-M-V
SEQ ID No. 112: C-Y-T-L-R-A-P-R-S-P-K-M-V-Q
SEQ ID No. 113: C-Y-T-L-R-A-P-R-S-P-K-M-V-Q-G
SEQ ID No. 19: C-Y-T-L-R-A-P-R-S-P-K-M-V-Q-G-S-βA
SEQ ID No. 20: C-Y-T-L-R-A-P-R-S-P-K-M-V-Q-A-T-βA
SEQ ID No. 114: Acetyl-C-T-L-R-A-P-R-S-P-K-M-V-Q
SEQ ID No. 115: C-T-L-R-A-P-R-S-P-K-M-V-Q-G
SEQ ID No. 116: C-T-L-R-A-P-R-S-P-K-M-V-Q-G-S

SEQ ID No. 117: C-T-L-R-A-P-R-S-P-K-M-V-Q-G-S-G
SEQ ID No. 118: C-L-R-A-P-R-S-P-K-M-V
SEQ ID No. 119: C-L-R-A-P-R-S-P-K-M-V-Q
SEQ ID No. 120: L-R-A-P-R-S-P-K-M-V-Q-C
SEQ ID No. 121: C-L-R-A-P-R-S-P-K-M-V-Q-G-S
SEQ ID No. 122: C-L-R-A-P-R-S-P-K-M-V-Q-G-S-G

**14.** A kit for detecting proBNP(1-108) in a biological sample, containing at least one antibody as defined in one of claims 1 to 5.

**15.** A kit for detecting proBNP(1-108) in a biological sample, containing, as standard and/or control, at least one peptide as defined in one of claims 7 to 13.

**16.** A kit for detecting proBNP(1-108) in a biological sample according to claim 14 or 15, containing:

(i) in one receptacle, at least one antibody as defined in one of claims 1 to 5;
(ii) in another receptacle, at least one antibody as defined in one of claims 7 to 13.

**17.** A process for obtaining an anti proBNP(1-108) antibody as defined in one of claims 1, 2 and 3, in which an animal is immunised with the entire proBNP(1-108) molecule,
then the antiserum obtained is depleted using the peptide BNP(77-108) and/or the peptide BNP(1-76).

**18.** A process for obtaining an anti proBNP(1-108) antibody, as defined in one of claims 1, 2 and 3, in which an animal is immunised with a peptide as defined in one of claims 7 to 13 and, optionally, the antiserum obtained is depleted using the peptide BNP(77-108) and/or the peptide BNP(1-76).

**19.** A process for obtaining a hybridoma which secretes an anti proBNP(1-108) antibody, as defined in claim 4 or 5, in which

an animal is immunised with a peptide as defined in one of claims 7 to 13,
lymphocytes which secrete immunoglobulins are taken from this animal,
the lymphocytes are fused with myeloma cells so as to obtain at least one hybridoma which secretes immunoglobulin,
and a hybridoma which secretes specific monoclonal antibodies are selected.

**20.** A process according to either one of claims 18 and 19, in which the peptide is a peptide of the formula (II) having the sequence $Y_{70}TLRAPR_{76}S_{77}PKMVQGSG_{85}$ (SEQ ID No. 4).

**21.** A process according to either one of claims 18 and 19, in which the peptide is a peptide of the formula (III) having the sequence $Y_{70}TLRAPR_{76}S_{77}PKMVQGS_{84}$ (SEQ ID No. 108).

**22.** A method for *in vitro* diagnosis of heart failure in a human, comprising bringing a biological sample into contact with an anti proBNP(1-108) antibody as defined in one of claims 1 to 5, and detecting the proBNP(1-108) in the sample.

**23.** A method for *in vitro* diagnosis of heart failure in a human, comprising:

a) bringing a biological sample into contact with an anti proBNP(1-108) antibody as defined in one of claims 1 to 5,
b) incubating the mixture under conditions allowing the formation of antigen-antibody complexes; and
c) visualising the antigen-antibody complexes formed,

optionally using a (labelled) detection antibody capable of binding specifically with the proBNP(1-108) present in the first complex, or using a (labelled) detection antigen capable of binding with the antibody directed against said proBNP(1-108) present in the first complex.

**24.** A diagnostic method according to claim 23 which additionally comprises a stage d) of correlating the quantity of visualised antigen-antibody complexes with the clinical status of the subject.

## Patentansprüche

1. Ein anti-proBNP (1-108) Antikörper, **dadurch gekennzeichnet, dass** er einerseits ein Epitop erkennt, dass die Sequenz $RAPR_{76}S_{77}P$ (SEQ ID Nr. 5) von proBNP (1-108) umfasst, und im wesentlichen weder die Peptide BNP (1-76) noch BNP (77-108) erkennt, und dass er andererseits die Fähigkeit hat, spezifisch das im humanen Serum oder in Plasmaproben zirkulierende proBNP (1-108) zu erkennen.

2. Der anti-proBNP (1-108) Antikörper nach Anspruch 1, der spezifisch die Sequenz $Y_{70}TLRAPR_{76}S_{77}PKMVQGSG_{85}$ (SEQ ID Nr. 4) von proBNP (1-108) erkennt.

3. Der anti-proBNP (1-108) Antikörper nach Anspruch 1, der spezifisch die Sequenz $Y_{70}TLRAPR_{76}S_{77}PKMVQGS_{84}$ (SEQ ID Nr. 108) von proBNP (1-108) erkennt.

4. Der anti-proBNP (1-108) Antikörper nach einem der Ansprüche 1 bis 3, wobei der Antikörper monoklonal ist.

5. Der Antikörper nach Anspruch 4, der durch das Hybridom 3D4, das am CNCM unter der Nr. CNCM I-3073 hinterlegt wurde, abgesondert wird.

6. Ein Hybridom, das einen Antikörper erzeugt, der nach Anspruch 5 definiert wird, das 3D4 genannt wird und am CNCM unter der Nr. CNCM I-3073 hinterlegt wurde.

7. Ein Peptid der Formel:

$$a_1\text{-}X_1\text{-RAPRSP-}X_2\text{-}a_2 \qquad \text{(I)}$$

wobei

a1 H sein kann oder eine Funktion oder eine chemische Gruppe repräsentieren kann, die aus einer Thiol-, Alkohol-, Aminoxy-, primären Amin- oder sekundären Aminfunktion, einer Aminocarboxyl-Gruppe, einer Biotinyl-Gruppe und eine Acetyl-Gruppe ausgewählt wird,
$X_1$ eine Peptidsequenz von 0 bis 3 Aminosäuren repräsentiert, die aus der natürlichen Sequenz von proBNP (1-108) hervorgeht oder nicht,
$X_2$ eine Peptidsequenz von 0 bis 7 Aminosäuren repräsentiert, die aus der natürlichen Sequenz von proBNP (1-108) hervorgeht oder nicht,
$a_2$ eine OH-Funktion, eine $NH_2$-Funktion oder eine Alkoxyl-Gruppe repräsentieren kann.

8. Ein Peptid der Formel

$$X\text{-}Y_{70}TLRAPR_{76}S_{77}PKMVQGSG_{85}\text{-}Z \qquad \text{(II)}$$

wobei X H sein kann oder entweder eine Acetyl-Gruppe oder 1 bis 3 Aminosäuren, die nicht zu der Sequenz von proBNP (1-108) gehören, repräsentieren kann, und wobei Z eine OH-Funktion oder 1 bis 3 Aminosäuren, die nicht zu der Sequenz von proBNP (1-108) gehören, repräsentieren kann.

9. Das Peptid nach Anspruch 8, dass die Sequenz $Y_{70}TLRAPR_{76}S_{77}PKMVQGSG_{85}$ (SEQ ID Nr. 4) hat.

10. Ein Peptid der Formel:

$$X\text{-}Y_{70}TLRAPR_{76}S_{77}PKMVQGS_{84}\text{-}Z \qquad \text{(III)}$$

wobei X H sein kann oder entweder eine Acetyl-Gruppe oder 1 bis 3 Aminosäuren, die nicht zu der Sequenz von proBNP (1-108) gehören, repräsentieren kann, und wobei Z eine OH-Funktion oder 1 bis 3 Aminosäuren, die nicht zu der Sequenz von proBNP (1-108) gehören, repräsentieren kann.

11. Das Peptid nach Anspruch 10, das die Sequenz $Y_{70}TLRAPR_{76}S_{77}PKMVQGS_{84}$ (SEQ ID Nr. 108) hat.

12. Ein Peptid, das eine Sequenz umfasst, die sich von der Sequenz des Peptids nach Formel (II), das in Anspruch 8 definiert wird, oder des Peptids nach Formel (III), das in Anspruch 10 definiert wird, durch Substitution von einer oder mehreren unter den Aminosäuren $Y_{70}$, $T_{71}$, $L_{72}$, $K_{79}$, $M_{80}$, $V_{81}$, $Q_{82}$, $G_{83}$, $S_{84}$ und $G_{85}$ ableitet.

**13.** Das Peptid nach Anspruch 7, das eine Sequenz hat, die aus der Gruppe ausgewählt wird, die aus den folgenden Sequenzen besteht
SEQ ID Nr. 16: C-Y-T-L-R-A-P-R-S-P-K-M-V-Q-G-S-G (Peptid C13P30)
SEQ ID Nr. 109: C-Y-T-L-R-A-P-R-S-P-K-M-V-Q-G-S (Peptid CN32)
SEQ ID Nr. 6: C-G-R-A-P-R-S-P
SEQ ID Nr. 7: Acetyl-C-G-R-A-P-R-S-P
SEQ ID Nr. 8: C-G-R-A-P-R-S-P-K
SEQ ID Nr. 9: Acetyl-C-G-R-A-P-R-S-P-K
SEQ ID Nr. 10: C-G-R-A-P-R-S-P-K-M-V
SEQ ID Nr. 11: C-G-R-A-P-R-S-P-K-M-V-Q-G-S-G SEQ ID Nr. 12: R-A-P-R-S-P-G-C
SEQ ID Nr. 13: Acetyl-R-A-P-R-S-P-G-C
SEQ ID Nr. 110: C-Y-T-L-R-A-P-R-S-P-K
SEQ ID Nr. 111: C-Y-T-L-R-A-P-R-S-P-K-M-V
SEQ ID Nr. 112: C-Y-T-L-R-A-P-R-S-P-K-M-V-Q
SEQ ID Nr. 113: C-Y-T-L-R-A-P-R-S-P-K-M-V-Q-G
SEQ ID Nr. 19: C-Y-T-L-R-A-P-R-S-P-K-M-V-Q-G-S-βA
SEQ ID Nr. 20: C-Y-T-L-R-A-P-R-S-P-K-M-V-Q-A-T-βA
SEQ ID Nr. 114: Acetyl-C-T-L-R-A-P-R-S-P-K-M-V-Q
SEQ ID Nr. 115: C-T-L-R-A-P-R-S-P-K-M-V-Q-G
SEQ ID Nr. 116: C-T-L-R-A-P-R-S-P-K-M-V-Q-G-S
SEQ ID Nr. 117: C-T-L-R-A-P-R-S-P-K-M-V-Q-G-S-G
SEQ ID Nr. 118: C-L-R-A-P-R-S-P-K-M-V
SEQ ID Nr. 119: C-L-R-A-P-R-S-P-K-M-V-Q
SEQ ID Nr. 120: L-R-A-P-R-S-P-K-M-V-Q-C
SEQ ID Nr. 121: C-L-R-A-P-R-S-P-K-M-V-Q-G-S SEQ ID Nr. 122: C-L-R-A-P-R-S-P-K-M-V-Q-G-S-G.

**14.** Ein Kit zum Nachweisen von proBNP (1-108) in einer biologischen Probe, das mindestens einen Antikörper, wie in einem der Ansprüche 1 bis 5 definiert, enthält.

**15.** Ein Kit zum Nachweisen von proBNP (1-108) in einer biologischen Probe, das als Standard und/oder Kontrolle mindestens ein Peptid wie in einem der Ansprüche 7 bis 13 definiert enthält.

**16.** Ein Kit zum Nachweisen von proBNP (1-108) in einer biologischen Probe nach Anspruch 14 oder 15, das enthält:

(i) in einem Behälter mindestens ein Antikörper wie in einem der Ansprüche 1 bis 5 definiert;
(ii) in einem anderen Behälter mindestens ein Peptid wie in einem der Ansprüche 7 bis 13 definiert.

**17.** Ein Verfahren zum Erhalten eines wie in einem der Ansprüche 1, 2 oder 3 definierten anti-proBNP (1-108) Antikörpers, in dem ein Tier mit dem gesamten Molekül von proBNP (1-108) immunisiert wird, wobei dann das erhaltene Antiserum unter Verwendung des BNP (77-108) Peptids und/oder des BNP (1-76) Peptids aufgebraucht wird.

**18.** Ein Verfahren zum Erhalten eines wie in einem der Ansprüche 1, 2 oder 3 definierten anti-proBNP (1-108) Antikörpers, in dem ein Tier mit einem Peptid wie in einem der Ansprüche 7 bis 13 definiert immunisiert wird, wobei optional das erhaltene Antiserum unter Verwendung des BNP (77-108) Peptids und/oder des BNP (1-76) Peptids aufgebraucht wird.

**19.** Ein Verfahren zum Erhalten eines Hybridoms, das einen anti-proBNP (1-108) Antikörper, wie in Anspruch 4 oder 5 definiert, absondert, bei dem:

- ein Tier mit einem Peptid wie in einem der Ansprüche 7 bis 13 definiert immunisiert wird,
- Immunglobulin-absondernde Lymphozyten von diesem Tier genommen werden,
- die Lymphozyten mit Myelomzellen fusioniert werden, um mindestens ein Immunoglobulin-absonderndes Hybridom zu erhalten, und
- ein spezifische monoklonale Antikörper absonderndes Hybridom ausgewählt wird.

**20.** Das Verfahren nach einem der Ansprüche 18 und 19, bei dem das Peptid ein Peptid der Formel (II) ist, das die

Sequenz $Y_{70}TLRAPR_{76}S_{77}PKMVQGSG_{85}$ (SEQ ID Nr. 4) hat.

**21.** Das Verfahren nach einem der Ansprüche 18 und 19, bei dem das Peptid ein Peptid der Formel (III) ist, das die Sequenz $Y_{70}TLRAPR_{76}S_{77}PKMVQGS_{84}$ (SEQ ID Nr. 108) hat.

**22.** Ein Verfahren zur in-vitro Diagnose von Herzversagen bei einem Menschen, das das In-Kontakt-bringen einer biologischen Probe mit einem anti-proBNP (1-108) Antikörper wie in einem der Ansprüche 1 bis 5 definiert, und das Nachweisen des proBNP (1-108) in der Probe umfasst.

**23.** Ein Verfahren zur in-vitro Diagnose von Herzversagen bei einem Menschen, das umfasst:

a) das In-Kontakt-bringen einer biologischen Probe mit einem anti-proBNP (1-108) Antikörper wie in einem der Ansprüche 1 bis 5 definiert,
b) Inkubieren der Mischung unter Bedingungen, die die Bildung von Antigen-Antikörper-Komplexen ermöglichen, und
c) Enthüllen der gebildeten Antigen-Antikörper-Komplexe, optional unter Verwendung eines markierten Nachweisantikörper, der spezifisch an das proBNP (1-108), das in dem primären Komplex vorliegt, binden kann, oder unter Verwendung eines markierten Nachweisantigens, das an den Antikörper, der gegen das in dem primären Komplex vorliegende proBNP (1-108) gerichtet ist, binden kann.

**24.** Das Verfahren zur Diagnose nach Anspruch 23, das ebenfalls einen Schritt d) für das Korrelieren der Menge der Antigen-Antikörper-Komplexe, die bei der klinischen Bedingung des Individuums offengelegt wurden, umfasst.

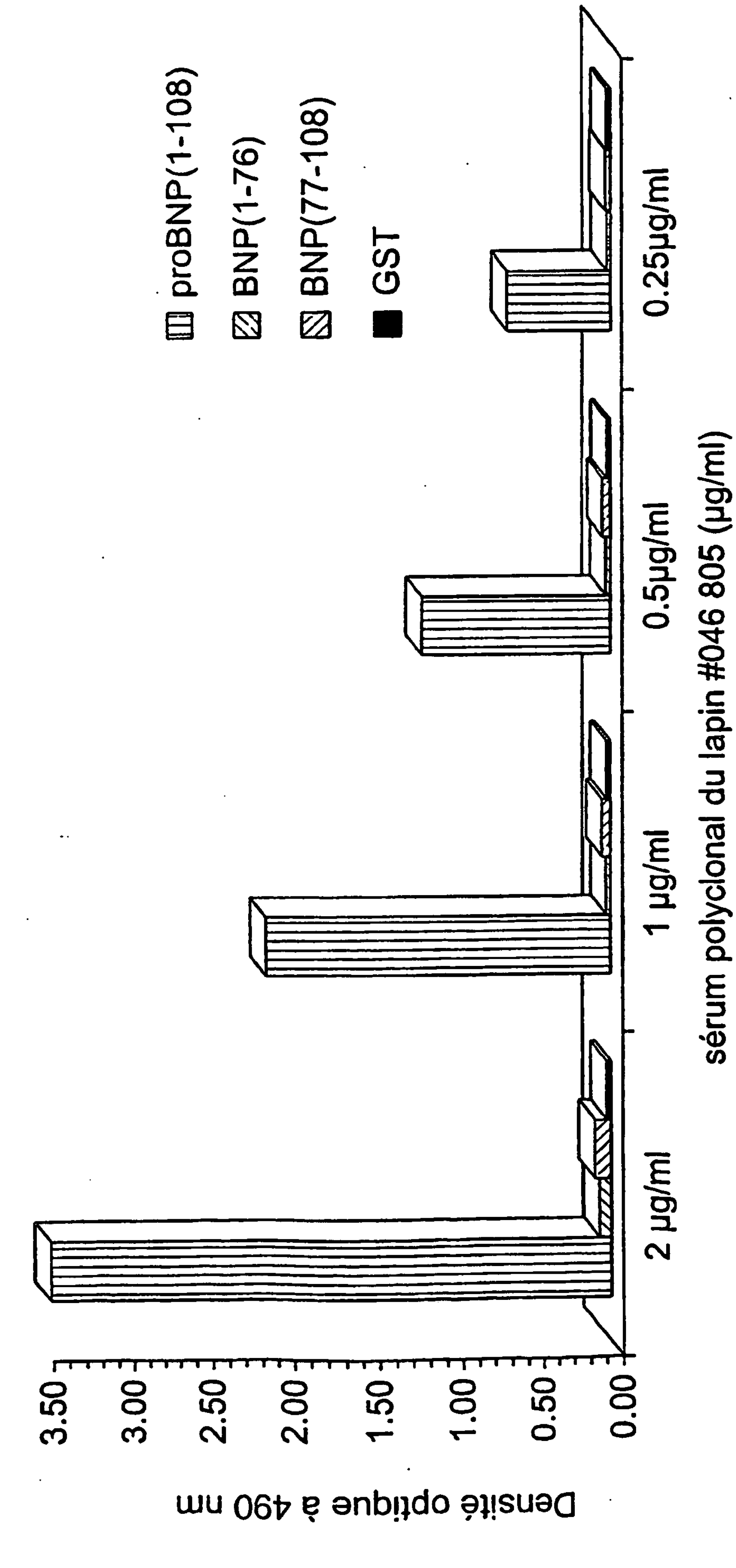
proBNP(1-108)
BNP(1-76)
BNP(77-108)
GST
Densité optique à 490 nm
3.50
3.00
2.50
2.00
1.50
1.00
0.50
0.00
2 µg/ml
1 µg/ml
0.5µg/ml
0.25µg/ml
sérum polyclonal du lapin #046 805 (µg/ml)

FIG.1

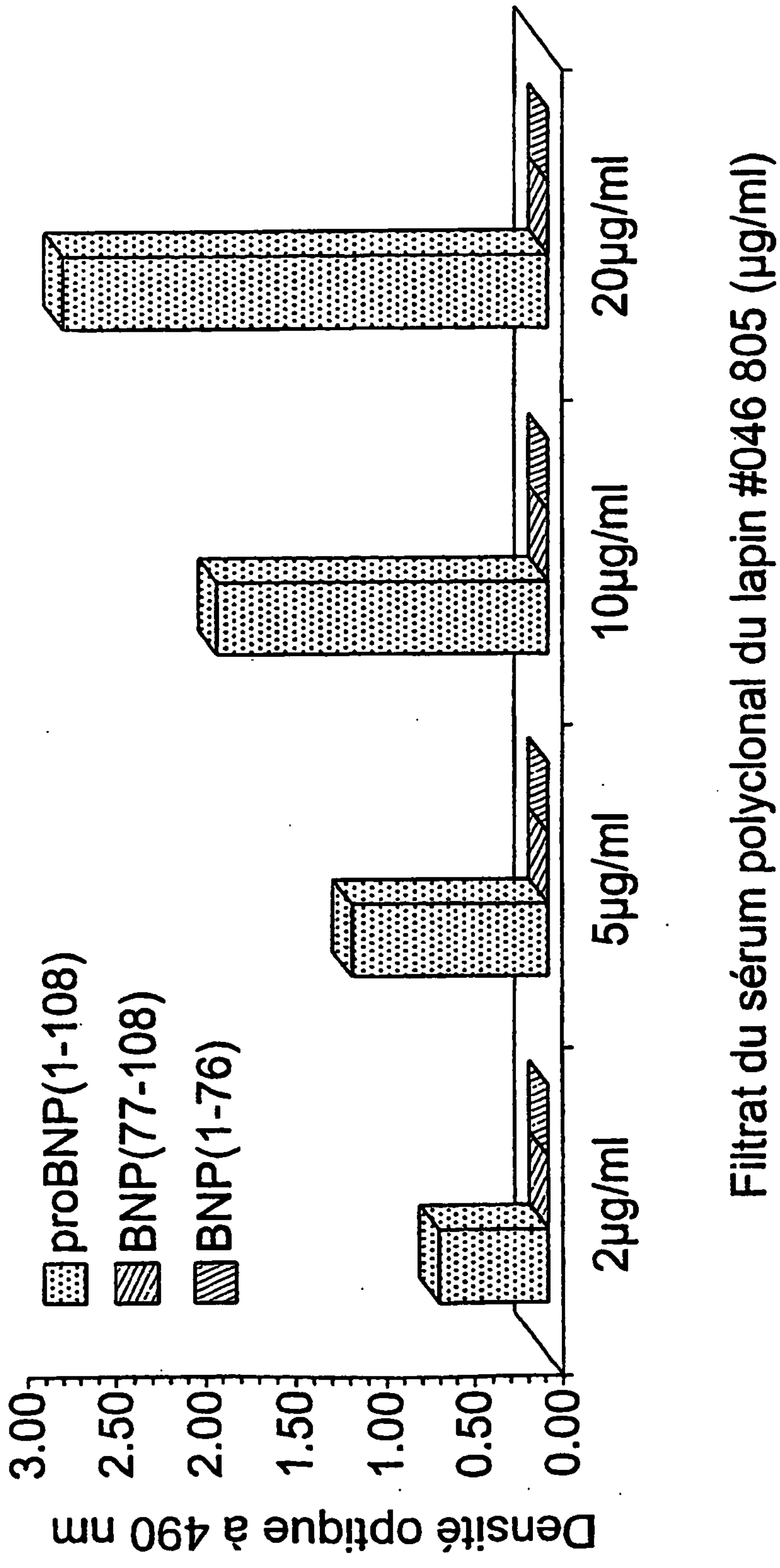

Densité optique à 490 nm
3.00
2.50
2.00
1.50
1.00
0.50
0.00
proBNP(1-108)
BNP(77-108)
BNP(1-76)
2µg/ml
5µg/ml
10µg/ml
20µg/ml
Filtrat du sérum polyclonal du lapin #046 805 (µg/ml)


FIG.2

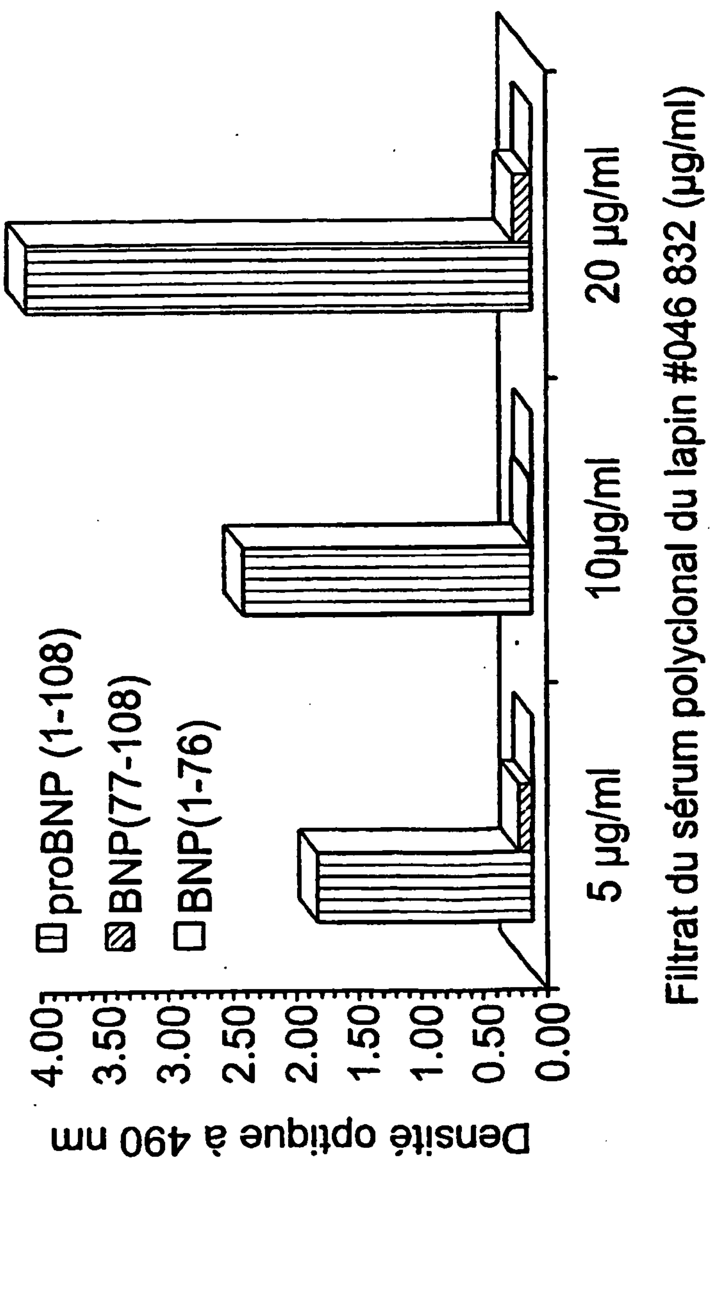
Densité optique à 490 nm
4.00
3.50
3.00
2.50
2.00
1.50
1.00
0.50
0.00
proBNP (1-108)
BNP(77-108)
BNP(1-76)
5 µg/ml
10µg/ml
20 µg/ml
Filtrat du sérum polyclonal du lapin #046 832 (µg/ml)

FIG.3

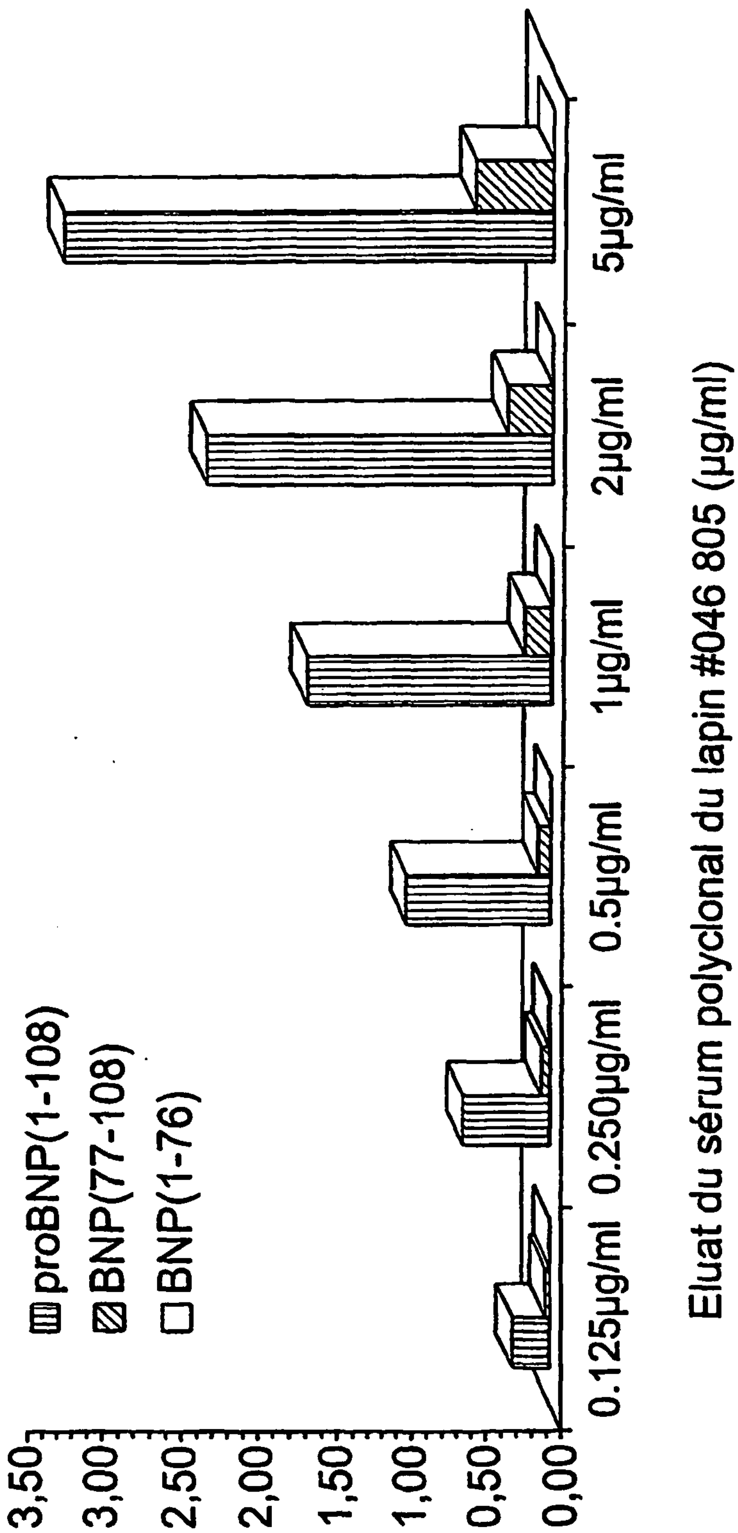
Densité optique à 490nm
3,50
3,00
2,50
2,00
1,50
1,00
0,50
0,00
proBNP(1-108)
BNP(77-108)
BNP(1-76)
0.125µg/ml
0.250µg/ml
0.5µg/ml
1µg/ml
2µg/ml
5µg/ml
Eluat du sérum polyclonal du lapin #046 805 (µg/ml)

FIG.4

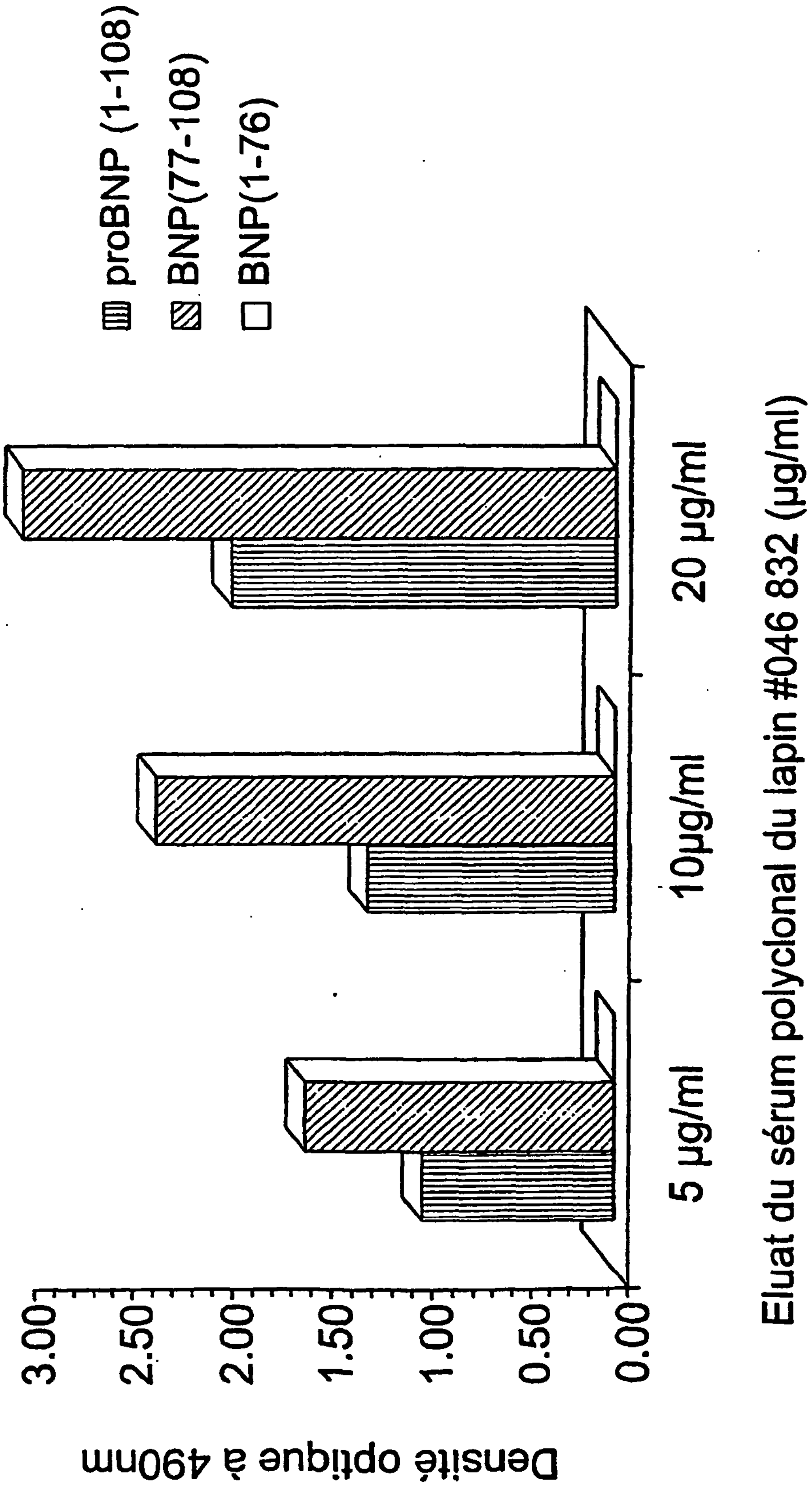
proBNP (1-108)
BNP(77-108)
BNP(1-76)
Densité optique à 490nm
3.00
2.50
2.00
1.50
1.00
0.50
0.00
5 µg/ml
10µg/ml
20 µg/ml
Eluat du sérum polyclonal du lapin #046 832 (µg/ml)

FIG.5

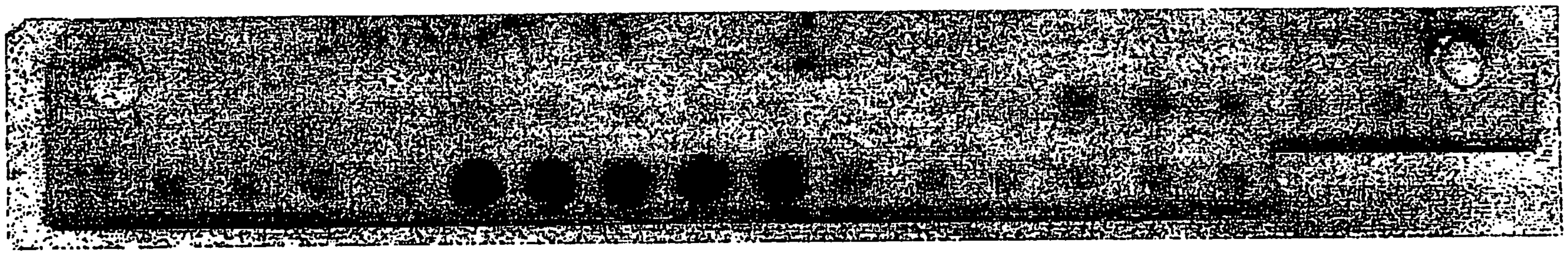

Réactivité (en unités relatives d'intensité) :

| | | | |
|---|---|---|---|
| 21 | IRGHRKMVLYTLRAP | 32.8 | |
| 22 | HRKMVLYTLRAPRSP | 104.8 | Spots détectés |
| 23 | MVLYTLRAPRSPKMV | 99.2 | Spots détectés |
| 24 | YTLRAPRSPKMVQGS | 117.5 | Spots détectés |
| 25 | RAPRSPKMVQGSGCF | 119.0 | Spots détectés |
| 26 | RSPKMVQGSGCFGRK | 84.5 | Spots détectés |
| 27 | KMVQGSGCFGRKMDR | 33.0 | |

FIG.6

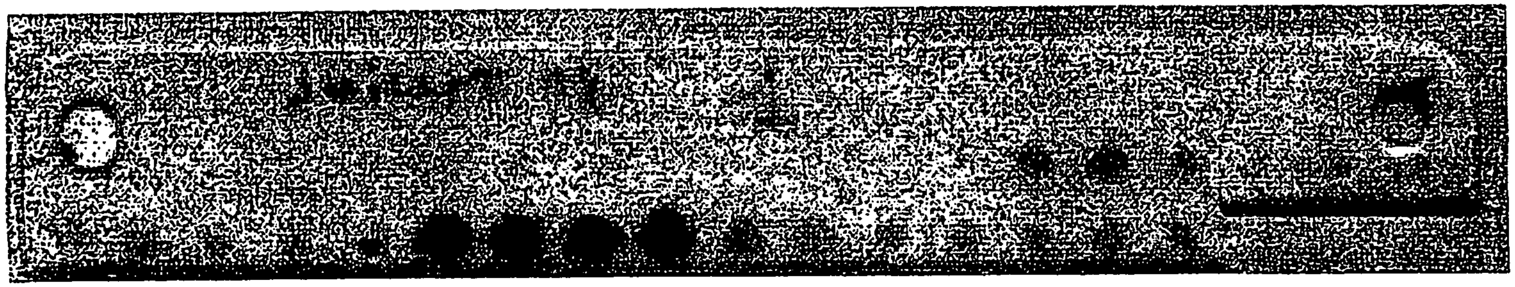

| | | Réactivité (en unités relatives d'intensité) | |
|---|---|---|---|
| 21 | IRGHRKMVLYTLRAP | 32,0 | |
| 22 | HRKMVLYTL RAPRSP | 125,9 | Spots détectés |
| 23 | MVLYTL RAPRSPKMV | 104,2 | Spots détectés |
| 24 | YTL RAPRSP KMVQGS | 121,1 | Spots détectés |
| 25 | RAPRSP KMVQGSGCF | 113,7 | Spots détectés |
| 26 | RSPKMVQGSGCFGRK | 35,0 | |

FIG.7

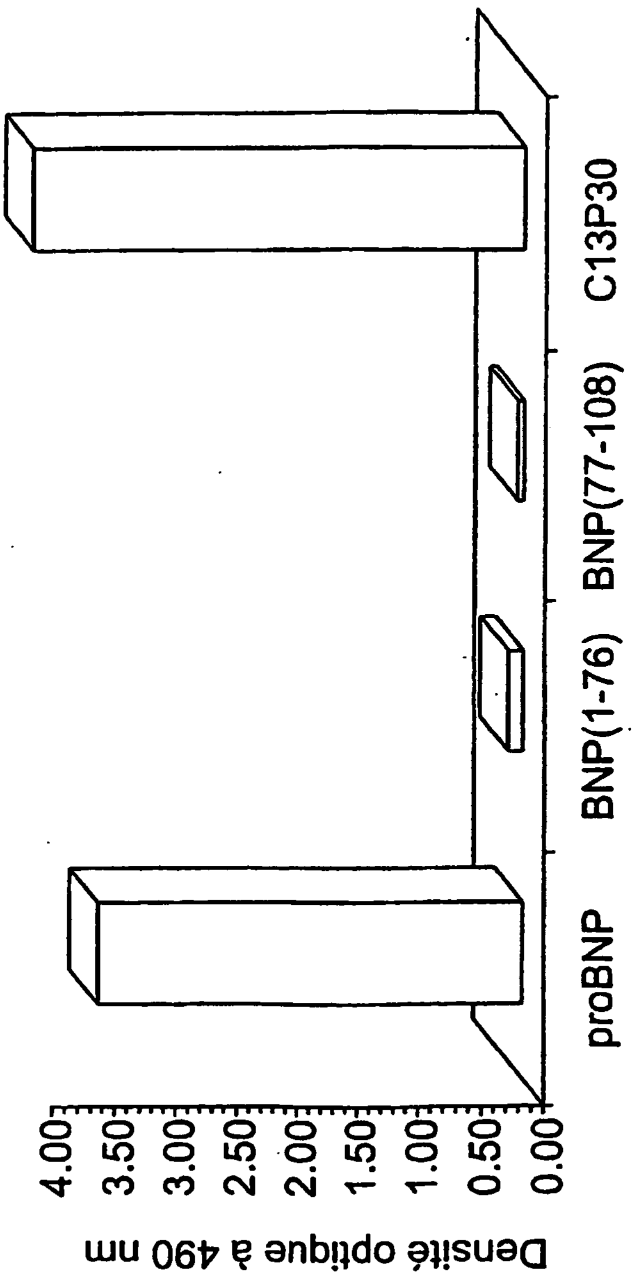
Densité optique à 490 nm
4.00
3.50
3.00
2.50
2.00
1.50
1.00
0.50
0.00
proBNP
BNP(1-76)
BNP(77-108)
C13P30

FIG.8

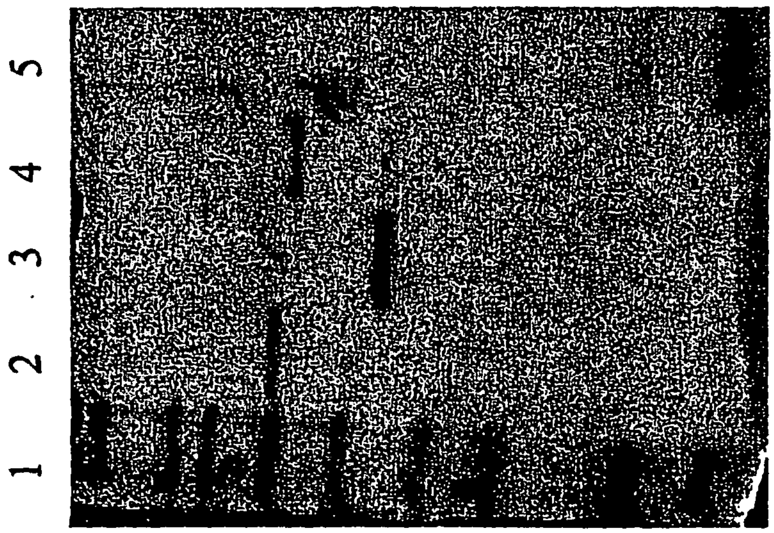


FIG.9

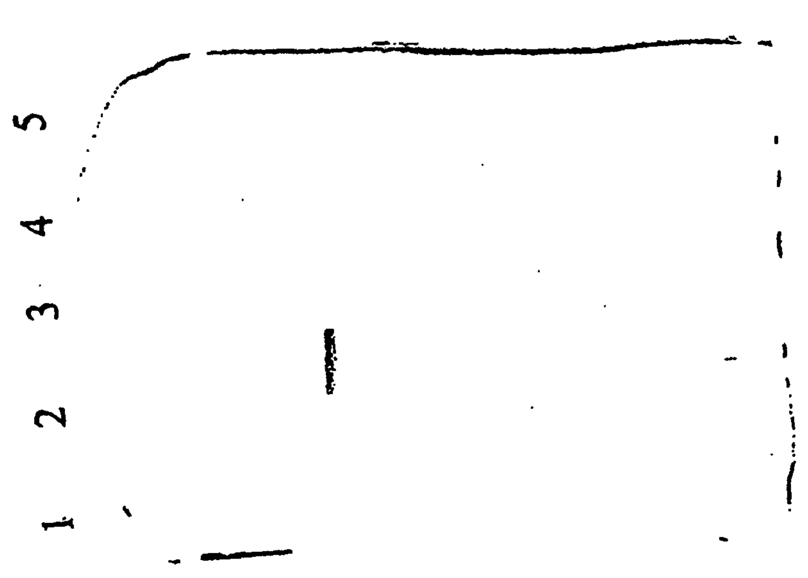


FIG.10

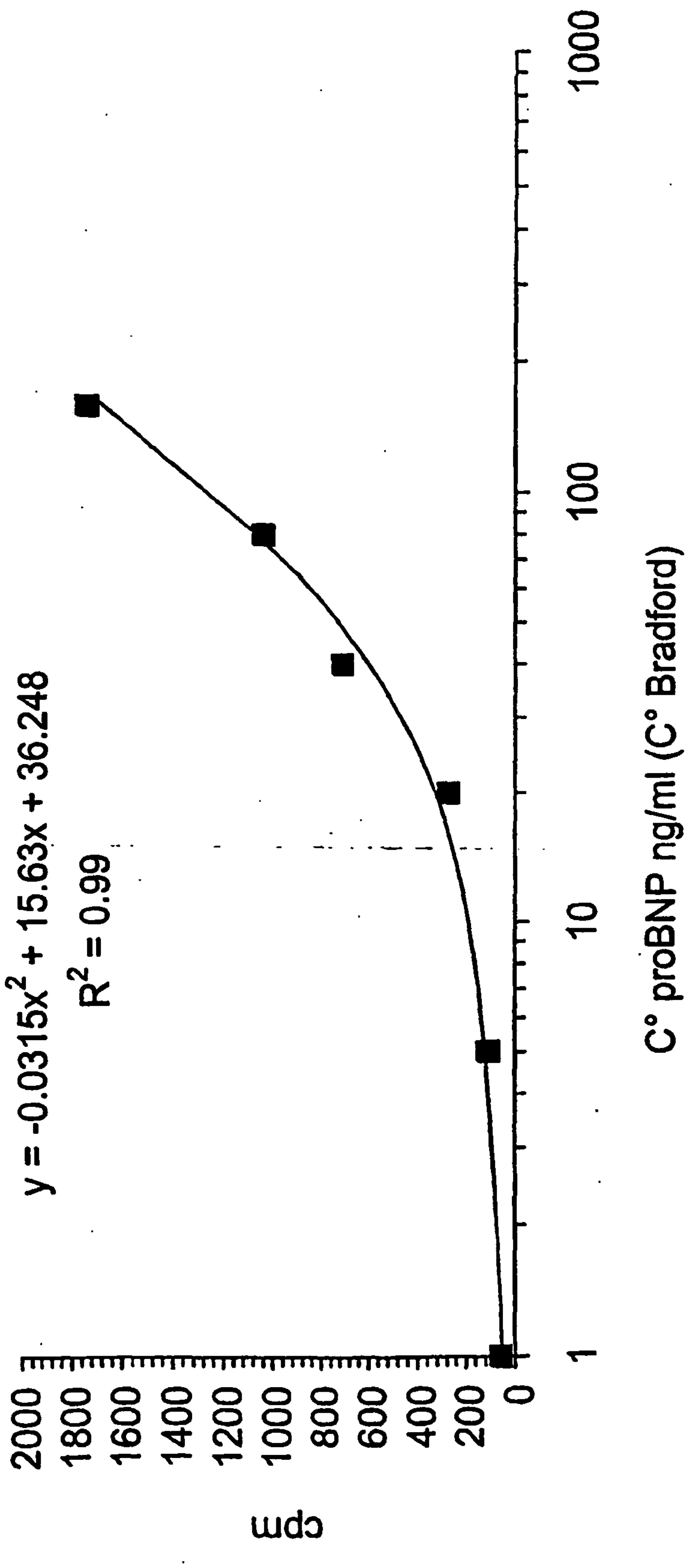
y = -0.0315x^2 + 15.63x + 36.248
R^2 = 0.99
cpm
2000
1800
1600
1400
1200
1000
800
600
400
200
0
1
10
100
1000
C° proBNP ng/ml (C° Bradford)

FIG.11

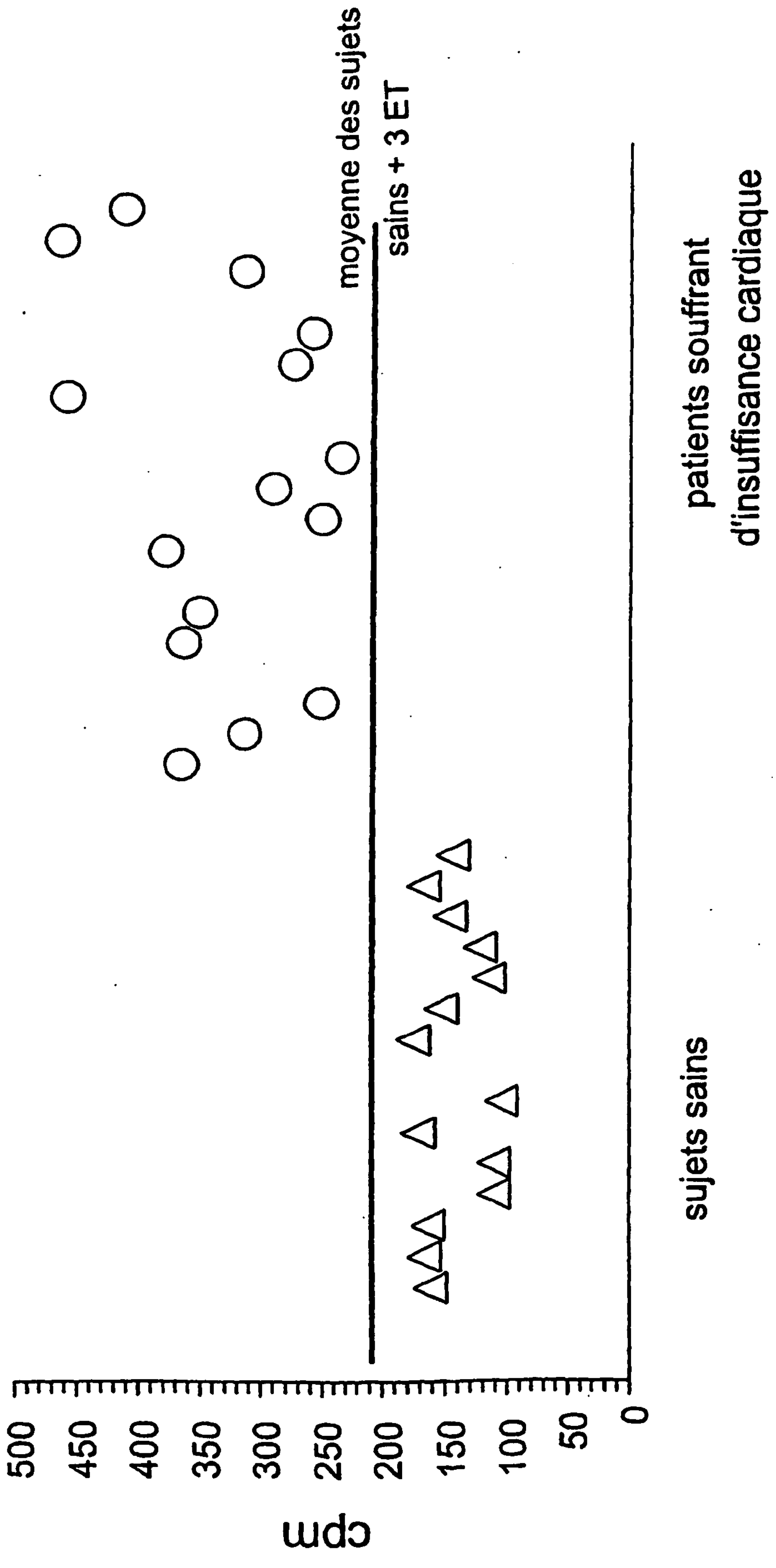
cpm
500
450
400
350
300
250
200
150
100
50
0
sujets sains
patients souffrant d'insuffisance cardiaque
moyenne des sujets sains + 3 ET

FIG.12

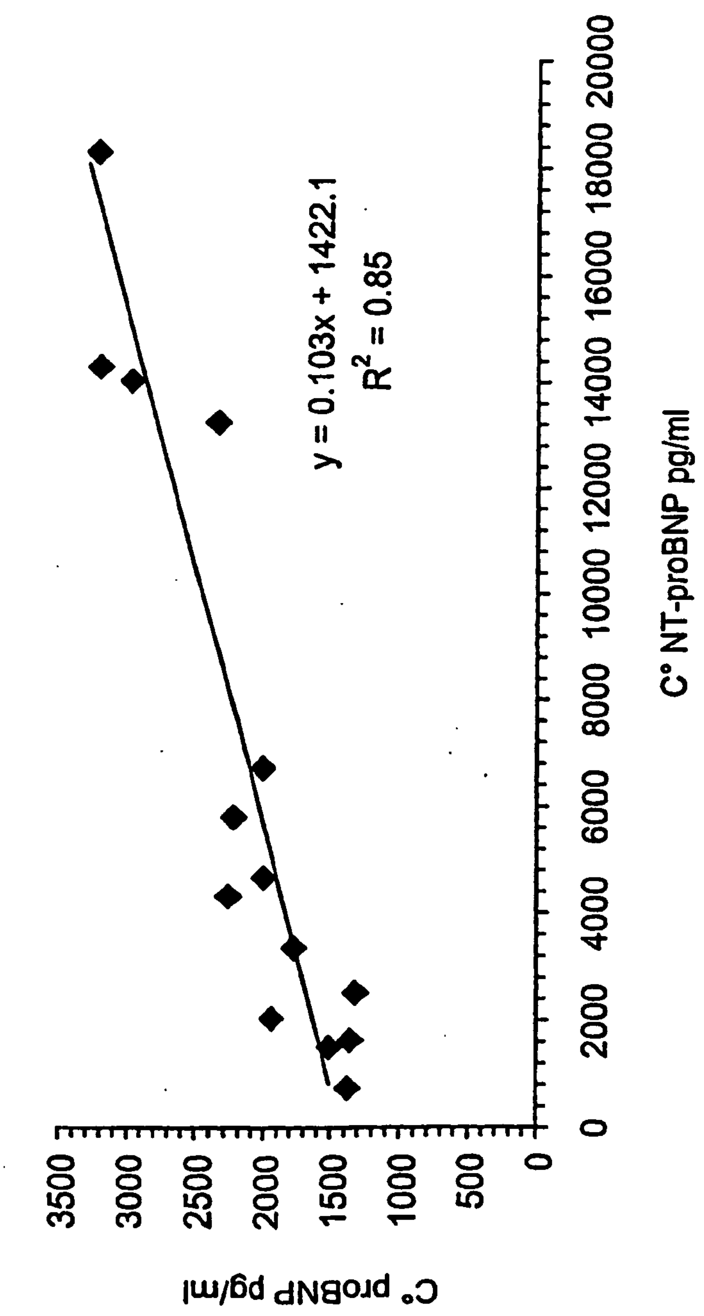
C° proBNP pg/ml
3500
3000
2500
2000
1500
1000
500
0
0
2000
4000
6000
8000
10000
12000
14000
16000
18000
20000
y = 0.103x + 1422.1
$R^2$ = 0.85
C° NT-proBNP pg/ml

FIG.13

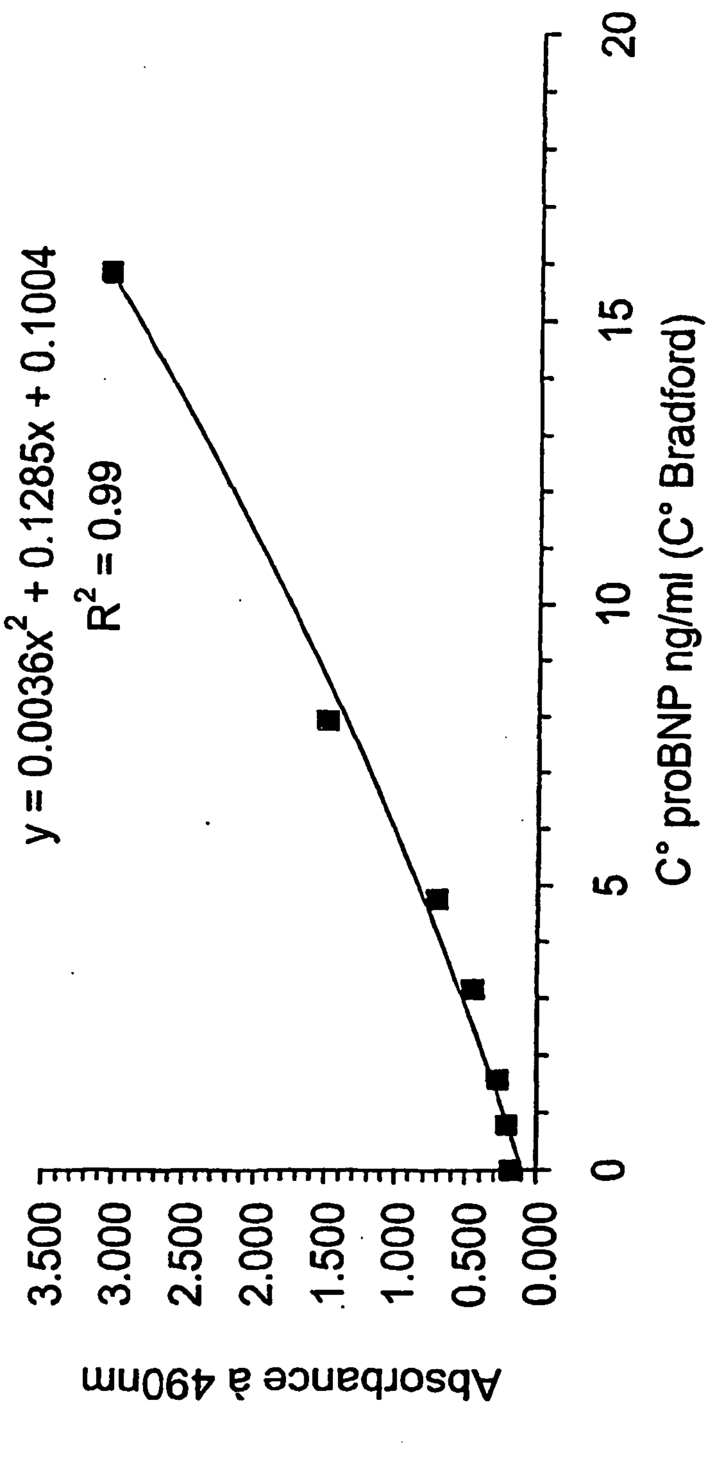
y = 0.0036x2 + 0.1285x + 0.1004
R2 = 0.99
Absorbance à 490nm
3.500
3.000
2.500
2.000
1.500
1.000
0.500
0.000
0
5
10
15
20
C° proBNP ng/ml (C° Bradford)

FIG.14

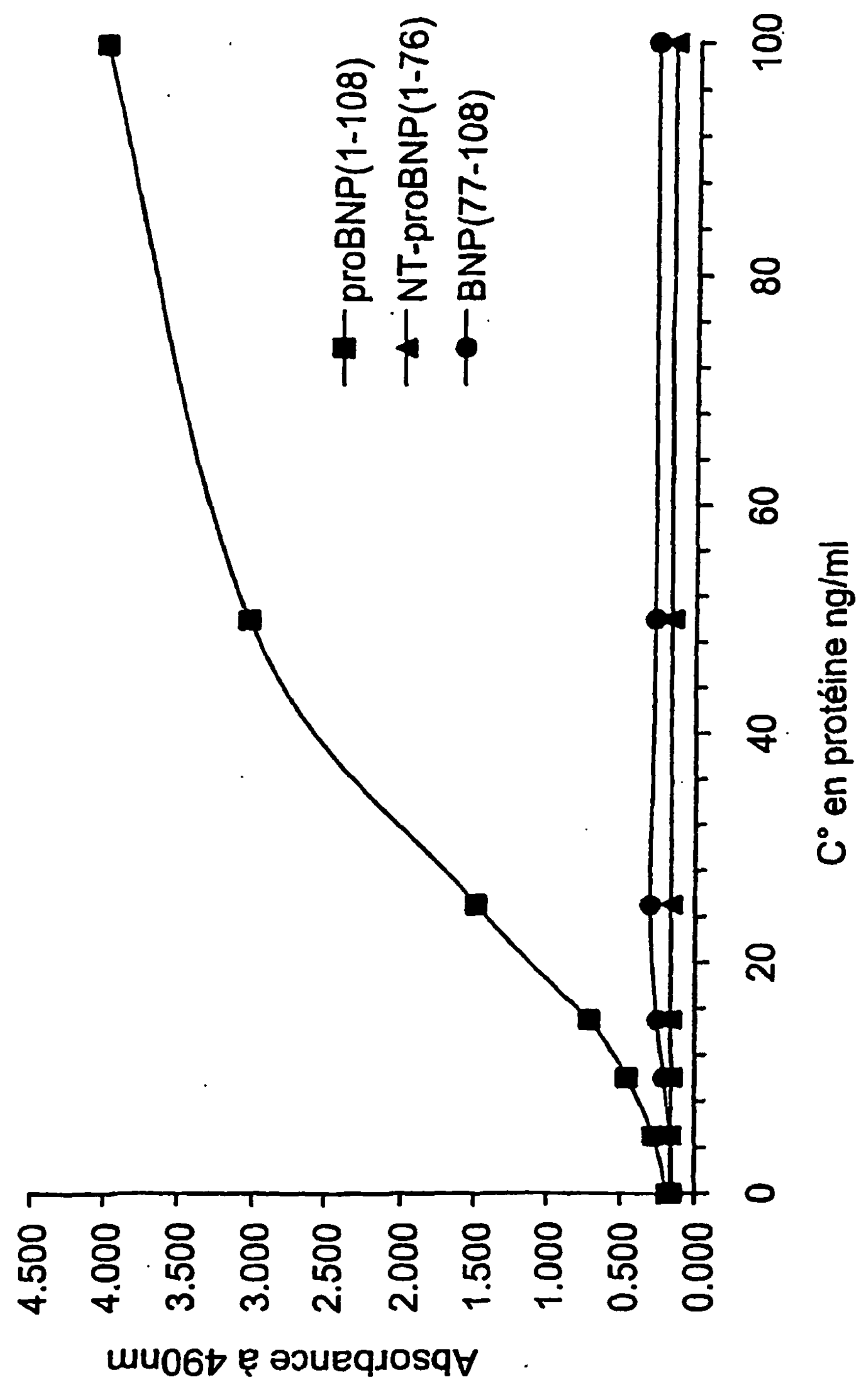
Absorbance à 490nm
4.500
4.000
3.500
3.000
2.500
2.000
1.500
1.000
0.500
0.000
0
20
40
60
80
100
C° en protéine ng/ml
proBNP(1-108)
NT-proBNP(1-76)
BNP(77-108)

FIG.15

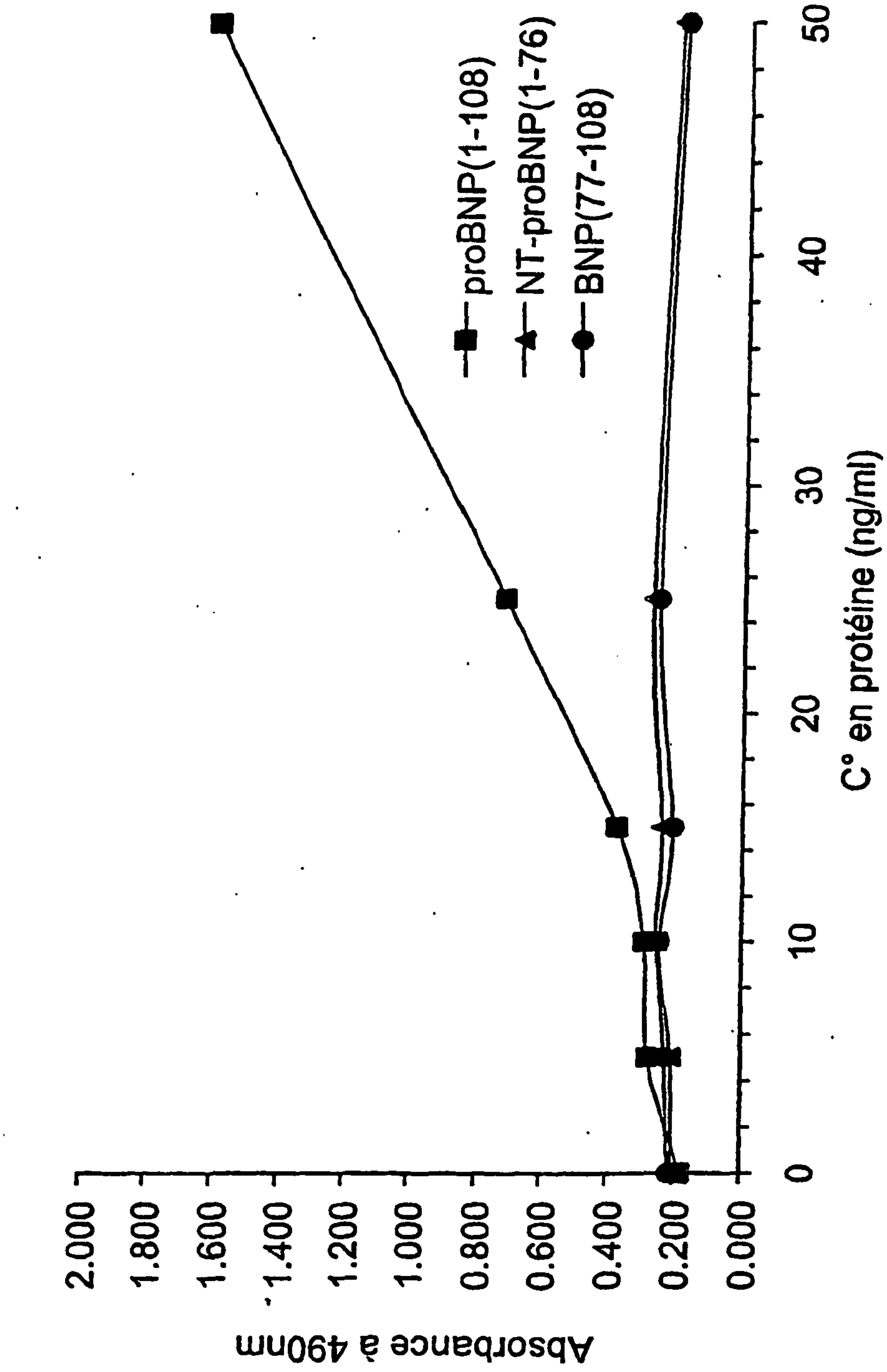
Absorbance à 490nm
2.000
1.800
1.600
1.400
1.200
1.000
0.800
0.600
0.400
0.200
0.000
0
10
20
30
40
50
C° en protéine (ng/ml)
proBNP(1-108)
NT-proBNP(1-76)
BNP(77-108)

FIG.16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9324531 A
- WO 9732900 A
- WO 0035951 A
- WO 0045176 A
- FR 0210063


### Littérature non-brevet citée dans la description


- **de Bold et al.** *Life Science,* 1981, vol. 28 (1), 89-94
- **Sudoh et al.** *Nature,* 1988, vol. 332, 78-81
- **Davidson, N. C. et al.** *Circulation,* 1995, vol. 91, 1276
- **Gobinet-Georges et al.** *Clin. Chem. Lab. Med.,* 2000, vol. 38, 519-23
- **Clerico A. et al.** *J. Endocrinol. Invest.,* 1998, vol. 21, 170-9
- **Del Ry S. et al.** *Scand. J. Clin. Lab. Invest.,* 2000, vol. 60, 81-90
- **Hunt et al.** *Biochemical and Biophysical Research Communications,* 1995, vol. 214 (3), 1175-1183
- **Karl et al.** *Scand. J. Clin. Lab. Invest.,* 1999, vol. 59 (230), 177-181
- **Schulz et al.** *Scand. J. Clin. Lab. Invest.,* 2001, vol. 61, 33-42
- **Shimizu et al.** *Clinica Chimica Acta,* 2002, vol. 316, 129-135
- **Köhler ; Milsteln.** *Nature (London),* 1976, vol. 256, 495-497
- **Köhler ; Milstein.** *Nature (London),* 1975, vol. 256, 495-497
- **Köhler ; Milstein.** *Nature,* 1975, vol. 256, 495-497
- Antibodies : a laboratory manual. 1988
- **Scott, J.K. ; Smith, G.P.** *Science,* 1990, vol. 249, 386-390
- **Marks et al.** *J. Mol. Biol,* 1991, vol. 222, 581-597
- **Yan et al.** *PNAS,* 2000, vol. 97, 8525-8529
- **Bradford M.** *Anal. Biochem.,* 1976, vol. 72, 248-54
- **Frank.** *Tetrahedron,* 1992, vol. 48, 9217-32
- **Molina et al.** *Pept. Res.,* 1996, vol. 9, 151-5
- **Koëhler ; Milstein.** *Nature,* 1975, vol. 256, 495-97

SEQUENCE LISTING

<110> BIO-RAD PASTEUR
CNRS
Université de Montpellier

<120> Anticorps spécifiques pour le diagnostic de l'insuffisance cardiaque

<130> BET 03P0750

<150> FR 0210063
<151> 2002-08-07

<160> 124

<170> PatentIn version 3.1

<210> 1
<211> 108
<212> PRT
<213> Homo sapiens : proBNP(1-108)

<400> 1

```
His Pro Leu Gly Ser Pro Gly Ser Ala Ser Asp Leu Glu Thr Ser Gly
1               5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
        35                  40                  45

Gly Val Trp Lys Ser Arg Glu Val Ala Thr Glu Gly Ile Arg Gly His
    50                  55                  60
```

```
Arg Lys Met Val Leu Tyr Thr Leu Arg Ala Pro Arg Ser Pro Lys Met
65                  70                  75                  80

Val Gln Gly Ser Gly Cys Phe Gly Arg Lys Met Asp Arg Ile Ser Ser
                85                  90                  95

Ser Ser Gly Leu Gly Cys Lys Val Leu Arg Arg His
            100                 105
```

<210> 2
<211> 32
<212> PRT
<213> Homo sapiens : proBNP(77-108)

<400> 2

```
Ser Pro Lys Met Val Gln Gly Ser Gly Cys Phe Gly Arg Lys Met Asp
1               5                   10                  15

Arg Ile Ser Ser Ser Ser Gly Leu Gly Cys Lys Val Leu Arg Arg His
            20                  25                  30
```

<210> 3
<211> 76
<212> PRT
<213> Homo sapiens : proBNP(1-76)

<400> 3

```
His Pro Leu Gly Ser Pro Gly Ser Ala Ser Asp Leu Glu Thr Ser Gly
1               5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
        35                  40                  45

Gly Val Trp Lys Ser Arg Glu Val Ala Thr Glu Gly Ile Arg Gly His
    50                  55                  60

Arg Lys Met Val Leu Tyr Thr Leu Arg Ala Pro Arg


    65                  70                  75
```

<210> 4
<211> 16
<212> PRT
<213> Séquence artificielle : proBNP(70-85)

<400> 4

Tyr Thr Leu Arg Ala Pro Arg Ser Pro Lys Met Val Gln Gly Ser Gly
1 5 10 15

<210> 5
<211> 6
<212> PRT
<213> Séquence artificielle : proBNP(73-78)

<400> 5

Arg Ala Pro Arg Ser Pro
1 5

<210> 6
<211> 8
<212> PRT
<213> Séquence artificielle : peptide

<400> 6

Cys Gly Arg Ala Pro Arg Ser Pro
1 5

<210> 7
<211> 8
<212> PRT
<213> Séquence artificielle : peptide

<220>
<221> MOD_RES
<222> (1)..(1)
<223> ACETYLATION

<400> 7

Cys Gly Arg Ala Pro Arg Ser Pro
1 5

<210> 8
<211> 9
<212> PRT
<213> Séquence artificielle : peptide

<400> 8

Cys Gly Arg Ala Pro Arg Ser Pro Lys
1               5

<210> 9
<211> 9
<212> PRT
<213> Séquence artificielle : peptide

<220>
<221> MOD_RES
<222> (1) .. (1)
<223> ACETYLATION

<400> 9

Cys Gly Arg Ala Pro Arg Ser Pro Lys
1               5

<210> 10
<211> 11
<212> PRT
<213> Séquence artificielle : peptide

<400> 10

Cys Gly Arg Ala Pro Arg Ser Pro Lys Met Val
1               5                   10

<210> 11
<211> 15
<212> PRT
<213> Séquence artificielle : peptide

<400> 11

Cys Gly Arg Ala Pro Arg Ser Pro Lys Met Val Gln Gly Ser Gly
1               5                   10                  15

<210> 12
<211> 8
<212> PRT
<213> Séquence artificielle : peptide

<400> 12

Arg Ala Pro Arg Ser Pro Gly Cys
1 5

<210> 13
<211> 8
<212> PRT
<213> Séquence artificielle : peptide

<220>
<221> MOD_RES
<222> (1)..(1)
<223> ACETYLATION

<400> 13

Arg Ala Pro Arg Ser Pro Gly Cys
1 5

<210> 14
<211> 11
<212> PRT
<213> Séquence artificielle : peptide

<220>
<221> MOD_RES
<222> (1)..(1)
<223> ACETYLATION

<400> 14

Cys Tyr Thr Leu Arg Ala Pro Arg Ser Pro Lys
1 5 10

<210> 15
<211> 17
<212> PRT
<213> Séquence artificielle : peptide

<400> 15

Cys His Arg Lys Met Val Leu Tyr Thr Leu Arg Ala Pro Arg Ser Pro
1 5 10 15

Lys

<210> 16
<211> 17
<212> PRT
<213> Séquence artificielle : peptide

<400> 16

Cys Tyr Thr Leu Arg Ala Pro Arg Ser Pro Lys Met Val Gln Gly Ser
1 5 10 15

Gly

<210> 17
<211> 17
<212> PRT
<213> Séquence artificielle : peptide

<400> 17

Cys Phe Thr Leu Arg Ala Pro Arg Ser Pro Lys Met Val Gln Gly Ser
1 5 10 15

Gly

<210> 18
<211> 17
<212> PRT
<213> Séquence artificielle : peptide

<400> 18

Cys Phe Ser Ile Arg Ala Pro Arg Ser Pro Lys Met Val Gln Gly Ser
1 5 10 15

Gly

<210> 19
<211> 17
<212> PRT
<213> Séquence artificielle : peptide

<220>
<221> MOD_RES
<222> (17)..(17)
<223> bAla

<400> 19

Cys Tyr Thr Leu Arg Ala Pro Arg Ser Pro Lys Met Val Gln Gly Ser
1 5 10 15

Ala

<210> 20
<211> 17
<212> PRT
<213> Séquence artificielle : peptide

<220>
<221> MOD_RES
<222> (17)..(17)
<223> bAla

<400> 20

Cys Tyr Thr Leu Arg Ala Pro Arg Ser Pro Lys Met Val Gln Ala Thr
1 5 10 15

Ala

<210> 21
<211> 17
<212> PRT
<213> Séquence artificielle : peptide

<220>
<221> MOD_RES
<222> (17)..(17)
<223> bAla

<400> 21

Cys Phe Ser Ile Arg Ala Pro Arg Ser Pro Lys Met Val Gln Ala Thr
1 5 10 15

Ala

<210> 22
<211> 17
<212> PRT
<213> Séquence artificielle : peptide

<400> 22

```
Cys Phe Ser Ile Arg Ala Pro Arg Ser Pro Ala Leu Ala Ser Gly Thr
1               5                   10                  15

Ala
```

<210> 23
<211> 15
<212> PRT
<213> Séquence artificielle : peptide

<400> 23

```
His Pro Leu Gly Ser Pro Gly Ser Ala Ser Asp Leu Glu Thr Ser
1               5                   10                  15
```

<210> 24
<211> 15
<212> PRT
<213> Séquence artificielle : peptide

<400> 24

```
Gly Ser Pro Gly Ser Ala Ser Asp Leu Glu Thr Ser Gly Leu Gln
1               5                   10                  15
```

<210> 25
<211> 15
<212> PRT
<213> Séquence artificielle : peptide

<400> 25

```
Gly Ser Ala Ser Asp Leu Glu Thr Ser Gly Leu Gln Glu Gln Arg
1               5                   10                  15
```

<210> 26
<211> 15
<212> PRT
<213> Séquence artificielle : peptide

<400> 26

```
Ser Asp Leu Glu Thr Ser Gly Leu Gln Glu Gln Arg Asn His Leu
1               5                   10                  15
```

<210> 27
<211> 15

<212> PRT
<213> Séquence artificielle : peptide

<400> 27

Glu Thr Ser Gly Leu Gln Glu Gln Arg Asn His Leu Gln Gly Lys
1 5 10 15

<210> 28
<211> 15
<212> PRT
<213> Séquence artificielle : peptide

<400> 28

Gly Leu Gln Glu Gln Arg Asn His Leu Gln Gly Lys Leu Ser Glu
1 5 10 15

<210> 29
<211> 15
<212> PRT
<213> Séquence artificielle : peptide

<400> 29

Glu Gln Arg Asn His Leu Gln Gly Lys Leu Ser Glu Leu Gln Val
1 5 10 15

<210> 30
<211> 15
<212> PRT
<213> Séquence artificielle : peptide

<400> 30

Asn His Leu Gln Gly Lys Leu Ser Glu Leu Gln Val Glu Gln Thr
1 5 10 15

<210> 31
<211> 15
<212> PRT
<213> Séquence artificielle : peptide

<400> 31

Gln Gly Lys Leu Ser Glu Leu Gln Val Glu Gln Thr Ser Leu Glu
1 5 10 15

<210> 32
<211> 15
<212> PRT
<213> Séquence artificielle : peptide

<400> 32

```
Leu Ser Glu Leu Gln Val Glu Gln Thr Ser Leu Glu Pro Leu Gln
1               5                   10                  15
```

<210> 33
<211> 15
<212> PRT
<213> Séquence artificielle : peptide

<400> 33

```
Leu Gln Val Glu Gln Thr Ser Leu Glu Pro Leu Gln Glu Ser Pro
1               5                   10                  15
```

<210> 34
<211> 15
<212> PRT
<213> Séquence artificielle : peptide

<400> 34

```
Glu Gln Thr Ser Leu Glu Pro Leu Gln Glu Ser Pro Arg Pro Thr
1               5                   10                  15
```

<210> 35
<211> 15
<212> PRT
<213> Séquence artificielle : peptide

<400> 35

```
Ser Leu Glu Pro Leu Gln Glu Ser Pro Arg Pro Thr Gly Val Trp
1               5                   10                  15
```

<210> 36
<211> 15
<212> PRT
<213> Séquence artificielle : peptide

<400> 36

Pro Leu Gln Glu Ser Pro Arg Pro Thr Gly Val Trp Lys Ser Arg
1 5 10 15

<210> 37
<211> 15
<212> PRT
<213> Séquence artificielle : peptide

<400> 37

Glu Ser Pro Arg Pro Thr Gly Val Trp Lys Ser Arg Glu Val Ala
1 5 10 15

<210> 38
<211> 15
<212> PRT
<213> Séquence artificielle : peptide

<400> 38

Arg Pro Thr Gly Val Trp Lys Ser Arg Glu Val Ala Thr Glu Gly
1 5 10 15

<210> 39
<211> 15
<212> PRT
<213> Séquence artificielle : peptide

<400> 39

Gly Val Trp Lys Ser Arg Glu Val Ala Thr Glu Gly Ile Arg Gly
1 5 10 15

<210> 40
<211> 15
<212> PRT
<213> Séquence artificielle : peptide

<400> 40

Lys Ser Arg Glu Val Ala Thr Glu Gly Ile Arg Gly His Arg Lys
1 5 10 15

<210> 41
<211> 15
<212> PRT
<213> Séquence artificielle : peptide

<400> 41

Glu Val Ala Thr Glu Gly Ile Arg Gly His Arg Lys Met Val Leu
1 5 10 15

<210> 42
<211> 15
<212> PRT
<213> Séquence artificielle : peptide

<400> 42

Thr Glu Gly Ile Arg Gly His Arg Lys Met Val Leu Tyr Thr Leu
1 5 10 15

<210> 43

<211> 15
<212> PRT
<213> Séquence artificielle : peptide

<400> 43

Ile Arg Gly His Arg Lys Met Val Leu Tyr Thr Leu Arg Ala Pro
1 5 10 15

<210> 44
<211> 15
<212> PRT
<213> Séquence artificielle : peptide

<400> 44

His Arg Lys Met Val Leu Tyr Thr Leu Arg Ala Pro Arg Ser Pro
1 5 10 15

<210> 45
<211> 15
<212> PRT
<213> Séquence artificielle : peptide

<400> 45

Met Val Leu Tyr Thr Leu Arg Ala Pro Arg Ser Pro Lys Met Val
1 5 10 15

<210> 46
<211> 15

<212> PRT
<213> Séquence artificielle : peptide

<400> 46

```
Tyr Thr Leu Arg Ala Pro Arg Ser Pro Lys Met Val Gln Gly Ser
1               5                   10                  15
```

<210> 47
<211> 15
<212> PRT
<213> Séquence artificielle : peptide

<400> 47

```
Arg Ala Pro Arg Ser Pro Lys Met Val Gln Gly Ser Gly Cys Phe
1               5                   10                  15
```

<210> 48
<211> 15
<212> PRT
<213> Séquence artificielle : peptide

<400> 48

```
Arg Ser Pro Lys Met Val Gln Gly Ser Gly Cys Phe Gly Arg Lys
1               5                   10                  15
```

<210> 49
<211> 15
<212> PRT
<213> Séquence artificielle : peptide

<400> 49

```
Lys Met Val Gln Gly Ser Gly Cys Phe Gly Arg Lys Met Asp Arg
1               5                   10                  15
```

<210> 50
<211> 15
<212> PRT
<213> Séquence artificielle : peptide

<400> 50

Gln Gly Ser Gly Cys Phe Gly Arg Lys Met Asp Arg Ile Ser Ser
1 5 10 15

<210> 51
<211> 15
<212> PRT
<213> Séquence artificielle : peptide

<400> 51

Gly Cys Phe Gly Arg Lys Met Asp Arg Ile Ser Ser Ser Ser Gly
1 5 10 15

<210> 52
<211> 15
<212> PRT
<213> Séquence artificielle : peptide

<400> 52

Gly Arg Lys Met Asp Arg Ile Ser Ser Ser Ser Gly Leu Gly Cys
1 5 10 15

<210> 53
<211> 15
<212> PRT
<213> Séquence artificielle : peptide

<400> 53

Met Asp Arg Ile Ser Ser Ser Ser Gly Leu Gly Cys Lys Val Leu
1 5 10 15

<210> 54
<211> 15
<212> PRT
<213> Séquence artificielle : peptide

<400> 54

Ile Ser Ser Ser Ser Gly Leu Gly Cys Lys Val Leu Arg Arg His
1 5 10 15

<210> 55
<211> 15
<212> PRT
<213> Séquence artificielle : peptide

<400> 55

Ala Thr Leu Arg Ala Pro Arg Ser Pro Lys Met Val Gln Gly Ser
1 5 10 15

<210> 56
<211> 15
<212> PRT
<213> Séquence artificielle : peptide

<400> 56

Tyr Ala Leu Arg Ala Pro Arg Ser Pro Lys Met Val Gln Gly Ser
1 5 10 15

<210> 57
<211> 15
<212> PRT
<213> Séquence artificielle : peptide

<400> 57

Tyr Thr Ala Arg Ala Pro Arg Ser Pro Lys Met Val Gln Gly Ser
1 5 10 15

<210> 58
<211> 15
<212> PRT
<213> Séquence artificielle : peptide

<400> 58

Tyr Thr Leu Ala Ala Pro Arg Ser Pro Lys Met Val Gln Gly Ser
1 5 10 15

<210> 59
<211> 15
<212> PRT
<213> Séquence artificielle : peptide

<400> 59

Tyr Thr Leu Arg Gly Pro Arg Ser Pro Lys Met Val Gln Gly Ser
1 5 10 15

<210> 60
<211> 15
<212> PRT
<213> Séquence artificielle : peptide

<400> 60

Tyr Thr Leu Arg Ala Ala Arg Ser Pro Lys Met Val Gln Gly Ser
1 5 10 15

<210> 61
<211> 15
<212> PRT
<213> Séquence artificielle : peptide

<400> 61

Tyr Thr Leu Arg Ala Pro Ala Ser Pro Lys Met Val Gln Gly Ser
1 5 10 15

<210> 62
<211> 15
<212> PRT
<213> Séquence artificielle : peptide

<400> 62

Tyr Thr Leu Arg Ala Pro Arg Ala Pro Lys Met Val Gln Gly Ser
1 5 10 15

<210> 63
<211> 15
<212> PRT
<213> Séquence artificielle : peptide

<400> 63

Tyr Thr Leu Arg Ala Pro Arg Ser Ala Lys Met Val Gln Gly Ser
1 5 10 15

<210> 64
<211> 15
<212> PRT
<213> Séquence artificielle : peptide

<400> 64

```
Tyr Thr Leu Arg Ala Pro Arg Ser Pro Ala Met Val Gln Gly Ser
1               5                   10                  15
```

<210> 65
<211> 15
<212> PRT
<213> Séquence artificielle : peptide

<400> 65

```
Tyr Thr Leu Arg Ala Pro Arg Ser Pro Lys Ala Val Gln Gly Ser
1               5                   10                  15
```

<210> 66
<211> 15
<212> PRT
<213> Séquence artificielle : peptide

<400> 66

```
Tyr Thr Leu Arg Ala Pro Arg Ser Pro Lys Met Ala Gln Gly Ser
1               5                   10                  15
```

<210> 67
<211> 15
<212> PRT
<213> Séquence artificielle : peptide

<400> 67

```
Tyr Thr Leu Arg Ala Pro Arg Ser Pro Lys Met Val Ala Gly Ser
1               5                   10                  15
```

<210> 68
<211> 15
<212> PRT
<213> Séquence artificielle : peptide

<400> 68

```
Tyr Thr Leu Arg Ala Pro Arg Ser Pro Lys Met Val Gln Ala Ser
1               5                   10                  15
```

<210> 69
<211> 15
<212> PRT
<213> Séquence artificielle : peptide

<400> 69

```
Tyr Thr Leu Arg Ala Pro Arg Ser Pro Lys Met Val Gln Gly Ala
1               5                   10                  15
```

<210> 70
<211> 15
<212> PRT
<213> Séquence artificielle : peptide

<400> 70

```
Pro Leu Gly Ser Pro Gly Ser Ala Ser Asp Leu Glu Thr Ser Gly
1               5                   10                  15
```

<210> 71
<211> 15
<212> PRT
<213> Séquence artificielle : peptide

<400> 71

```
Leu Gly Ser Pro Gly Ser Ala Ser Asp Leu Glu Thr Ser Gly Leu
1               5                   10                  15
```

<210> 72
<211> 15
<212> PRT
<213> Séquence artificielle : peptide

<400> 72

```
Ser Pro Gly Ser Ala Ser Asp Leu Glu Thr Ser Gly Leu Gln Glu
1               5                   10                  15
```

<210> 73
<211> 15
<212> PRT
<213> Séquence artificielle : peptide

<400> 73

Pro Gly Ser Ala Ser Asp Leu Glu Thr Ser Gly Leu Gln Glu Gln
1 5 10 15

<210> 74
<211> 15
<212> PRT
<213> Séquence artificielle : peptide

<400> 74

Ser Ala Ser Asp Leu Glu Thr Ser Gly Leu Gln Glu Gln Arg Asn
1 5 10 15

<210> 75
<211> 15
<212> PRT
<213> Séquence artificielle : peptide

<400> 75

Ala Ser Asp Leu Glu Thr Ser Gly Leu Gln Glu Gln Arg Asn His
1 5 10 15

<210> 76
<211> 15
<212> PRT
<213> Séquence artificielle : peptide

<400> 76

Ser Asp Leu Glu Thr Ser Gly Leu Gln Glu Gln Arg Asn His Leu
1 5 10 15

<210> 77
<211> 15
<212> PRT
<213> Séquence artificielle : peptide

<400> 77

Asp Leu Glu Thr Ser Gly Leu Gln Glu Gln Arg Asn His Leu Gln
1 5 10 15

<210> 78
<211> 15
<212> PRT

<213> Séquence artificielle : peptide

<400> 78

```
Leu Glu Thr Ser Gly Leu Gln Glu Gln Arg Asn His Leu Gln Gly
1               5                   10                  15
```

<210> 79
<211> 15
<212> PRT
<213> Séquence artificielle : peptide

<400> 79

```
Leu Gln Glu Gln Arg Asn His Leu Gln Gly Lys Leu Ser Glu Leu
1               5                   10                  15
```

<210> 80
<211> 15
<212> PRT
<213> Séquence artificielle : peptide

<400> 80

```
Gln Glu Gln Arg Asn His Leu Gln Gly Lys Leu Ser Glu Leu Gln
1               5                   10                  15
```

<210> 81
<211> 15
<212> PRT
<213> Séquence artificielle : peptide

<400> 81

```
Gln Arg Asn His Leu Gln Gly Lys Leu Ser Glu Leu Gln Val Glu
1               5                   10                  15
```

<210> 82
<211> 15
<212> PRT
<213> Séquence artificielle : peptide

<400> 82

```
Arg Asn His Leu Gln Gly Lys Leu Ser Glu Leu Gln Val Glu Gln
1               5                   10                  15
```

<210> 83
<211> 15
<212> PRT
<213> Séquence artificielle : peptide

<400> 83

```
His Leu Gln Gly Lys Leu Ser Glu Leu Gln Val Glu Gln Thr Ser
1               5                   10                  15
```

<210> 84
<211> 15
<212> PRT
<213> Séquence artificielle : peptide

<400> 84

```
Leu Gln Gly Lys Leu Ser Glu Leu Gln Val Glu Gln Thr Ser Leu
1               5                   10                  15
```

<210> 85
<211> 15
<212> PRT
<213> Séquence artificielle : peptide

<400> 85

```
Gln Gly Lys Leu Ser Glu Leu Gln Val Glu Gln Thr Ser Leu Glu
1               5                   10                  15
```

<210> 86
<211> 15
<212> PRT
<213> Séquence artificielle : peptide

<400> 86

```
Gly Lys Leu Ser Glu Leu Gln Val Glu Gln Thr Ser Leu Glu Pro
1               5                   10                  15
```

<210> 87
<211> 15
<212> PRT
<213> Séquence artificielle : peptide

<400> 87

Lys Leu Ser Glu Leu Gln Val Glu Gln Thr Ser Leu Glu Pro Leu
1 5 10 15

<210> 88
<211> 15
<212> PRT
<213> Séquence artificielle : peptide

<400> 88

Leu Glu Pro Leu Gln Glu Ser Pro Arg Pro Thr Gly Val Trp Lys
1 5 10 15

<210> 89
<211> 15
<212> PRT
<213> Séquence artificielle : peptide

<400> 89

Glu Pro Leu Gln Glu Ser Pro Arg Pro Thr Gly Val Trp Lys Ser
1 5 10 15

<210> 90
<211> 15
<212> PRT
<213> Séquence artificielle : peptide

<400> 90

Pro Leu Gln Glu Ser Pro Arg Pro Thr Gly Val Trp Lys Ser Arg
1 5 10 15

<210> 91
<211> 15
<212> PRT
<213> Séquence artificielle : peptide

<400> 91

Leu Gln Glu Ser Pro Arg Pro Thr Gly Val Trp Lys Ser Arg Glu
1 5 10 15

<210> 92
<211> 15
<212> PRT

<213> Séquence artificielle : peptide

<400> 92

Gln Glu Ser Pro Arg Pro Thr Gly Val Trp Lys Ser Arg Glu Val
1 5 10 15

<210> 93
<211> 15
<212> PRT
<213> Séquence artificielle : peptide

<400> 93

Glu Ser Pro Arg Pro Thr Gly Val Trp Lys Ser Arg Glu Val Ala
1 5 10 15

<210> 94
<211> 15
<212> PRT
<213> Séquence artificielle : peptide

<400> 94

Ser Pro Arg Pro Thr Gly Val Trp Lys Ser Arg Glu Val Ala Thr
1 5 10 15

<210> 95
<211> 15
<212> PRT
<213> Séquence artificielle : peptide

<400> 95

Pro Arg Pro Thr Gly Val Trp Lys Ser Arg Glu Val Ala Thr Glu
1 5 10 15

<210> 96
<211> 15
<212> PRT
<213> Séquence artificielle : peptide

<400> 96

Pro Thr Gly Val Trp Lys Ser Arg Glu Val Ala Thr Glu Gly Ile
1 5 10 15

<210> 97
<211> 15
<212> PRT
<213> Séquence artificielle : peptide

<400> 97

Thr Gly Val Trp Lys Ser Arg Glu Val Ala Thr Glu Gly Ile Arg
1 5 10 15

<210> 98
<211> 15
<212> PRT
<213> Séquence artificielle : peptide

<400> 98

Ile Arg Gly His Arg Lys Met Val Leu Tyr Thr Leu Arg Ala Pro
1 5 10 15

<210> 99
<211> 15
<212> PRT
<213> Séquence artificielle : peptide

<400> 99

Arg Gly His Arg Lys Met Val Leu Tyr Thr Leu Arg Ala Pro Arg
1 5 10 15

<210> 100
<211> 15
<212> PRT
<213> Séquence artificielle : peptide

<400> 100

Gly His Arg Lys Met Val Leu Tyr Thr Leu Arg Ala Pro Arg Ser
1 5 10 15

<210> 101
<211> 15
<212> PRT
<213> Séquence artificielle : peptide

<400> 101

Arg Lys Met Val Leu Tyr Thr Leu Arg Ala Pro Arg Ser Pro Lys
1 5 10 15

<210> 102
<211> 15
<212> PRT
<213> Séquence artificielle : peptide

<400> 102

Lys Met Val Leu Tyr Thr Leu Arg Ala Pro Arg Ser Pro Lys Met
1 5 10 15

<210> 103
<211> 15
<212> PRT
<213> Séquence artificielle : peptide

<400> 103

Val Leu Tyr Thr Leu Arg Ala Pro Arg Ser Pro Lys Met Val Gln
1 5 10 15

<210> 104
<211> 35
<212> PRT
<213> Séquence artificielle : peptide

<400> 104

Ser Pro Lys Met Val Gln Gly Ser Gly Cys Phe Gly Arg Lys Met Asp
1 5 10 15

Arg Ile Ser Ser Ser Ser Gly Leu Gly Cys Lys Val Leu Arg Arg His
20 25 30

Lys Lys Lys
35

<210> 105
<211> 10
<212> PRT
<213> Séquence artificielle : peptide

<400> 105

Ser Pro Lys Met Val Gln Gly Ser Gly Cys
1 5 10

<210> 106
<211> 12
<212> PRT
<213> Séquence artificielle : peptide

<400> 106

Arg Lys Met Val Leu Tyr Thr Leu Arg Ala Pro Arg
1 5 10

<210> 107
<211> 13
<212> PRT
<213> Séquence artificielle : peptide

<400> 107

His Arg Lys Met Val Leu Tyr Thr Leu Arg Ala Pro Arg
1 5 10

<210> 108
<211> 15
<212> PRT
<213> Séquence artificielle : peptide

<400> 108

Tyr Thr Leu Arg Ala Pro Arg Ser Pro Lys Met Val Gln Gly Ser
1 5 10 15

<210> 109
<211> 16
<212> PRT
<213> Séquence artificielle : peptide

<400> 109

Cys Tyr Thr Leu Arg Ala Pro Arg Ser Pro Lys Met Val Gln Gly Ser
1 5 10 15

<210> 110
<211> 11
<212> PRT
<213> Séquence artificielle : peptide

<400> 110

```
Cys Tyr Thr Leu Arg Ala Pro Arg Ser Pro Lys
1               5                   10
```

<210> 111
<211> 13
<212> PRT
<213> Séquence artificielle : peptide

<400> 111

```
Cys Tyr Thr Leu Arg Ala Pro Arg Ser Pro Lys Met Val
1               5                   10
```

<210> 112
<211> 14
<212> PRT
<213> Séquence artificielle : peptide

<400> 112

```
Cys Tyr Thr Leu Arg Ala Pro Arg Ser Pro Lys Met Val Gln
1               5                   10
```

<210> 113
<211> 15
<212> PRT
<213> Séquence artificielle : peptide

<400> 113

```
Cys Tyr Thr Leu Arg Ala Pro Arg Ser Pro Lys Met Val Gln Gly
1               5                   10                  15
```

<210> 114
<211> 13
<212> PRT
<213> Séquence artificielle : peptide

<220>
<221> MOD_RES
<222> (1) .. (1)
<223> ACETYLATION

<400> 114

Cys Thr Leu Arg Ala Pro Arg Ser Pro Lys Met Val Gln
1 5 10

<210> 115
<211> 14
<212> PRT
<213> Séquence artificielle : peptide

<400> 115

Cys Thr Leu Arg Ala Pro Arg Ser Pro Lys Met Val Gln Gly
1 5 10

<210> 116
<211> 15
<212> PRT
<213> Séquence artificielle : peptide

<400> 116

Cys Thr Leu Arg Ala Pro Arg Ser Pro Lys Met Val Gln Gly Ser
1 5 10 15

<210> 117
<211> 16
<212> PRT
<213> Séquence artificielle : peptide

<400> 117

Cys Thr Leu Arg Ala Pro Arg Ser Pro Lys Met Val Gln Gly Ser Gly
1 5 10 15

<210> 118
<211> 11
<212> PRT
<213> Séquence artificielle : peptide

<400> 118

Cys Leu Arg Ala Pro Arg Ser Pro Lys Met Val
1 5 10

<210> 119
<211> 12
<212> PRT
<213> Séquence artificielle : peptide

<400> 119

Cys Leu Arg Ala Pro Arg Ser Pro Lys Met Val Gln
1               5                   10

<210> 120
<211> 12
<212> PRT
<213> Séquence artificielle : peptide

<400> 120

Leu Arg Ala Pro Arg Ser Pro Lys Met Val Gln Cys
1               5                   10

<210> 121
<211> 14
<212> PRT
<213> Séquence artificielle : peptide

<400> 121

Cys Leu Arg Ala Pro Arg Ser Pro Lys Met Val Gln Gly Ser
1               5                   10

<210> 122
<211> 15
<212> PRT
<213> Séquence artificielle : peptide

<400> 122

Cys Leu Arg Ala Pro Arg Ser Pro Lys Met Val Gln Gly Ser Gly
1               5                   10                  15

<210> 123
<211> 11
<212> PRT
<213> Séquence artificielle : peptide

<400> 123

Leu Gln Glu Gln Arg Asn His Leu Gln Gly Lys
1               5                   10

<210> 124
<211> 12

<212> PRT
<213> Séquence artificielle : peptide

<400> 124

```
Leu Glu Pro Leu Gln Glu Ser Pro Arg Pro Thr Gly
1               5                   10
```